# EUROPEAN PATENT APPLICATION

(11) **EP 1 956 016 A1**
(43) Date of publication of application: **13.08.2008**
(21) Application number: 06077263.9
(22) Date of filing: 15.12.2006
(51) Int. Cl.: C07D 307/79, C07D 311/04, C07D 313/08, C07D 407/14, A61K 31/4045, A61P 15/00

(54) **Bicyclic acyltryptophanols**

(71) Applicant: Bayer Schering Pharma Aktiengesellschaft, 13353 Berlin (DE)
(72) Inventor: Wortmann, Lars, 10435 Berlin (DE); Menzenbach, Bernd, 07743 Jena (DE); Koppitz, Marcus, 10115 Berlin (DE); Kosemund, Dirk, 99091 Erfurt (DE); Muhn, Hans-Peter, 13465 Berlin (DE); Schrey, Anna, 10179 Berlin (DE); Kuehne, Ronald, 12683 Berlin (DE); Frenzel, Thomas, 65719 Hofheim (Taunus) (DE); Liesener, Florian Peter, 83308 Trostberg (DE)

(57) **Abstract**

The present invention relates to acyltryptophanols of the general formula I, 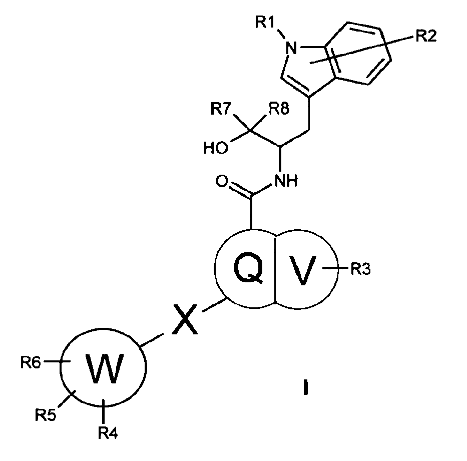 in which Q, V, X, W, R1, R2, R3, R4, R5, R6, R7 and R8 have the meaning as defined in the description.

The compounds according to the invention are effective FSH antagonists and can be used for example for fertility control in men or in women, or for the prevention and/or treatment of osteoporosis.

## Description

The present invention relates to novel bicyclic acyltryptophanols, process for their preparation, pharmaceutical compositions comprising the compounds according to the invention, and the use thereof for fertility control in men or in women as well as for treatment and prevention of osteoporosis.

Follicle-stimulating hormone (FSH) and luteinizing hormone (LH) are together responsible for the control of male and female fertility and of the production of sex steroids.

In the female mammal, FSH controls the early ripening of ovarian primary follicles and the biosynthesis of sex steroids. In the advanced stage of differentiation (preantral follicles), the influence of LH becomes increasingly important for further development of the follicles until ovulation occurs.

In male mammals, FSH is primarily responsible for the differentiation and stimulation of Sertoli cells. Their function consists of assisting spermatogenesis on many levels. LH is primarily responsible for stimulating the Leydig cells and thus androgen production. FSH, LH and TSH (thyrotropic hormone) together form the group of glycoprotein hormones which are formed in the pituitary and are secreted from there. Whereas the alpha subunit is common to the three hormones, their specificity of action is determined by the beta chain which is unique in each case. The molecular weight of FSH including the sugar portion is about 30 kD.

FSH and the other glycoprotein hormones act specifically via their selectively expressed G protein-coupled receptor (GPCR). FSH stimulates, through binding to its receptor, the association thereof with a stimulating G protein (Gₛ) which is thereby stimulated to hydrolyse guanosine triphosphate (GTP) and to activate the membrane-associated adenylate cyclase. Cyclic adenosine monophosphate (cAMP) is accordingly an important and readily quantifiable secondary messenger substance of FSH (G. Vassart, L. Pardo, S. Costagliola, Trends Biochem. Sci. 2004, 29, 119-126).

The importance of FSH for male fertility is the subject of intensive research. It has been possible to show that FSH influences several processes of spermatogenesis such as the proliferation of spermatogonia, the antiapoptotic effect on spermatogonia and spermatocytes and the stimulation of sperm maturation including motility thereof.

The following arguments are also in favour of the FSH receptor as target for male fertility control:
1. The FSH receptor is exclusively expressed on Sertoli cells (high specificity).
2. Contraceptive vaccination against FSH beta chain or the FSH receptor induces infertility in male primates (N. R. Mougdal, M. Jeyakumar, H. N. Krishnamurthy, S. Sridhar, H. Krishnamurthy, F. Martin, Human Reproduction Update 1997, 3, 335-346).
3. Naturally occurring mutations in the FSH receptor or the FSH beta chain may lead to sub- or infertility in men (I. Huhtaniemi, Journal of Reproduction and Fertility 2000, 119, 173-186; L. C. Layman, P. G. McDonough, Molecular and Cellular Endocrinology 2000, 161, 9-17).
4. Neutralizing FSH antiserum has no effect on testis weight and testosterone production (V. Sriraman, A. J. Rao, Molecular and Cellular Endocrionology 2004, 224, 73-82). Adverse effects of FSH blockade on androgen production therefore appear unlikely.

In line with these arguments, FSH antagonists are expected to be suitable for spermatogenesis inhibition (prevention) in men. Moreover, a suitable FSH antagonist may just as well lead to infertility in women, because it suppresses follicle ripening and thus also ovulation. On the other hand, the skilled person expects advantages from non-peptidergic FSH agonists when used to promote fertility in women (stimulation of follicle ripening). There are no reports of experience on the use of FSH or FSH agonists in male infertility, but specific indications are also conceivable in this connection.

New findings demonstrate that there is also a direct effect of FSH on cells of bone metabolism. There are two fundamentally different cell types in bones: osteoclasts and osteoblasts. While osteoclasts play a central role in bone resorption (breakdown of bone), osteoblasts simulate bone density (anabolic effect).

FSH receptors have been detected in osteoclasts but not in osteoblasts. In vitro, FSH stimulates bone resorption by mouse osteoclasts ( Li Sun et al. Cell 2006; 125: 247-60). A clinical correlation between the height of the serum FSH levels and low bone density has been observed in postmenopausal women (Devleta et al, J. Bone Miner. Metab. 2004, 22: 360-4).

These findings among others suggest that FSH stimulates loss of bone mass, and accordingly FSH antagonists will display an antiresorptive effect on bone and are therefore suitable for the therapy and/or prevention of peri- and postmenopausal loss of bone mass and osteoporosis.

FSH receptor modulators, i.e. FSH receptor antagonists and FSH receptor agonists of various compound classes of low molecular weight have been reported on recently. FSH receptor modulators are disclosed in WO 2004/056779, WO 2004/056780; J. Med. Chem. 2005, 48, 1697 [tetrahydroquinolines]; WO 02/70493, Bioorg. Med. Chem. Lett. 2004, 14, 1713 and 1717 [diketopiperazines]; and WO 01/47875 [sulphonamides].

FSH receptor agonists are disclosed in WO 02/09706; J. Comb. Chem. 2004, 6, 196 [Thiazolidinones]; WO 2003/020726 and WO 03/20727, Chem. Biochem. 2002, 10, 1023 {thieno[2,3-d] pyrimidines)}; WO 01/87287 [pyrazoles]; WO 00/08015 [carbazoles]; WO 06/117023, WO 06/117368, WO 06/117370, WO 06/117371, [hexahydroquino-lines].

FSH receptor antagonists are disclosed in WO 03/004028 [tetrahydroquinolines], WO 02/09705 [thiazolidinones], WO 00/58277, Bioorg. Med. Chem. 2002, 10, 639 [sulphonic acids]; WO 00/58276, Endocr. 2002, 143, 3822; Synth. Comm. 2002, 32, 2695 [azo compounds]; US 2006/0199806, US 2006/0258644, US 2006/0258645 [pyrrolobenzodiazepines] and PCT/EP2006/00794 [broad class of acyltryptohanols, as prior right only].

In view of the prior art, the objective technical problem to be solved according to the present invention may therefore be seen in providing alternative compounds having an FSH receptor antagonistic effect.

The technical problem has been solved according to the present invention by the provision of novel compounds of the formula I
in which
- R1: is hydrogen, C₁-C₆-alkyl, C₃-C₆-alkenyl, C₃-C₆-alkynyl, C₃-C₇-cycloalkyl, C₁-C₆-alkyloxy-C₁-C₆-alkylene, C₃-C₇-cycloalkyloxy-C₁-C₆-alkylene, C₁-C₆-alkylamino-C₁-C₆-alkylene, di(C₁-C₆-alkyl)amino-C₁-C₆-alkylene, phenyloxy-C₁-C₆-alkylene; wherein the hydrocarbon chains may optionally be substituted once or more times by fluorine, cyano, hydroxy, amino or the groups:

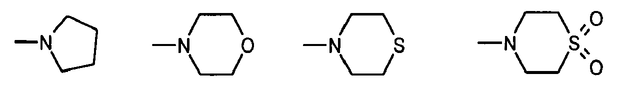

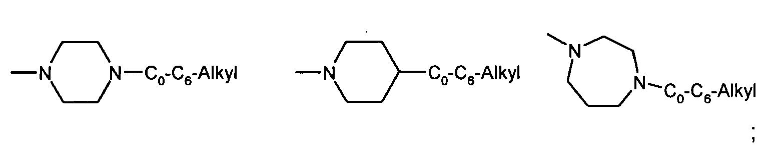

- R2: is hydrogen, halogen, cyano, -SO₂Me, C₁-C₆-alkyl, C₂-C₆-alkenyl, C₂-C₆-alkynyl, C₁-C₆-alkyloxy or benzyloxy,
wherein the hydrocarbon chains may optionally be fluorinated once or more times;

- R3: is hydrogen, hydroxy, halogen, nitro, amino, cyano, C₁-C₆-alkyl, C₂-C₆-alkenyl or C₂-C₆-alkynyl, C₃-C₇-cycloalkyl, hydroxy-C₁-C₆-alkylene, hydroxy-C₃-C₆-alkenylene, hydroxy-C₃-C₆-alkynylene, C₁-C₆-alkyloxy, C₁-C₆-alkyloxy-C₁-C₆-alkylene, C₃-C₇-cycloalkyloxy, C₃-C₇-cycloalkyl-C₁-C₆-alkylenoxy, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, C₁-C₆-alkylamino-C₁-C₆-alkylene, di(C₁-C₆-alkyl)amino-C₁-C₆-alkylene, phenyloxy-C₁-C₆-alkylene;
where the hydrocarbon chains therein may optionally be substituted one or more times by fluorine, cyano, hydroxy, amino or the groups

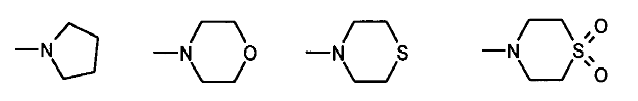

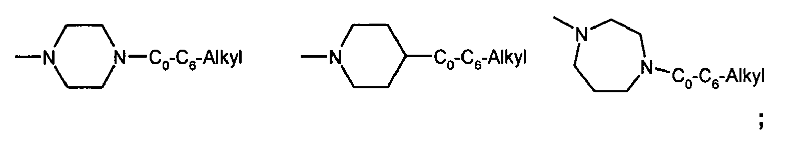

- R4, R5, R6: are independently of one another hydrogen, hydroxy, halogen, nitro, amino, cyano, phenyl, C₁-C₆-alkyl, C₂-C₆-alkenyl or C₂-C₆-alkynyl, C₃-C₇-cycloalkyl, C₃-C₇-cycloalkyl-C₁-C₆-alkylene, C₃-C₇-heterocycloalkyl, where the hydrocarbon chains therein may optionally be substituted one or more times by fluorine, cyano or the groups: 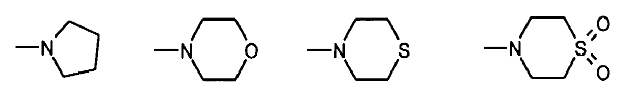 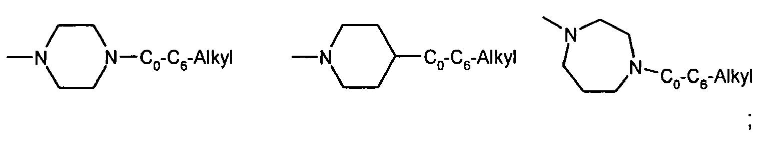
or
independently of one another hydroxy-C₁-C₆-alkylene, hydroxy-C₃-C₆-alkenylene, hydroxy-C₃-C₆-alkynylene, C₁-C₆-alkyloxy, C₃-C₇-cycloalkyl-oxy, C₃-C₇-cycloalkyl-C₁-C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, phenyloxy-C₁-C₆-alkylene,
C₁-C₆-alkylamino, di(C₁-C₆-alkyl)amino, C₁-C₆-alkylamino-C₁-C₆-alkylene, di(C₁-C₆)-alkylamino-C₁-C₆-alkylene, C₃-C₇-cycloalkyl-(C₀-C₆-alkyl)amino,
C₁-C₆-acyl-(C₀-C₆-alkyl)amido, C₁-C₆-alkylaminocarbonyl, di(C₁-C₆-alkyl)aminocarbonyl, (C₃-C₇-cycloalkyl)aminocarbonyl, di(C₃-C₇-cyclo-alkyl)aminocarbonyl, C₃-C₇-cycloalkyl-C₁-C₆-alkyleneaminocarbonyl, C₁-C₆-alkylcarbonyl, C₃-C₇-cycloalkylcarbonyl, carboxy, carboxamido [-C(O)NH₂], C₁-C₆-alkyloxycarbonyl, C₁-C₃-alkylsulphanyl, C₁-C₆-alkysulphonyl, C₃-C₇-cycloalkylsulphonyl, C₃-C₇-cycloalkyl-C₁-C₆-alkylenesulphonyl,
C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido, -N(C₀-C₆-alkyl)-C(O)-C₁-C₆-alkyl, -N(C₀-C₆-alkyl)-C(O)-C₃-C₇-cycloalkyl, -N(C₀-C₆-alkyl)-C(O)-N-di(C₀-C₆-alkyl), -N(C₀-C₆-alkyl)-C(O)-O-(C₀-C₆)alkyl, -N(C₀-C₆-alkyl)-C(O)-NH-C₃-C₇-cycloalkyl,
-N(C₀-C₆-alkyl)-SO₂-C₁-C₆-alkyl, -N(C₀-C₆-alkyl)-SO₂-C₃-C₇-cycloalkyl, -N(C₀-C₆-alkyl)-SO₂-N-di(C₀-C₆-alkyl), -N(C₀-C₆-alkyl)-SO₂-NH-(C₃-C₇)-cycloalkyl,
-C(O)-N(H)-C₂-C₆-alkylene-(C₁-C₆-alkyl)amine, -C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycloalkyl-C₁-C₆-alkyl)amine,
-S(O₂)-N(H)-C₂-C₆-alkylene-(C₁-C₆-alkyl)amine, -S(O₂)-N(H)-C₂C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine,
-O-C₂-C₆-alkylene-(C₁-C₆-alkyl)amine, -O-C₂-C₆-alkylene-[di(C₁-C₆-alkylene)]amine,
or the groups: 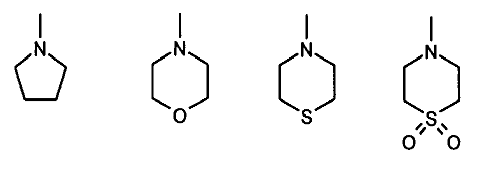 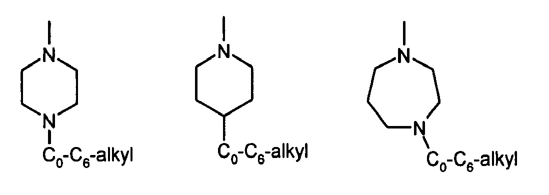 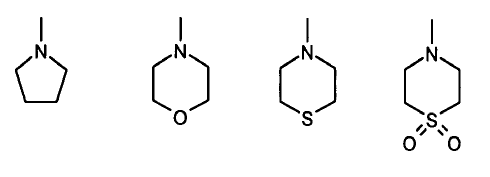 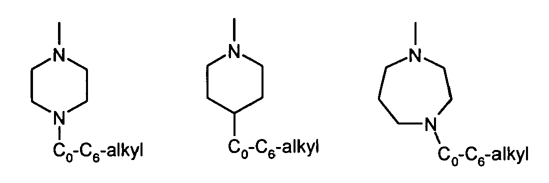 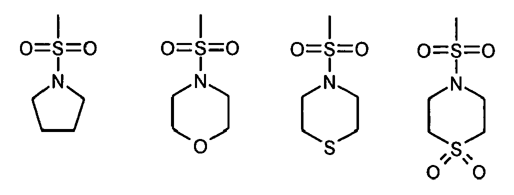 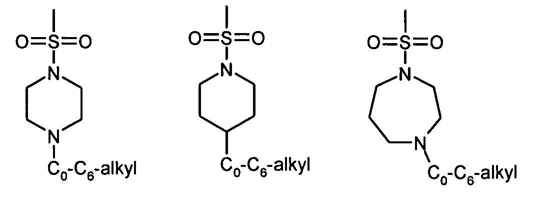 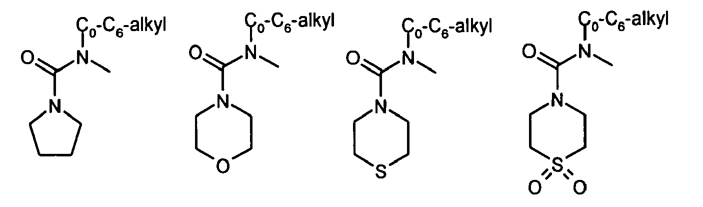 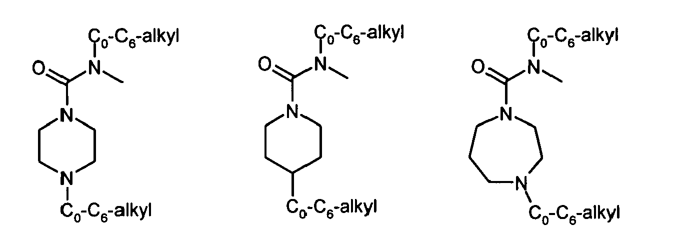 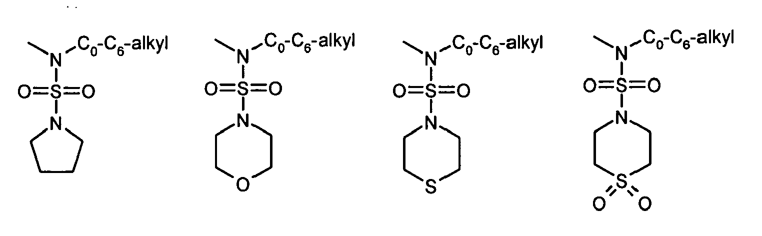 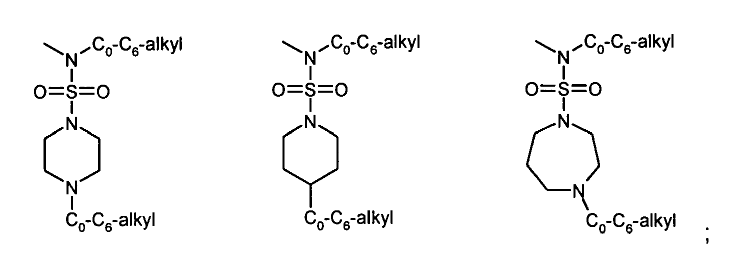
- R7, R8: may be independently of one another hydrogen, methyl, ethyl, where the methyl and ethyl radicals may be fluorinated one or more times;
wherein
- R2: may substitute one or more positions of the aryl or heteroaryl ring in the indole residue;
- R3: may substitute one or more positions of the monocyclic aryl or mono-cyclic heteroaryl ring of Q and/or V;
- R5 and R6: may together form a heterocycloalkyl or cycloalkyl group;
- Q: is a monocyclic aryl or a monocyclic heteroaryl group;
- V: is a cycloalkylen, cycloalkenylen, heterocycloalkylen or heterocycloalkenylen group;
- W: is an aryl or heteroaryl group;
- X: is a bond, C₁-C₄-alkylene, C₂-C₄-alkenylene or C₂-C₄-alkynylene group.

The present invention relates to both possible enantiomerics of compounds of the formula I with respect to the chiral centre in the tryptophanol moiety.

The unbranched C₁-C₆-alkyl groups for the radicals R1 to R6 may be for example a methyl, ethyl, propyl, butyl, pentyl or a hexyl group; and the branched C₃-C₆-alkyl groups for the radicals R1 to R6 may be an isopropyl, isobutyl, *sec*-butyl, *tert*-butyl, isopentyl, 2-methylbutyl, 1-methylbutyl, 1-ethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or a 1,2-dimethylbutyl group.

The branched or unbranched C₃-C₆-alkenyl groups for the radical R1 may be for example an allyl, (*E*)-2-methylviny), (*Z*)-2-methylvinyl, homoallyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (*Z*)-3-methylpent-3-enyl, (*E*)-2-methylpent-3-enyl, (*Z*)-2-methy)pent-3-enyl, (*E*)-1-methylpent-3-enyl, (*Z*)-1-methylpent-3-enyl, (*E*)-4-methylpent-2-enyl, (*Z*)-4-methylpent-2-enyl, (*E*)-3-methylpent-2-enyl, (*Z*)-3-methylpent-2-enyl, (*E*)-2-methylpent-2-enyl, (*Z*)-2-methylpent-2-enyl, (*E*)-1-methylpent-2-enyl, (Z)-1-methylpent-2-enyl, (*E*)-4-methylpent-1-enyl, (*Z*)-4-methylpent-1-enyl, (*E*)-3-methylpent-1-eny), (*Z*)-3-methylpent-1-enyl, (*E*)-2-methylpent-1-enyl, (*Z*)-2-methylpent-1-enyl, (*E*)-1-methylpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (Z)-3-ethylbut-2-enyl, (*E*)-2-ethylbut-2-enyl, (*Z*)-2-ethylbut-2-enyl, (*E*)-1-ethylbut-2-enyl, (Z)-1-ethylbut-2-enyl, (*E*)-3-ethylbut-1-enyl, (*Z*)-3-ethylbut-1-enyl, 2-ethylbut-1-enyl, (*E*)-1-ethylbut-1-enyl, (Z)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propylprop-1-enyl, (*Z*)-1-propylprop-1-enyl, (*E*)-2-isopropylprop-1-enyl, (*Z*)-2-isopropylprop-1-enyl, (*E*)-1-isopropylprop-1-enyl, (*Z*)-1-isopropylprop-1-enyl, (*E*)-3,3-dimethylprop-1-enyl, (*Z*)-3,3-dimethylprop-1-enyl- or a 1-(1,1-dimethylethyl)ethenyl group.

The C₃-C₆-alkynyl groups for the radical R1 may be for example a prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or a 3,3-dimethylbut-1-ynyl group.

The C₂-C₆-alkenyl groups for the radicals R2 to R6 may, in addition to the C₃-C₆-alkenyl groups mentioned for the radical R1, be for example a vinyl group.

The C₂-C₆-alkynyl groups for the radicals R2 to R6 may, in addition to the C₃-C₆-alkynyl groups mentioned for the radical R1, be for example an ethynyl group. The C₁-C₆-alkyloxy groups for the radicals R2 to R6 may be for example a methyloxy, ethyloxy, propyloxy, isopropyloxy, butyloxy, isobutyloxy, sec-butyloxy, tert-butyloxy, pentyloxy, isopentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1,3-dimethylbutyl)oxy or a (1,2-dimethylbutyl)oxy group.

The halogens for the radicals R2 to R6 are fluorine, chlorine, bromine or iodine.

The C₁-C₃-alkylsulphanyl groups for the radicals R4 to R6 may be for example a methylsulphanyl (CH₃S-), ethylsulphanyl (CH₃CH₂S-), propylsulphanyl, isopropylsulphanyl group.

The C₁-C₆-alkylaminocarbonyl groups for the radicals R4 to R6 may be for example a methylaminocarbonyl-, ethylaminocarbonyl-, propylaminocarbonyl-, isopropylaminocarbonyl-, butylaminocarbonyl-, *iso*butylaminocarbonyl-, sec-butylaminocarbonyl-, *tert*butylaminocarbonyl-, pentylaminocarbonyl-, *iso*pentylaminocarbonyl-, (2-methylbutyl)-aminocarbonyl-, (1-methylbutyl)aminocarbonyl-, (1-ethylpropyl)aminocarbonyl-, *neo*pentylaminocarbonyl-, (1,1-dimethylpropyl)aminocarbonyl-, hexylaminocarbonyl-, (4-methylpentyl)aminocarbonyl-, (3-methylpentyl)aminocarbonyl-, (2-methylpentyl)aminocarbonyl-, (1-methylpentyl)aminocarbonyl-, (1-ethylbutyl)aminocarbonyl-, (2-ethylbutyl)-aminocarbonyl-, (3,3-dimethylbutyl)aminocarbonyl-, (2,2-dimethylbutyl)aminocarbonyl-, (1,1-dimethylbutyl)aminocarbonyl-, (2,3-dimethylbutyl)aminocarbonyl-, (1,3-dimethylbutyl)aminocarbonyl- or a (1,2-dimethylbutyl)aminocarbonyl group.
The hydroxy-C₁-C₆-alkylene groups for the radicals R3 to R6 may be a hydroxymethyl (HOCH₂-), 2-hydroxyethyl (HOCH₂CH₂-), 1-hydroxyethyl [CH₃CH(OH)-], 3-hydroxypropyl (HOCH₂CH₂CH₂-), 2-hydroxypropyl [CH₃CH(OH)CH₂-], 1-hydroxypropyl [CH₃CH₂CH(OH)-], 2-hydroxy-1-methylethyl [HOCH₂CH(CH₃)-], 1-hydroxy-1-methylethyl [(CH₃)₂C(OH)-], 4-hydroxybutyl (HOCH₂CH₂CH₂CH₂-), 3-hydroxybutyl [CH₃CH(OH)CH₂CH₂-], 2-hydroxybutyl [CH₃CH₂CH(OH)CH₂-], 1-hydroxybutyl [CH₃CH₂CH₂CH(OH)-], 3-hydroxy-1-methylpropyl [HOCH₂CH₂CH(CH₃)-], 2-hydroxy-1-methylpropyl [CH₃CH(OH)CH(CH₃)-], 1-hydroxy-1-methylpropyl [CH₃CH₂C(CH₃)(OH)-], 1-(hydroxymethyl)propyl [CH₃CH(CH₂OH)-], 3-hydroxy-2-methylpropyl [HOCH₂CH(CH₃)CH₂-], 2-hydroxy-2-methylpropyl [(CH₃)₂C(OH)CH₂-], 1-hydroxy-2-methylpropyl [CH₃CH(CH₃)CH(OH)-] or a 2-hydroxy-1,1-dimethylethyl group [HOCH₂C(CH₃)₂-].

The heterocycloalkyl groups which may be formed by the groups R5 and R6 together include for example the following (biradical) groups:

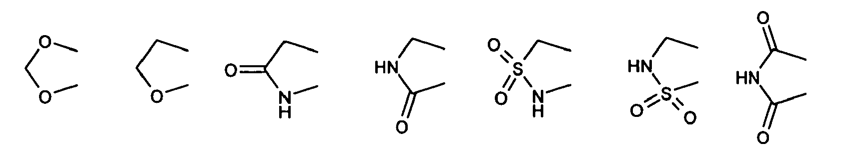

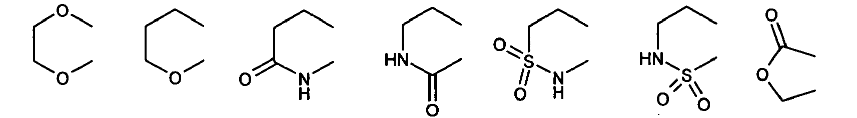

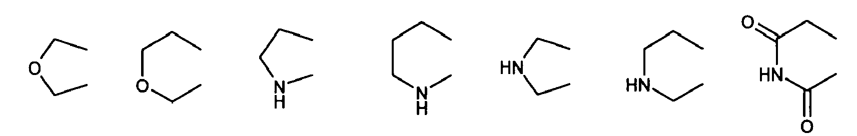

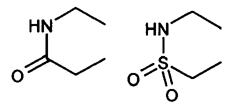

The cycloalkyl groups which may be formed by the groups R5 and R6 together include for example the following (biradical) groups:

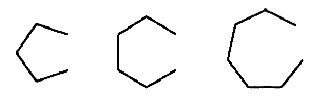

The C₃-C₇-cycloalkyl groups for the groups R1 to R6 may be for example a cyclopropyl, cyclobutyl, cyclopentyl, cyclohexyl, cycloheptyl group.

The C₃-C₇-heterocycloalkyl groups for the radicals R1 to R6 may be for example a cyclopropyl, cyclobutyl, cycopentyl, cyclohexyl, cycloheptyl group in which one or two carbon atoms of the ring are replaced independently of one another by an oxygen, nitrogen or sulphur atom.

The monocyclic aryl group for Q may be for example a phenyl group which is linked via substitutable positions

The aryl group for W may be for example a phenyl, naphthyl group which is linked via substitutable positions.

The monocylic heteroaryl group for Q may be for example a pyridinyl, pyrimidinyl, furanyl, thienyl, oxazolyl, isoxazolyl, thiazolyl, pyrrolyl, pyrazolyl or an imidazolyl group which is linked via substitutable positions.

The heteroaryl group for W may be for example a pyridinyl, pyrimidinyl, quinolinyl, isoquinolinyl, quinazolinyl, quinoxalinyl, phthalazinyl, 1.5-naphthyridinyl, 1,6-naphthyridinyl, 1,7-naphthyridinyl, 1,8-naphthyridinyl, benzofuranyl, benzothienyl, 1,3-benzodioxolyl, 2,1,3-benzothiadiazolyl, indolyl, indazolyl, furanyl, thienyl, oxazolyl, isoxazolyl, thiazolyl, pyrrolyl, pyrazolyl or an imidazolyl group which is linked via substitutable positions.

The heterocycloalkylen groups for V may be for example the following groups:

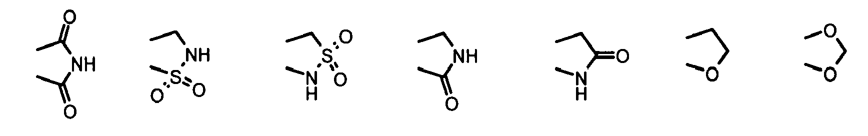

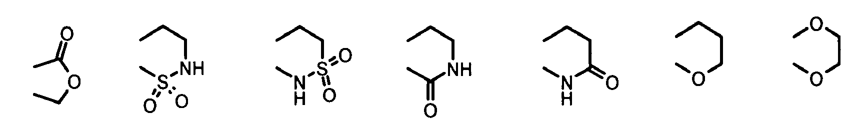

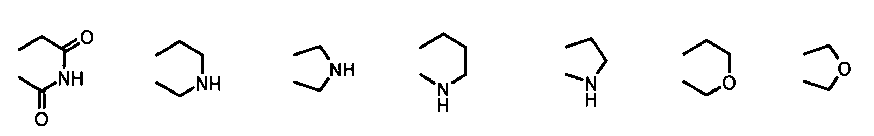

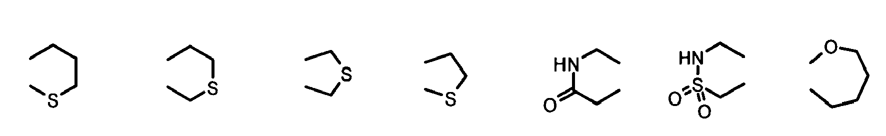

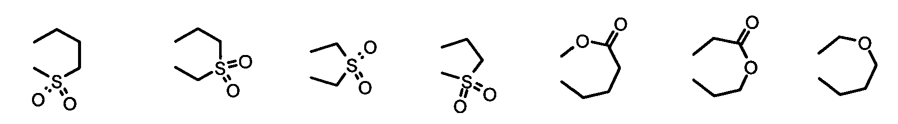

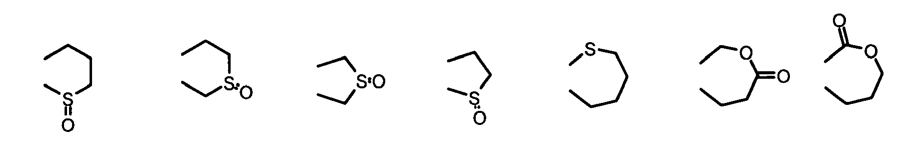

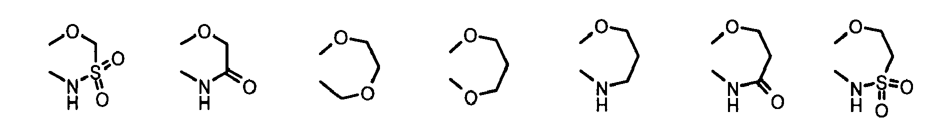

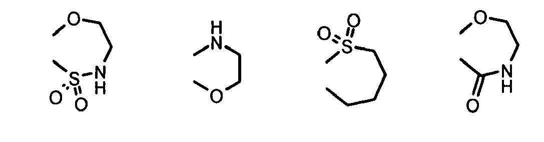

The heterocycloalkenylen groups for V may be for example the following groups:

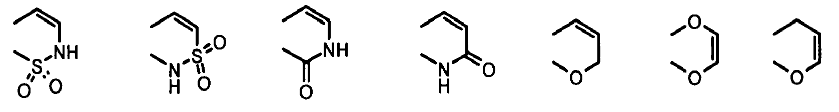

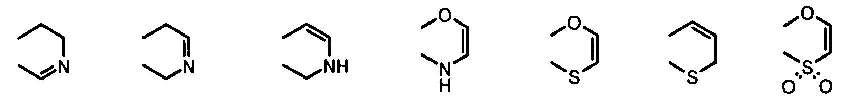

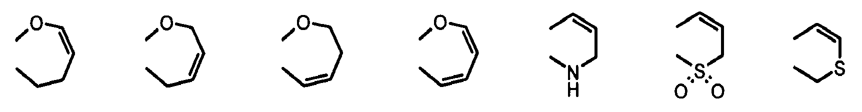

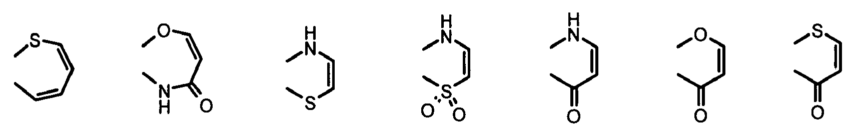

The cycloalkylen groups for V may be for example the following groups:

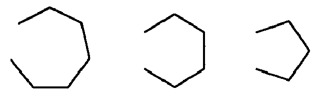

The cycloalkenylen groups for V may be for example the following groups:

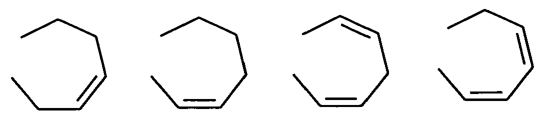

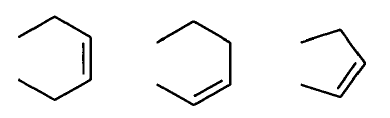

The C₁-C₄-alkylene groups for X and Y may be for example a methylene (-CH₂-), ethylidene [-CH(CH₃)-], ethylene (-CH₂CH₂-), prop-1,3-ylene (-CH₂CH₂CH₂-), prop-1,2-ylene [-CH₂CH(CH₃)-], but-1,4-ylene (-CH₂CH₂CH₂CH₂-), but-1,3-ylene [-CH₂CH₂CH(CH₃)-], but-1,2-ylene [-CH₂CH(CH₂CH₃)-], but-2,3-ylene [-CHCH(CH₃)-], 2-methylprop-1,2-ylene [-CH₂C(CH₃)₂-] or a 2-methylprop-1,3-ylene group [-CH₂CH(CH₃)CH₂-].

The C₂-C₄-alkenylene groups for X may be for example an ethen-1,2-ylidene (-CH=CH-), prop-2-en-1,3-ylidene (-CH₂-CH=CH-), prop-1-en-1,3-ylidene (-CH=CH-CH₂-), but-1-en-1,4-ylidene (-CH=CH-CH₂-CH₂-), but-2-en-1,4-ylidene (-CH₂-CH=CH-CH₂-) or a but-3-en-1,4-ylidene group (-CH₂-CH₂-CH=CH-).

The C₂-C₄-alkynylene groups for X may be for example an ethyn-1,2-ylidene (-C≡C-), prop-2-yn-1,3-ylidene (-CH₂-C≡C-), prop-1-yn-1,3-ylidene (-C≡C-CH₂-), but-1-yn-1,4-ylidene (-C≡C-CH₂-CH₂-), but-2-yn-1,4-ylidene (-CH₂-C≡C-CH₂-) or a but-3-yn-1,4-ylidene group (-CH₂-CH₂-C≡C-).

The C₃-C₇-cycloalkyloxy groups for the groups R1 to R6 may be for example a cyclopropyloxy, cyclobutyloxy, cyclopentyloxy, cyclohexyloxy, cycloheptyloxy group.

The C₁-C₆-alkylamino groups for the groups R1 to R6 may be for example methylamino, ethylamino, propylamino, isopropylamino, butylamino, isobutylamino, sec-butylamino, *tert*-butylamino, pentylamino, isopentylamino, (2-methylbutyl)amino, (1-methylbutyl)amino, (1-ethylpropyl)amino, *neo*pentylamino, (1,1-dimethylpropyl)amino, hexylamino, (4-methylpentyl)amino, (3-methylpentyl)amino, (2-methylpentyl)amino, (1-methylpentyl)amino, (1-ethylbutyl)amino, (2-ethylbutyl)amino, (3,3-dimethylbutyl)amino, (2,2-dimethylbutyl)amino, (1,1-dimethylbutyl)amino, (2,3-dimethylbutyl)amino, (1,3-dimethylbutyl)amino or a (1,2-dimethylbutyl)amino group.

Within the di(C₁-C₆-alkyl)amino groups for the groups R1 to R6, each of the two C₁-C₆-alkyl substituents may be chosen independently of one another from the following groups: possible examples are a methyl, ethyl, propyl, isopropyl, butyl, isobutyl, sec-butyl, *tert*-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the C₃-C₇-cycloalkyl-C₁-C₆-alkyleneoxy groups for the radicals R1 to R6 it is possible to combine each of the C₃-C₇-cycloalkyl groups of the C₃-C₇-cycloalkyl-C₁-C₆-alkyleneoxy group, for example of a cyclopropyl, cyclobutyl, cyclopentyl, cyclohexyl or cycloheptyl group, independently of one another with each C₁-C₆-alkyleneoxy group, for example with a methyleneoxy, ethyleneoxy, propyleneoxy, butyleneoxy, pentyleneoxy, hexyleneoxy group.

In the hydroxy-C₃-C₆-alkenylene groups for the radicals R1 to R6 it is possible for the hydroxy group to be located on any desired position of the C₃-C₆-alkenyl group, for example of an allyl, (*E*)-2-methylvinyl, (*Z*)-2-methylvinyl, homoallyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-Pent-2-enyl-, (*Z*)-Pent-2-enyl-, (*E*)-Pent-1-enyl-, (*Z*)-Pent-1-enyl-, hex-5-enyl-, (*E*)-hex-4-enyl, (*Z*)-hex-4-enyl, (*E*)-hex-3-enyl, (Z)-hex-3-enyl, (*E*)-hex-2-enyl, (Z)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (*Z*)-3-methylpent-3-enyl, (*E*)-2-methylpent-3-enyl, (*Z*)-2-methylpent-3-enyl, (*E*)-1-methylpent-3-enyl, (*Z*)-1-methylpent-3-enyl, (*E*)-4-methylpent-2-enyl, (*Z*)-4-methylpent-2-enyl, (*E*)-3-methylpent-2-enyl, (*Z*)-3-methylpent-2-enyl, (*E*)-2-methylpent-2-enyl, (*Z*)-2-methylpent-2-enyl, (*E*)-1-methylpent-2-enyl, (Z)-1-methylpent-2-enyl, (*E*)-4-methylpent-1-enyl, (*Z*)-4-methylpent-1-enyl, (*E*)-3-methylpent-1-enyl, (*Z*)-3-methylpent-1-enyl, (*E*)-2-methylpent-1-enyl, (*Z*)-2-methylpent-1-enyl, (*E*)-1-methylpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (*Z*)-3-ethylbut-2-enyl, (*E*)-2-ethylbut-2-enyl, (*Z*)-2-ethylbut-2-enyl, (*E*)-1-ethylbut-2-enyl, (*Z*)-1-ethylbut-2-enyl, (*E*)-3-ethylbut-1-enyl, (*Z*)-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propylprop-1-enyl, (*Z*)-1-propylprop-1-enyl, (*E*)-2-isopropylprop-1-enyl, (*Z*)-2-isopropylprop-1-enyl, (*E*)-1-isopropylprop-1-enyl, (*Z*)-1-isopropylprop-1-enyl, (*E*)-3,3-dimethylprop-1-enyl, (*Z*)-3,3-dimethylprop-1-enyl or a 1-(1,1-dimethylethyl)ethenyl group, and to be combined independently of one another.

In the hydroxy-C₃-C₆-alkynyl groups for the radicals R1 to R6 it is possible for the hydroxy group to be located at any desired position of the C₃-C₆-alkynyl group, for example of a prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or a 3,3-dimethylbut-1-ynyl group.

In the C₁-C₆-alkyloxy-C₃-C₆-alkenylene groups for the radicals R1 to R6 it is possible for the C₁-C₆-alkyloxy group, for example a methyloxy, ethyloxy, propyloxy, isopropyloxy, butyloxy, isobutyloxy, sec-butyloxy, *tert*-butyloxy, pentyloxy, isopentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)oxy, (1,3-dimethylbutyl)oxy or a (1,2-dimethylbutyl)oxy group, to be located on any desired position of the C₃-C₆-alkenyl group, for example of an allyl, (*E*)-2-methylvinyl, (*Z*)-2-methylvinyl, homoallyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (E)-hex-4-enyl, (*Z*)-hex-4-enyl, (*E*)-hex-3-enyl, (Z)-hex-3-enyl, (*E*)-hex-2-enyl, (Z)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (Z)-1-methylbut-1-enyl, 1,1-dimethylprop-2-enyl, 1-ethylprop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (*Z*)-3-methylpent-3-enyl, (*E*)-2-methylpent-3-enyl, (*Z*)-2-methylpent-3-enyl, (*E*)-1-methylpent-3-enyl, (*Z*)-1-methylpent-3-enyl, (*E*)-4-methylpent-2-enyl, (Z)-4-methylpent-2-enyl, (*E*)-3-methylpent-2-enyl, (Z)-3-methylpent-2-enyl, (*E*)-2-methylpent-2-enyl, (Z)-2-methylpent-2-enyl, (*E*)-1-methylpent-2-enyl, (*Z*)-1-methylpent-2-enyl, (*E*)-4-methylpent-1-enyl, (Z)-4-methylpent-1-enyl, (*E*)-3-methylpent-1-enyl, (*Z*)-3-methylpent-1-enyl, (*E*)-2-methylpent-1-enyl, (*Z*)-2-methylpent-1-enyl, (*E*)-1-methylpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (*Z*)-3-ethylbut-2-enyl, (*E*)-2-ethylbut-2-enyl, (*Z*)-2-ethylbut-2-enyl, (*E*)-1-ethy)but-2-enyt, (*Z*)-1-ethylbut-2-enyl, (*E*)-3-ethylbut-1-enyl, (*Z*)-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (Z)-2-propylprop-1-enyl, (*E*)-1-propylprop-1-enyl, (Z)-1-propylprop-1-enyl, (*E*)-2-isopropylprop-1-enyl, (*Z*)-2-isopropylprop-1-enyl, (*E*)-1-isopropylprop-1-enyl, (*Z*)-1-isopropylprop-1-enyl, (*E*)-3,3-dimethylprop-1-enyl, (*Z*)-3,3-dimethylprop-1-enyl or a 1-(1,1-dimethylethyl)ethenyl group and to be combined independently of one another.

In the C₁-C₆-alkyloxy-C₃-C₆-alkynylene groups for the radicals R1 to R6 it is possible for the C₁-C₆-alkyloxy group, for example a methyloxy, ethyloxy, propyloxy, isopropyloxy, butyloxy, *isobutyloxy, sec*-butyloxy, *tert*-butyloxy, pentyloxy, *isopentyloxy,* (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)oxy, (1,3-dimethylbutyl)oxy or a (1,2-dimethylbutyl)oxy group, to be located at any desired position of the C3-C6-alkynyl group, for example of a prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or a 3,3-dimethylbut-1-ynyl group, and to be combined independently of one another.

In the C₁-C₆-alkyloxyphenyl-C₁-C₆-alkylene groups for the radical R1 to R6 it is possible for the C₁-C₆-alkyloxy group to be selected independently of one another from methyloxy, ethyloxy, propyloxy, isopropyloxy, butyloxy, isobutyloxy, sec-butyloxy, *tert*-butyloxy, pentyloxy, isopentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1,3-dimethylbutyl)oxy or a (1,2-dimethylbutyl)oxy, and to be combined independently of one another with C₁-C₆-alkylene groups such as, for example, methylene, ethylene, propylene, butylene, pentylene, hexylene.

In the C₃-C₇-cycloalkyl-(C₀-C₆)-alkyleneamino groups of the radicals R3 to R6 it is possible for each of the C₃-C₇-cycloalkyl groups of the C₃-C₇-cycloalkyl-(C₀-C₆)-alkyleneamino group, for example of a cyclopropyl, cyclobutyl, cyclopentyl, cyclohexyl or cycloheptyl group, to be combined independently of one another with each C0-C6-alkylene group, for example with a bond, a methylene, ethylene, propylene, butylene, pentylene, hexylene group.

In the C₁-C₆-alkyloxy-C₁-C₆-alkylene groups for the radical R1 to R6, it is possible for the C₁-C₆-alkyloxy group to be selected independently for example from methyloxy, ethyloxy, propyloxy, isopropyloxy, butyloxy, isobutyloxy, *sec*-butyloxy, *tert*-butyloxy, pentyloxy, isopentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1,3-dimethylbutyl)oxy or a (1,2-dimethylbutyl)oxy and to be combined independently of one another with C₁-C₆-alkylene groups such as, for example, methylene, ethylene, propylene, butylene, pentylene, hexylene.

In the di(C₁-C₆-alkyl)amino-C₁-C₆-alkylene group for the radical R1 it is possible for each of the two radicals on the nitrogen atom of the amino group to be selected independently for example from methyl, ethyl, propyl, isopropyl, butyl, isobutyl, *sec*-butyl, *tert*-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, and to be combined with C₁-C₆-alkylene groups such as, for example, methylene, ethylene, propylene, butylene, pentylene, hexylene.

The C₃-C₇-cycloalkyl-C₁-C₆-alkylene groups for the radicals R1 to R6 may be for example a cyclopropyloxymethylene, cyclopropyloxyethylene, cyclopropyloxypropylene, cyclopropyloxybutylene, cyclopropyloxypentylene, cyclopropyloxyhexylene, cyclobutyloxymethylene, cyclobutyloxyethylene, cyclobutyloxypropylene, cyclobutyloxybutylene, cyclobutyloxypentylene, cyclobutyloxyhexylene, cyclopentyloxymethylene, cyclopentyloxyethylene, cyclopentyloxypropylene, cyclopentyloxybutylene, cyclopentyloxypentylene, cyclopentyloxyhexylene, cyclohexyloxymethylene, cyclohexyloxyethylene, cyclohexyloxypropylene, cyclohexyloxybutylene, cyclohexyloxypentylene, cyclohexyloxyhexylene, cycloheptyloxymethylene, cycloheptyloxyethylene, cycloheptyloxypropylene, cycloheptyloxybutylene, cycloheptyloxypentylene, cycloheptyloxyhexylen group.

In the C₁-C₆-alkylamino-C₁-C₆-alkylene groups for the radicals R1 to R6 it is possible for the C₁-C₆-alkylamino group to be selected independently for example from methylamino, ethylamino, propylamino, *iso*propylamino, butylamino, *iso*butylamino, *sec*-butylamino, *tert*-butylamino, pentylamino, *iso*pentylamino, (2-methylbutyl)amino, (1-methylbutyl)amino, (1-ethylpropyl)amino, *neopentylamino,* (1,1-dimethylpropyl)amino, hexylamino, (4-methylpentyl)amino, (3-methylpentyl)amino, (2-methylpentyl)amino, (1-methylpentyl)amino, (1-ethylbutyl)amino, (2-ethylbutyl)amino, (3,3-dimethylbutyl)amino, (2,2-dimethylbutyl)amino, (1,1-dimethylbutyl)amino, (2,3-dimethylbutyl)amino, (1,3-dimethylbutyl)amino or a (1,2-dimethylbutyl)amino and to be combined with C₁-C₆-alkylene groups such as, for example, methylene, ethylene, propylene, butylene, pentylene, hexylene.

The phenyloxy-C₁-C₆-alkylene groups for the radicals R1 to R6 may be for example a phenyloxymethyl, phenyloxyethyl, phenyloxypropyl, phenyloxybutyl, phenyloxypentyl, phenyloxyhexyl group.

In the C₁-C₆-acyl-(C₀-C₆-alkyl)amido groups for the radicals R4 to R6, it is possible for each of the C₁-C₆-acyl groups, for example a formyl, acetyl, propionyl, 2-methylpropionyl, 2,2-dimethylpropionyl, butyryl, 2-methylbutyryl, 3-methylbutyryl, 2,2-dimethylbutyryl, 2-ethylbutyryl, pentanoyl, 2-methylpentanoyl, 3-methylpentanoyl, 4-methylpentanoyl or a hexanoyl group, to be combined independently of one another with each (C₀-C₆-alkyl)amido group, for example a hydrogen atom, a methylamido, ethylamido, propylamido, isopropylamido, butylamido, isobutylamido, sec-butylamido, tert-butylamido, pentylamido, isopentylamido, (2-methylbutyl)amido, (1-methylbutyl)-amido, (1-ethylpropyl)amido, neopentylamido, (1,1-dimethylpropyl)amido, hexylamido, (4-methylpentyl)amido, (3-methylpentyl)amido, (2-methylpentyl)amido, (1-methylpentyl)-amido, (1-ethylbutyl)amido, (2-ethylbutyl)amido, (3,3-dimethylbutyl)amido, (2,2-dimethylbutyl)amido, (1,1-dimethylbutyl)amido, (2,3-dimethylbutyl)amido, (1,3-dimethylbutyl)amido or a (1,2-dimethylbutyl)amido group.

The C₁-C₆-alkylaminocarbonyl groups for the radicals R4 to R6 may be for example a methylaminocarbonyl, ethylaminocarbonyl, propylaminocarbonyl, isopropylaminocarbonyl, butylaminocarbonyl, isobutylaminocarbonyl, sec-butylaminocarbonyl, tert-butylaminocarbonyl, pentylaminocarbonyl, isopentylaminocarbonyl, (2-methylbutyl)-aminocarbonyl, (1-methylbutyl)aminocarbonyl, (1-ethylpropyl)aminocarbonyl, neopentylaminocarbonyl, (1,1-dimethylpropyl)aminocarbonyl, hexylaminocarbonyl, (4-methylpentyl)aminocarbonyl, (3-methylpentyl)aminocarbonyl, (2-methylpentyl)aminocarbonyl, (1-methylpentyl)aminocarbonyl, (1-ethylbutyl)aminocarbonyl, (2-ethylbutyl)aminocarbonyl, (3,3-dimethylbutyl)aminocarbonyl, (2,2-dimethylbutyl)-aminocarbonyl, (1,1-dimethylbutyl)aminocarbonyl, (2,3-dimethylbutyl)aminocarbonyl, (1,3-dimethylbutyl)aminocarbonyl or a (1,2-dimethylbutyl)aminocarbonyl group.

In the di(C₁-C₆-alkyl)aminocarbonyl groups for the radicals R4 to R6, each of the two C₁-C₆-alkyl radicals on the nitrogen atom of the di(C₁-C₆-alkyl)aminocarbonyl group may be independently of one another for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

The (C₃-C₇-cycloalkyl)aminocarbonyl groups for the radicals R4 to R6 may be for example a cyclopropylaminocarbonyl, cyclobutylaminocarbonyl, cyclopentylaminocarbonyl, cyclohexylaminocarbonyl or cycloheptylaminocarbonyl group.

In the di(C₃-C₇-cycloalkyl)aminocarbonyl groups for the radicals R4 to R6, each of the two C₃-C₇-cycloalkyl radicals on the nitrogen atom of the di(C₃-C₇-cycloalkyl)aminocarbonyl group may be independently of one another for example a cyclopropyl, cyclobutyl, cyclopentyl, cyclohexyl or cycloheptyl group.

In the C₃-C₇-cycloalkyl-C₁-C₆-alkyleneaminocarbonyl groups of the radicals R4 to R6 it is possible for each of the C₃-C₇-cycloalkyl groups of the C₃-C₇-cycloalkyl-C₁-C₆-alkyleneaminocarbonyl groups, for example of a cyclopropyl, cyclobutyl, cyclopentyl, cyclohexyl or cycloheptyl group, to be combined independently of one another with each C₁-C₆-alkyleneaminocarbonyl group, for example with a methyleneaminocarbonyl, ethyleneaminocarbonyl, propyleneaminocarbonyl, butyleneaminocarbonyl, pentyleneaminocarbonyl, hexyleneaminocarbonyl group.

The C₁-C₆-alkylcarbonyl groups for the radicals R4 to R6 may be for example a methylcarbonyl, ethylcarbonyl, propylcarbonyl, isopropylcarbonyl, butylcarbonyl, isobutylcarbonyl, sec-butylcarbonyl, tert-butylcarbonyl, pentylcarbonyl, isopentylcarbonyl, (2-methylbutyl)carbonyl, (1-methylbutyl)carbonyl, (1-ethylpropyl)carbonyl, neopentylcarbonyl, (1,1-dimethylpropyl)carbonyl, hexylcarbonyl, (4-methylpentyl)carbonyl, (3-methylpentyl)carbonyl, (2-methylpentyl)carbonyl, (1-methylpentyl)carbonyl, (1-ethylbutyl)-carbonyl, (2-ethylbutyl)carbonyl, (3,3-dimethylbutyl)carbonyl, (2,2-dimethylbutyl)-carbonyl, (1,1-dimethylbutyl)carbonyl, (2,3-dimethylbutyl)carbonyl, (1,3-dimethylbutyl)-carbonyl or a (1,2-dimethylbutyl)carbonyl group.

The C₃-C₇-cycloalkylcarbonyl groups for the radicals R4 to R6 may be for example a cyclopropylcarbonyl, cyclobutylcarbonyl, cyclopentylcarbonyl, cyclohexylcarbonyl or cycloheptylcarbonyl group.

The C₁-C₆-alkyloxycarbonyl groups for the radicals R4 to R6 may be for example a methyloxycarbonyl, ethyloxycarbonyl, propyloxycarbonyl, isopropyloxycarbonyl, butyloxycarbonyl, isobutyloxycarbonyl, sec-butyloxycarbonyl, tert-butyloxycarbonyl, pentyloxycarbonyl, isopentyloxycarbonyl, (2-methylbutyl)oxycarbonyl, (1-methylbutyl)-oxycarbonyl, (1-ethylpropyl)oxycarbonyl, neopentyloxycarbonyl, (1,1-dimethylpropyl)-oxycarbonyl, hexyloxycarbonyl, (4-methylpentyl)oxycarbonyl, (3-methylpentyl)oxycarbonyl, (2-methylpentyl)oxycarbonyl, (1-methylpentyl)oxycarbonyl, (1-ethylbutyl)oxycarbonyl, (2-ethylbutyl)oxycarbonyl, (3,3-dimethylbutyl)oxycarbonyl, (2,2-dimethylbutyl)-oxycarbonyl, (1,1-dimethylbutyl)oxycarbonyl, (2,3-dimethylbutyl)oxycarbonyl, (1,3-dimethylbutyl)oxycarbonyl or a (1,2-dimethylbutyl)oxycarbonyl group.

The C₁-C₆-alkylsulphonyl groups for the radicals R4 to R6 may be for example a methylsulphonyl, ethylsulphonyl, propylsulphonyl, isopropylsulphonyl, butylsulphonyl, isobutylsulphonyl, sec-butylsulphonyl, tert-butylsulphonyl, pentylsulphonyl, isopentylsulphonyl, (2-methylbutyl)sulphonyl, (1-methylbutyl)sulphonyl, (1-ethylpropyl)sulphonyl, neopentylsulphonyl, (1,1-dimethylpropyl)sulphonyl, hexylsulphonyl, (4-methylpentyl)-sulphonyl, (3-methylpentyl)sulphonyl, (2-methylpentyl)sulphonyl, (1-methylpentyl)-sulphonyl, (1-ethylbutyl)sulphonyl, (2-ethylbutyl)sulphonyl, (3,3-dimethylbutyl)sulphonyl, (2,2-dimethylbutyl)sulphonyl, (1,1-dimethylbutyl)sulphonyl, (2,3-dimethylbutyl)sulphonyl, (1,3-dimethylbutyl)sulphonyl or a (1,2-dimethylbutyl)sulphonyl group.

The C₃-C₇-cycloalkylsulphonyl groups for the radicals R4 to R6 may be for example a cyclopropylsulphonyl, cyclobutylsulphonyl, cyclopentylsulphonyl, cyclohexylsulphonyl or cycloheptylsulphonyl group.

In the C₃-C₇-cycloalkyl-C₁-C₆-alkylenesulphonyl groups of the radicals R4 to R6 it is possible for each of the C₃-C₇-cycloalkyl groups of the C₃-C₇-cycloalkyl-C₁-C₆-alkylenesulphonyl groups, for example of a cyclopropyl, cyclobutyl, cyclopentyl, cyclohexyl or cycloheptyl group, to be combined independently of one another with each C₁-C₆-alkylenesulphonyl group, for example with a methylenesulphonyl, ethylenesulphonyl, propylenesulphonyl, butylenesulphonyl, pentylenesulphonyl, hexylenesulphonyl group. The C₁-C₆-alkylaminosulphonyl groups for the radicals R4 to R6 may be for example a methylaminosulphonyl, ethylaminosulphonyl, propylaminosulphonyl, isopropylaminosulphonyl, butylaminosulphonyl, isobutylaminosulphonyl, sec-butylaminosulphonyl, tert-butylaminosulphonyl, pentylaminosulphonyl, isopentylaminosulphonyl, (2-methylbutyl)aminosulphonyl, (1-methylbutyl)aminosulphonyl, (1-ethylpropyl)aminosulphonyl, neopentylaminosulphonyl, (1,1-dimethylpropyl)aminosulphonyl, hexylaminosulphonyl, (4-methylpentyl)aminosulphonyl, (3-methylpentyl)aminosulphonyl, (2-methylpentyl)-aminosulphonyl, (1-methylpentyl)aminosulphonyl, (1-ethylbutyl)aminosulphonyl, (2-ethylbutyl)aminosulphonyl, (3,3-dimethylbutyl)aminosulphonyl, (2,2-dimethylbutyl)-aminosulphonyl, (1,1-dimethylbutyl)aminosulphonyl, (2,3-dimethylbutyl)aminosulphonyl, (1,3-dimethylbutyl)aminosulphonyl or a (1,2-dimethylbutyl)aminosulphonyl group.

In the di(C₁-C₆-alkyl)aminosulphonyl groups for the radicals R4 to R6, each of the two C₁-C₆-alkyl radicals on the nitrogen atom of the di(C₁-C₆-alkyl)aminosulphonyl group may be independently of one another for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

The (C₃-C₇-cycloalkyl)aminosulphonyl groups for the radicals R4 to R6 may be for example a cyclopropylaminosulphonyl, cyclobutylaminosulphonyl, cyclopentylaminosulphonyl, cyclohexylaminosulphonyl or cycloheptylaminosulphonyl group.

In the di(C₃-C₇-cycloalkyl)aminosulphonyl groups for the radicals R4 to R6, each of the two C₃-C₇-cycloalkyl radicals on the nitrogen atom of the di(C₃-C₇-cycloalkyl)aminosulphonyl group may be independently of one another for example a cyclopropyl, cyclobutyl, cyclopentyl, cyclohexyl or cycloheptyl group.

In the C₃-C₇-cycloalkyl-C₁-C₆-alkyleneaminosulphonyl groups of the radicals R4 to R6, each of the C₃-C₇-cycloalkyl groups of the C₃-C₇-cycloalkyl-C₁-C₆-alkyleneaminosulphonyl groups, for example of a cyclopropyl, cyclobutyl, cyclopentyl, cyclohexyl or cycloheptyl group, can be combined independently of one another with each C₁-C₆-alkyleneaminosulphonyl group, for example with a methyleneaminosulphonyl, ethyleneaminosulphonyl, propyleneaminosulphonyl, butyleneaminosulphonyl, pentyleneaminosulphonyl, hexyleneaminosulphonyl group.

The C₁-C₆-alkylsulphonylamido groups for the radicals R4 to R6 may be for example a methylsulphonylamido, ethylsulphonylamido, propylsulphonylamido, isopropylsulphonylamido, butylsulphonylamido, isobutylsulphonylamido, sec-butylsulphonylamido, tert-butylsulphonylamido, pentylsulphonylamido, isopentylsulphonylamido, (2-methylbutyl)-sulphonylamido, (1-methylbutyl)sulphonylamido, (1-ethylpropyl)sulphonylamido, neopentylsulphonylamido, (1,1-dimethylpropyl)sulphonylamido, hexylsulphonylamido, (4-methylpentyl)sulphonylamido, (3-methylpentyl)sulphonylamido, (2-methylpentyl)-sulphonylamido, (1-methylpentyl)sulphonylamido, (1-ethylbutyl)sulphonylamido, (2-ethylbutyl)sulphonylamido, (3,3-dimethylbutyl)sulphonylamido, (2,2-dimethylbutyl)-sulphonylamido, (1,1-dimethylbutyl)sulphonylamido, (2,3-dimethylbutyl)sulphonylamido, (1,3-dimethylbutyl)sulphonylamido or a (1,2-dimethylbutyl)sulphonylamido group.

In the -N(C₀-C₆-alkyl)-C(O)-C₁-C₆-alkyl groups of the radicals R4 to R6, each of the (C₀-C₆-alkyl) groups on the nitrogen atom of the -N(C₀-C₆-alkyl)-C(O)-C₁-C₆-alkyl groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₁-C₆-alkyl group on the carbonyl group of the amide, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N-(C₀-C₆-alkyl)-C(O)-C₃-C₇-cycloalkyl groups of the radicals R4 to R6, each of the (C₀-C₆-alkyl) groups on the nitrogen atom of the -N(C₀-C₆-alkyl)-C(O)-C₁-C₆-alkyl groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group on the carbonyl group of the amide, for example with a cyclopropyl, cyclobutyl, cyclopentyl, cyclohexyl or cycloheptyl group.

In the -N(C₀-C₆-alkyl)-C(O)-N-di(C₀-C₆-alkyl) groups of the radicals R4 to R6, all three (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-C(O)-O-(C₀-C₆-alkyl) groups of the radicals R4 to R6, both (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-C(O)-NH-(C₃-C₇-cycloalkyl) groups of the radicals R4 to R6, each of the (C₀-C₆-alkyl) groups on the nitrogen atom of the -N(C₀-C₆-alkyl)-C(O)-NH-(C₃-C₇-cycloalkyl) groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may independently of one another be combined with each C₃-C₇-cycloalkyl group on the terminal nitrogen atom of the urea, for example with a cyclopropyl, cyclobutyl, cyclopentyl, cyclohexyl or cycloheptyl group.

In the -N(C₀-C₆-alkyl)-SO₂-(C₁-C₆-alkyl) groups of the radicals R4 to R6, each of the (C₀-C₆-alkyl) groups on the nitrogen atom of the -N(C₀-C₆-alkyl)-SO₂-(C₁-C₆-alkyl) group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may independently of one another be combined with each C₁-C₆-alkyl group on the sulphonyl group of the sulphonamide, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-SO₂-C₃-C₇-cycloalkyl groups of the radicals R4 to R6, each of the (C₀-C₆-alkyl) groups on the nitrogen atom of the -N(C₀-C₆-alkyl)-SO₂-C₃-C₇-cycloalkyl group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group on the sulphonyl group, for example with a cyclopropyl, cyclobutyl, cyclopentyl, cyclohexyl or cycloheptyl group.

In the -N(C₀-C₆-alkyl)-SO₂-N-di(C₀-C₆-alkyl) groups of the radicals R4 to R6, all three (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-SO₂-NH-(C₃-C₇)-cycloalkyl groups of the radicals R4 to R6, the C₀-C₆-alkyl group of the -N(C₀-C₆-alkyl)-SO₂-NH-(C₃-C₇)-cycloalkyl group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group, for example with a cyclopropyl, cyclobutyl, cyclopentyl, cyclohexyl or cycloheptyl group.

In the -C(O)-N(H)-C₂-C₆-alkylene-(C₁-C₆-alkyl)amine groups of the radicals R4 to R6, each of the C₂-C₆-alkylene groups on the nitrogen atom of the -C(O)-N(H)-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -C(O)-N(H)-C₂-C₆-alkylene-[di(C₁-C₆-alkyl)]amine groups of the radicals R4 to R6, each of the C₂-C₆-alkylene groups on the nitrogen atom of the -C(O)-N(H)-C₂-C₆-alkylene-[di(C₁-C₆-alkyl)]amine group, for example an ethylene, propylene, butylene, pentylene or hexylene group, may be combined independently of one another with each of the two identically or different C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -C(O)-N(H)-C₂-C₆-alkylene-(C₃-C₇-cycloalkyl)amine groups of the radicals R4 to R6, each of the (C₂-C₆-alkylene) groups of the -C(O)-N(H)-C₂-C₆-alkylene-(C₃-C₇-cycloalkyl)amine group, for example an ethylene, propylene, butylene, pentylene or hexylene group, may be combined independently of one another with each C₃-C₇-cycloalkyl group on the amine, for example with a cyclopropyl, cyclobutyl, cyclopentyl, cyclohexyl or cycloheptyl group.

In the -C(O)-N(H)-C₂-C₆-alkylene-(C₃-C₇-cycloalkyl-C₁-C₆-alkylene)amine groups of the radicals R4 to R6, each of the (C₂-C₆-alkylene) groups of the -C(O)-N(H)-C2-C6-alkylene-(C3-C6-cycloalkyl-C1-C6-alkylene)amine group, for example an ethylene, propylene, butylene, pentylene or hexylene group, may be combined independently of one another with each C₃-C₇-cycloalkyl-C₁-C₆-alkylene group on the amine, for example with a cyclopropylmethylene, cyclopropylethylene, cyclopropylpropylene, cyclopropylbutylene, cyclopropylpentylene, cyclopropylhexylene, cyclobutylmethylene, cyclobutylethylene, cyclobutylpropylene, cyclobutylbutylene, cyclobutylpentylene, cyclobutylhexylene, cyclopentylmethylene, cyclopentylethylene, cyclopentylpropylene, cyclopentylhexylene, cyclohexylmethylene, cyclohexylethylene, cyclohexylpropylene, cyclohexylbutylene, cyclohexylpentylene, cyclohexylhexylene, cycloheptylmethylene, cycloheptylethylene, cycloheptylpropylene, cycloheptylbutylene, cycloheptylpentylene or cycloheptylhexylene group.

In the -S(O₂)-N(H)-C₂-C₆-alkylene-(C₁-C₆-alkyl)amine groups of the radicals R4 to R6, the (C₂-C₆-alkylene) groups of the -S(O₂)-N(H)-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -S(O₂)-N(H)-C₂-C₆-alkylene-[di(C₁-C₆-alkyl)]amine groups of the radicals R4 to R6, the C₂-C₆-alkylene group of the -S(O₂)-N(H)-C₂-C₆-alkylene-[di(C₁-C₆-alkyl)]amine group, for example an ethylene, propylene, butylene, pentylene or hexylene group, may be combined independently of one another with each of the two C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -S(O₂)-N(H)-C₂-C₆-alkylene-(C₃-C₇-cycloalkyl)amine groups of the radicals R4 to R6, the C₂-C₆-alkylene group of the -S(O₂)-N(H)-C₂-C₆-alkylene-(C₃-C₇-cycloalkyl)-amine group, for example an ethylene, propylene, butylene, pentylene or hexylene group, may be combined independently of one another with each C₃-C₇-cycloalkyl group on the amino group, for example with a cyclopropyl, cyclobutyl, cyclopentyl, cyclohexyl or cycloheptyl group.

In the -S(O₂)-N(H)-C₂-C₆-alkylene-(C₃-C₇-cycloalkyl-C₁-C₆-alkylene)amine groups of the radicals R4 to R6, each C₂-C₆-alkylene group of the -S(O₂)-N(H)-C₂-C₆-alkylene-(C₃-C₇-cycloalkyl-C₁-C₆-alkylene)amine group, for example an ethylene, propylene, butylene, pentylene or hexylene group, may be combined independently of one another with each C₃-C₇-cycloalkyl-C₁-C₆-alkylene group on the amine, for example with a cyclopropylmethylene, cyclopropylethylene, cyclopropylpropylene, cyclopropylbutylene, cyclopropylpentylene, cyclopropylhexylene, cyclobutylmethylene, cyclobutylethylene, cyclobutylpropylene, cyclobutylbutylene, cyclobutylpentylene, cyclobutylhexylene, cyclopentylmethylene, cyclopentylethylene, cyclopentylpropylene, cyclopentylhexylene, cyclohexylmethylene, cyclohexylethylene, cyclohexylpropylene, cyclohexylbutylene, cyclohexylpentylene, cyclohexylhexylene, cycloheptylmethylene, cycloheptylethylene, cycloheptylpropylene, cycloheptylbutylene, cycloheptylpentylen or cycloheptylhexylene group.

In the -O-C₂-C₆-alkylene-(C₁-C₆-alkyl)amine groups of the radicals R4 to R6, the C₂-C₆-alkylene group of the -O-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -O-C₂-C₆-alkylene-[di(C₁-C₆-alkyl)]amine groups of the radicals R4 to R6, the C₂-C₆-alkylene group of the -O-C₂-C₆-alkylene-[di(C₁-C₆-alkyl)]amine group, for example an ethylene, propylene, butylene, pentylene or hexylene group, may be combined independently of one another with two freely selectable C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

Compounds preferred according to the present invention are those of the formula II 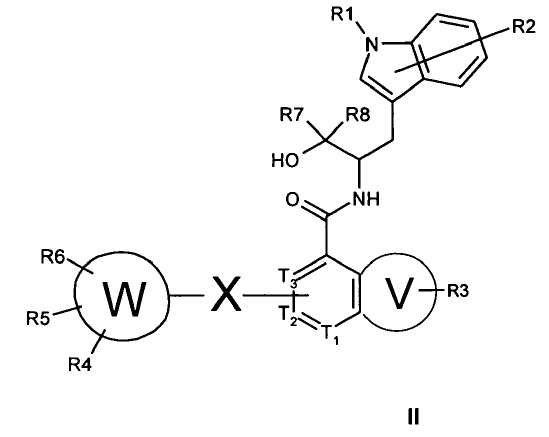 in which the radicals R1 to R8, X, V and W have the same meaning as in formula I
and
T1, T2, T3 are each independently of one another a nitrogen atom or an R3-C group.

Compounds particularly preferred according to the present invention are those of the formula III 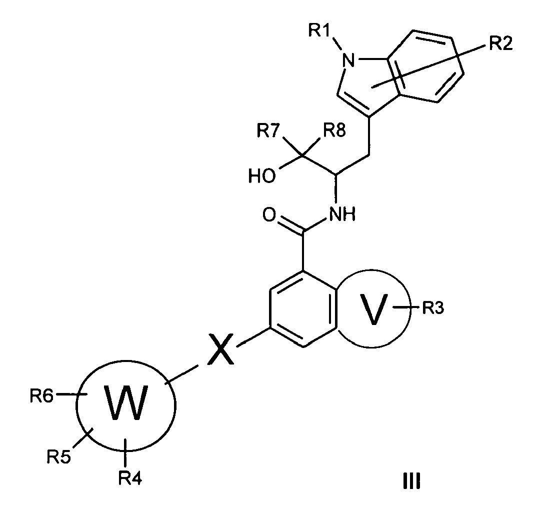 in which the radicals R1 to R8, X, V and W have the same meaning as in formula I.

The following compounds are very particularly preferred:
- **1**: 6-(3,4,5-Trimethoxy-phenyl)-2H-chromene-8-carboxylic acid [(R)-1-hydroxymethyl-2-(1H-indol-3-yl)-ethyl]-amide;
- **2**: 6-(3-Chloro-4-methylcarbamoyl-phenyl)-2H-chromene-8-carboxylic acid [(R)-2-hydroxy-1-(1H-indol-3-ylmethyl)-ethyl]-amide;
- **3**: 6-(3-Chloro-4-methylcarbamoyl-phenyl)-chroman-8-carboxylic acid [(R)-2-hydroxy-1-(1H-indol-3-ylmethyl)-ethyl]-amide;
- **4**: 6-o-Tolylethynyl-2H-chromene-8-carboxylic acid [(R)-1-hydroxymethyl-2-(1H-indol-3-yl)-ethyl]-amide;
- **5**: 6-(2-Methoxy-phenylethynyl)-2H-chromene-8-carboxylic acid [(R)-1-hydroxymethyl-2-(1H-indol-3-yl)-ethyl]-amide;
- **6**: 2-Methyl-6-(3,4,5-trimethoxy-phenyl)-2H-chromene-8-carboxylic acid [(R)-1-hydroxymethyl-2-(1H-indol-3-yl)-ethyl]-amide;
- **7**: 6-(3-Chloro-4-methylcarbamoyl-phenyl)-2,2-dimethyl-2H-chromene-8-carboxylic acid [(R)-2-hydroxy-1-(1H-indol-3-ylmethyl)-ethyl]-amide;
- **8**: 6-(3-Fluoro-5-methylcarbamoyl-phenyl)-2,2-dimethyl-2H-chromene-8-carboxylic acid [(R)-2-hydroxy-1-(1H-indol-3-ylmethyl)-ethyl]-amide;
- **9**: 2,2-Dimethyl-6-(3,4,5-trimethoxy-phenyl)-2H-chromene-8-carboxylic acid [(R)-2-hydroxy-1-(1H-indol-3-ylmethyl)-ethyl]-amide;
- **10**: 2,2-Dimethyl-6-(4-methylcarbamoyl-phenylethynyl)-2H-chromene-8-carboxylic acid [(R)-1-hydroxymethyl-2-(1H-indol-3-yl)-ethyl]-amide;
- **11**: 2,2-Dimethyl-6-(3-methylcarbamoyl-phenylethynyl)-2H-chromene-8-carboxylic acid [(R)-1-hydroxymethyl-2-(1H-indol-3-yl)-ethyl]-amide;
- **12**: 5-(3,4-Dimethoxy-phenyl)-2-vinyl-2,3-dihydro-benzofuran-7-carboxylic acid [(R)-1-hydroxymethyl-2-(1H-indol-3-yl)-ethyl]-amide;
- **13**: 5-(3,4-Dimethoxy-phenyl)-2-ethyl-2,3-dihydro-benzofuran-7-carboxylic acid [(R)-1-hydroxymethyl-2-(1H-indol-3-yl)-ethyl]-amide;
- **14**: 7-(3-Chloro-4-methylcarbamoyl-phenyl)-2,3-dihydro-benzo[1,4]dioxine-5-carboxylic acid [(R)-2-hydroxy-1-(1H-indol-3-ylmethyl)-ethyl]-amide;
- **15**: 7-(3,4,5-Trimethoxy-phenyl)-2,3-dihydro-benzo[1,4]dioxine-5-carboxylic acid [(R)-1-hydroxymethyl-2-(1H-indol-3-yl)-ethyl]-amide;
- **16**: 7-(3-Fluoro-5-methylcarbamoyl-phenyl)-2,3-dihydro-benzo[1,4]dioxine-5-carboxylic acid [(R)-2-hydroxy-1-(1H-indol-3-ylmethyl)-ethyl]-amide;
- **17**: 7-(3-Fluoro-5-methylcarbamoyl-phenyl)-2,3-dihydro-benzo[1,4]dioxine-5-carboxylic acid [2-(6-fluoro-1H-indol-3-yl)-1-hydroxymethyl-ethyl]-amide;
- **18**: 7-(3,4,5-Trimethoxy-phenyl)-2,3-dihydro-benzo-[1,4]dioxine-5-carboxylic acid [2-(6-fluoro-1H-indol-3-yl)-1-hydroxymethyl-ethyl]-amide;
- **19**: 7-(3-Chloro-4-methylcarbamoyl-phenyl)-2,3-dihydro-benzo[1,4]dioxine-5-carboxylic acid [2-(6-fluoro-1H-indol-3-yl)-1-hydroxymethyl-ethyl]-amide;
- **20**: 5-(3,4-Difluoro-5-methoxy-phenyl)-2,3-dihydro-benzofuran-7-carboxylic acid [(R)-2-hydroxy-1-(1H-indol-3-ylmethyl)-ethyl]-amide;
- **21**: 5-(3,4-Dimethoxy-phenyl)-2,3-dihydro-benzofuran-7-carboxylic acid [(R)-1-hydroxymethyl-2-(1H-indol-3-yl)-ethyl]-amide;
- **22**: 5-(3-Methoxy-phenyl)-2,3-dihydro-benzofuran-7-carboxylic acid [(R)-1-hydroxymethyl-2-(1H-indol-3-yl)-ethyl]-amide;
- **23**: 5-Benzo[1,3]dioxol-5-yl-2,3-dihydro-benzofuran-7-carboxylic acid [(R)-1-hydroxymethyl-2-(1H-indol-3-yl)-ethyl]-amide;
- **24**: 5-(3-Fluoro-4-methoxy-phenyl)-2,3-dihydro-benzofuran-7-carboxylic acid [(R)-2-hydroxy-1-(1H-indol-3-ylmethyl)-ethyl]-amide;
- **25**: 5-(3,4,5-Trimethoxy-phenyl)-2,3-dihydro-benzofuran-7-carboxylic acid [(R)-1-hydroxymethyl-2-(1H-indol-3-yl)-ethyl]-amide;
- **26**: 7-(3-Chloro-4-methylcarbamoyl-phenyl)-2,3,4,5-tetrahydro-benzo[b]oxepine-9-carboxylic acid [(R)-2-hydroxy-1-(1H-indol-3-ylmethyl)- ethyl]-amide;
- **27**: 7-(4-Carbamoyl-3-chloro-phenyl)-2,3,4,5-tetrahydro-benzo[b]oxepine-9-carboxylic acid [(R)-2-hydroxy-1-(1H-indol-3-ylmethyl)-ethyl]-amide;
- **28**: 7-[3-Chloro-4-(morpholine-4-carbonyl)-phenyl]-2,3,4,5-tetrahydro-benzo[b]oxepine-9-carboxylic acid [(R)-2-hydroxy-1-(1H-indol-3-ylmethyl)-ethyl]-amide;
- **29**: 7-(3-Chloro-4-methylcarbamoyl-phenyl)-2,3,4,5-tetrahydro-benzo[b]oxepine-9-carboxylic acid [1-(6-fluoro-1H-indol-3-ylmethyl)-2-hydroxy-ethyl]-amide;
- **30**: 7-(4-Carbamoyl-3-chloro-phenyl)-2,3,4,5-tetrahydro-benzo[b]oxepine-9-carboxylic acid [1-(6-fluoro-1H-indol-3-ylmethyl)-2-hydroxy-ethyl]-amide;
- **31**: 7-[3-Chloro-4-(morpholine-4-carbonyl)-phenyl]-2,3,4,5-tetrahydro-benzo[b]oxepine-9-carboxylic acid [(R)-1-(6-fluoro-1H-indol-3-ylmethyl)-2-hydroxyethyl]-amide;
- **32**: 7-(3-Fluoro-5-methylcarbamoyl-phenyl)-2,3,4,5-tetrahydro-benzo[b]oxepine-9-carboxylic acid [2-(4-fluoro-1H-indol-3-yl)-1-hydroxymethyl-ethyl]-amide;
- **33**: 7-(3-Fluoro-5-methylcarbamoyl-phenyl)-2,3,4,5-tetrahydro-benzo[b]oxepine-9-carboxylic acid [2-(5-fluoro-1H-indol-3-yl)-1-hydroxymethyl-ethyl]-amide;
- **34**: 7-(3-Fluoro-5-methylcarbamoyl-phenyl)-2,3,4,5-tetrahydro-benzo[b]oxepine-9-carboxylic acid [2-(6-fluoro-1H-indol-3-yl)-1-hydroxymethyl-ethyl]-amide;
- **35**: 7-(3-Fluoro-5-methylcarbamoyl-phenyl)-2,3,4,5-tetrahydro-benzo[b]oxepine-9-carboxylic acid ((R)-2-hydroxy-1-(1H-indol-3-ylmethyl)-ethyl]-amide;
- **36**: 7-(4-Methylcarbamoyl-phenylethynyl)-2,3,4,5-tetrahydro-benzo[b]oxepine-9-carboxylic acid [(R)-1-hydroxymethyl-2-(1H-indol-3-yl)-ethyl]-amide;
- **37**: 7-(3-Methylcarbamoyl-phenylethynyl)-2,3,4,5-tetrahydro-benzo[b]oxepine-9-carboxylic acid [(R)-1-hydroxymethyl-2-(1H-indol-3-yl)-ethyl]-amide;
- **38**: 7-(3-Fluoro-5-methylcarbamoyl-phenyl)-2,3,4,5-tetrahydro-benzo[b]oxepine-9-carboxylic acid [2-(5,6-difluoro-1H-indol-3-yl)-1-hydroxymethyl-ethyl]-amide;
- **39**: 7-(3-Fluoro-5-methylcarbamoyl-phenyl)-2,3,4,5-tetrahydro-benzo[b]oxepine-9-carboxylic acid [2-(5,7-difluoro-1H-indol-3-yl)-1-hydroxymethyl-ethyl]-amide;
- **40**: 7-((E)-Styryl)-2,3,4,5-tetrahydro-benzo[b]oxepine-9-carboxylic acid [(R)-1-hydroxymethyl-2-(1 H-indol-3-yl)-ethyl]-amide.

The present invention also relates to a process for preparing the compounds according to the invention. Compounds of the general formula I can be prepared as shown in Scheme 1 by an amide forming reaction between the tryptophanol derivative IV and the carboxylic acid V. Reagents suitable for this purpose are all suitable peptide-coupling reagents which are known to the skilled person and which convert the carboxylic acid, where appropriate in the presence of a base, into an intermediate active ester, for example PyBOP ([(1*H*-benzotriazol-1-yl)oxy]tris(pyrrolidin-1-yl)phosphonium hexafluorophosphate), HATU (2-(7-aza-1*H*-benzotriazol-1-yl)-1,1,3,3-tetramethyluronium hexafluorophosphate), HBTU (2-(1*H*-benzotriazol-1-yl)-1,1,3,3-tetramethyluronium hexafluorophosphate), EDC (*N*-[3-(dimethylamino)propyl]-*N*'-ethylcarbodiimide hydrochloride) / HOBt (1-hydroxy-1*H*-benzotriazole). It is possible as alternative for the carboxylic acid to be converted, where appropriate in the presence of a base, into the carbonyl chloride and reacted with the tryptophanol IV to give the product of the general formula I. Suitable reagents for preparing the intermediate carbonyl chloride are for example thionyl chloride, oxalyl chloride, phosgene or derivatives thereof.

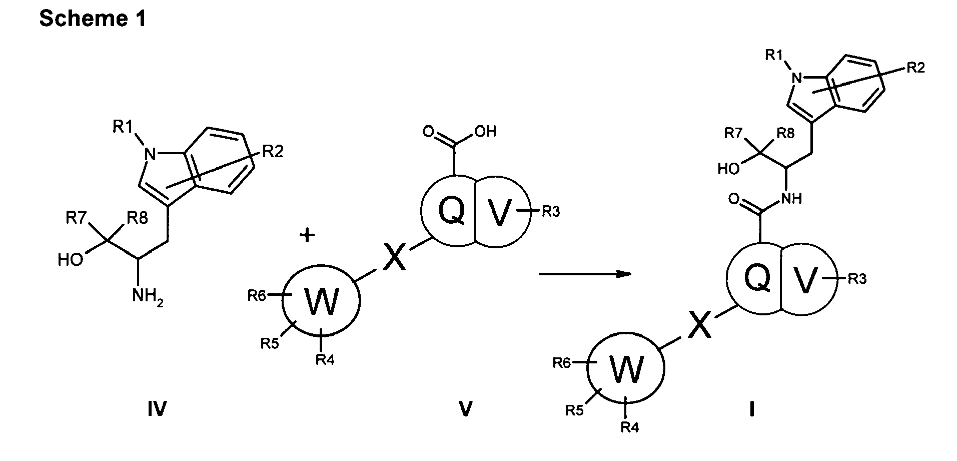

Compounds of general formula II can be prepared as shown in Scheme 2 by an amide forming reaction between the tryptophanol derivative IV and the appropriate carboxylic acid VI. Reagents suitable for this purpose are all known peptide-coupling reagents which convert the carboxylic acid, where appropriate in the presence of a base, into an intermediate active ester, for example PyBOP ([(1*H*-benzotriazol-1-yl)oxy]tris(pyrrolidin-1-yl)phosphonium hexafluorophosphate), HATU (2-(7-aza-1*H*-benzotriazol-1-yl)-1,1,3,3-tetramethyluronium hexafluorophosphate), HBTU (2-(1*H*-benzotriazol-1-yl)-1,1,3,3-tetramethyluronium hexafluorophosphate), EDC (*N*-[3-(dimethylamino)propyl]-*N*'-ethylcarbodiimide hydrochloride) / HOBt (1-hydroxy-1*H*-benzotriazole). It is possible as alternative for the carboxylic acid to be converted, where appropriate in the presence of a base, into the carbonyl chloride and reacted with the tryptophanol IV to give the product of the general formula II. Suitable reagents for preparing the intermediate carbonyl chloride are for example thionyl chloride, oxalyl chloride, phosgene or derivatives thereof.

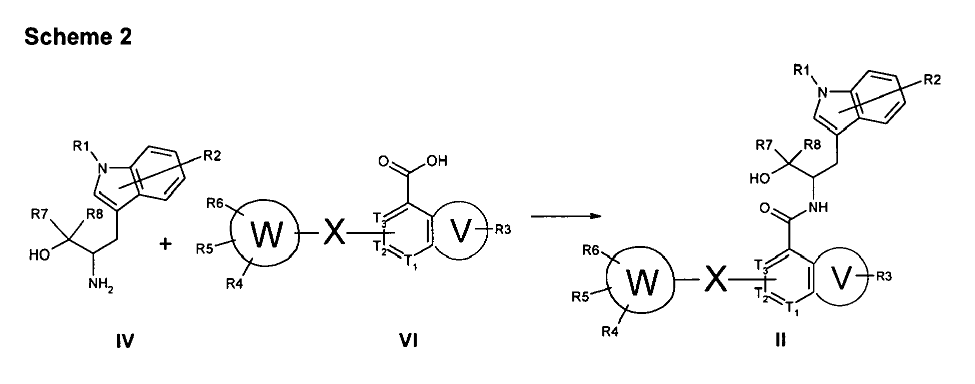

Compounds of general formula III can be prepared as shown in Scheme 3 by an amide forming reaction between the tryptophanol derivative IV and the appropriate carboxylic acid VII. Reagents suitable for this purpose are all suitable peptide-coupling reagents which are known to the skilled person and which convert the carboxylic acid, where appropriate in the presence of a base, into an intermediate active ester, for example PyBOP ([(1*H*-benzotriazol-1-yl)oxy]tris(pyrrolidin-1-yl)phosphonium hexafluorophosphate), HATU (2-(7-aza-1*H*-benzotriazol-1-yl)-1,1,3,3-tetramethyluronium hexafluorophosphate), HBTU (2-(1*H*-benzotriazol-1-yl)-1,1,3,3-tetramethyluronium hexafluorophosphate), EDC (N-[3-(dimethylamino)propyl]-N'-ethylcarbodiimide hydrochloride) / HOBt (1-hydroxy-1*H*-benzotriazole). It is possible as alternative for the carboxylic acid to be converted, where appropriate in the presence of a base, into the carbonyl chloride and reacted with the tryptophanol IV to give the product of the general formula III. Suitable reagents for preparing the intermediate carbonyl chloride are for example thionyl chloride, oxalyl chloride, phosgene or derivatives thereof.

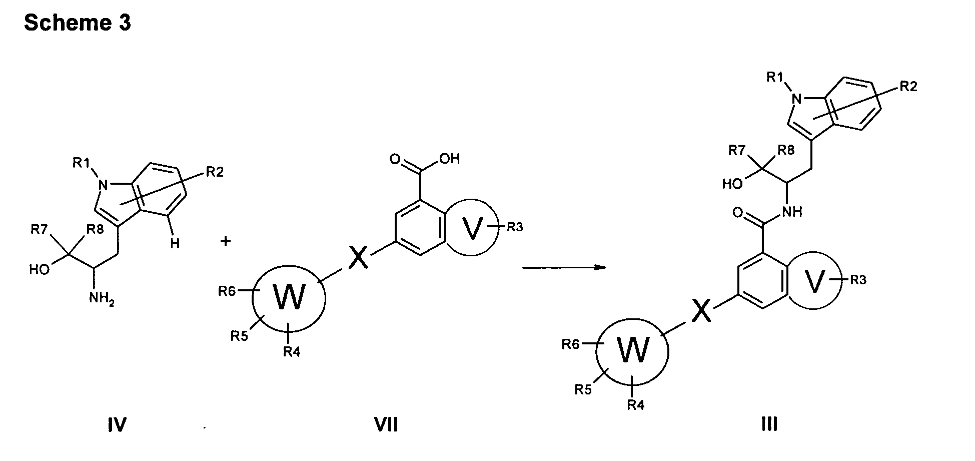

Alternatively, compounds of the general formula I can also be prepared as shown in Scheme 4 by a metal catalyzed cross coupling reaction between the aryl halide VIII (wherein Hal stands for chlorine, bromine or iodine) and the correspondingly functionalized building block XI known to the skilled person, for example: a Suzuki reaction (R = -B(OH)₂), a Sonogashira coupling (R = H, X = -C≡C-), a Negishi coupling (R = Zn-Hal) or a Stille coupling (R = Sn(alkyl)₃). Preferred metal catalysts used are typically those containing palladium or nickel, depending on the nature of the cross-coupling reaction. For a more detailed description of the metal catalyzed cross-coupling reactions applied and applicable for the synthesis of compounds of the formula I, see e.g. S. Takahashi, Y. Kuroyama, K. Sonogashira and N. Hagihara, Synthesis, 1980, 627; Metal catalyzed Cross-coupling Reactions, ed. F. Diederich and P. J. Stang, Wiley-VCH, Weinheim, 1998; K. Sonogashira, J. Organomet. Chem., 2002, 653 (1-2), 46.

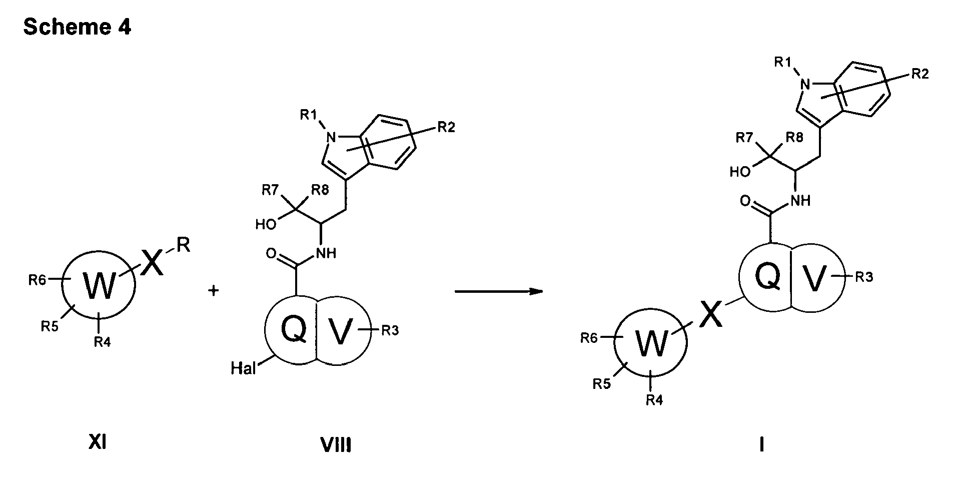

Likewise, compounds of the general formula II can be prepared as shown in Scheme 5 by a metal catalyzed cross coupling reaction between the aryl halide IX and the correspondingly functionalized building block XI known to the skilled person.

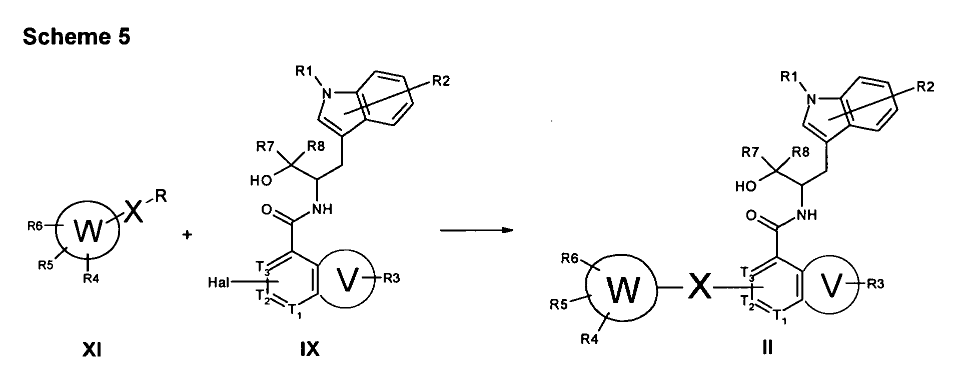

Likewise, compounds of the general formula III can be prepared as shown in Scheme 6 by a metal catalyzed cross coupling reaction between the aryl halide X and the correspondingly functionalized building block XI known to the skilled person.

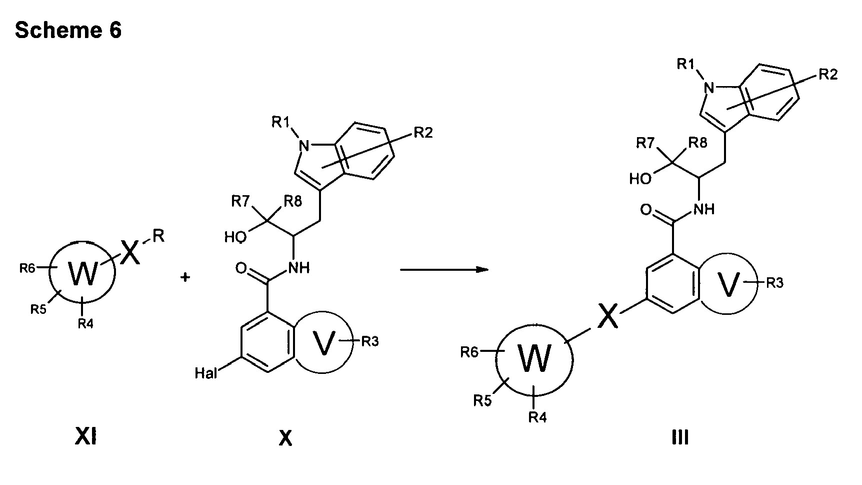

The present invention further relates to the carboxylic acids of the formulae V, VI and VII as intermediates of the process according to the invention for preparing the compounds according to the invention, namely:
6-(3,4,5-Trimethoxy-phenyl)-2H-chromene-8-carboxylic acid;
6-o-Tolylethynyl-2H-chromene-8-carboxylic acid;
6-(2-Methoxy-phenylethynyl)-2H-chromene-8-carboxylic acid;
5-(3,4-Dimethoxy-phenyl)-2-vinyl-2,3-dihydro-benzofuran-7-carboxylic acid;
5-(3,4-Dimethoxy-phenyl)-2-ethyl-2,3-dihydro-benzofuran-7-carboxylic acid;
5-(3,4-Difluoro-5-methoxy-phenyl)-2,3-dihydro-benzofuran-7-carboxylic acid;
5-(3,4-Dimethoxy-phenyl)-2,3-dihydro-benzofuran-7-carboxylic acid;
5-(3-Methoxy-phenyl)-2,3-dihydro-benzofuran-7-carboxylic acid;
5-Benzo[1,3]dioxol-5-yl-2,3-dihydro-benzofuran-7-carboxylic acid;
5-(3-Fluoro-4-methoxy-phenyl)-2,3-dihydro-benzofuran-7-carboxylic acid;
5-(3,4,5-Trimethoxy-phenyl)-2,3-dihydro-benzofuran-7-carboxylic acid;
7-(3-Fluoro-5-methylcarbamoyl-phenyl)-2,3,4,5-tetrahydro-benzo[b]oxepine-9-carboxylic acid
and the methyl, ethyl, propyl and butyl esters thereof.

The present invention further relates to the aryl halides of the formulae VIII, IX and X as intermediates of the process according to the invention for preparing the compounds according to the invention, namely:
6-Iodo-2H-chromene-8-carboxylic acid [(R)-2-hydroxy-1-(1H-indol-3-ylmethyl)-ethyl]-amide;
6-Iodo-2-methyl-2H-chromene-8-carboxylic acid [(R)-1-hydroxymethyl-2-(1H-indol-3-yl)-ethyl]-amide;
6-Iodo-2,2-dimethyl-2H-chromene-8-carboxylic acid [(R)-2-hydroxy-1-(1H-indol-3-ylmethyl)-ethyl]-amide;
7-Bromo-2,3-dihydro-benzo[1,4]dioxine-5-carboxylic acid [(R)-2-hydroxy-1-(1H-indol-3-ylmethyl)-ethyl]-amide;
7-Bromo-2,3-dihydro-benzo[1,4]dioxine-5-carboxylic acid [2-(6-fluoro-1H-indol-3-yl)-1-hydroxymethyl-ethyl]-amide;
7-Bromo-2,3,4,5-tetrahydro-benzo[b]oxepine-9-carboxylic acid [(R)-2-hydroxy-1-(1H-indol-3-ylmethyl)-ethyl]-amide;
7-Bromo-2,3,4,5-tetrahydro-benzo[b]oxepine-9-carboxylic acid [1-(6-fluoro-1H-indol-3-ylmethyl)-2-hydroxy-ethyl]-amide.

### Pharmacological data

### HTRF assay for measuring cAMP in cells

The method is based on a competitive immunoassay between native cAMP, which has been produced by the cells, and cAMP which is labelled with XL665. The tracer binding was visualized by a monoclonal antibody, anti-cAMP labelled with cryptate [HTRF = homogeneous time-resolved fluorescence].

The specific signal is inversely proportional to the cAMP concentration of the samples employed.

The 665nm/ 620nm fluorescence ratio was evaluated.

*The following material was used: 96-well plates for the tissue culture, 96-well plates with black edge and black base (e.g. Fluotrac 600 from Greiner), 96-well plates for the substance dilutions of polypropylene and CAMP Femtomolar (4000wells Kit, CIS Bio International # 62AM1PEC).*

The following reagents were used: BSA (bovine serum albumin) Fraction V protease-free, IBMX (3-isobutyl-1-methylxanthine), hFSH (human follicle stimulating hormone), Triton X-100 analytical grade, potassium fluoride analytical grade, G 418 (Geneticin) and Accutase.

Buffer 1 (washing and testing buffer) contained PBS, 1 mM CaCl2, 1 mM MgCl₂, 0.2% glucose; 0.1% BSA, 1 mM IBMX.

Buffer 2 (2x lysis buffer) contained 1 % Triton X-100 in PBS (without CaCl₂ and MgCl₂).

Buffer 3 (assay buffer) contained 50 mM potassium phosphate buffer (pH 7.0); 800 mM potassium fluoride; 0.2% BSA (always added fresh).

### Procedure:

On day 1, the cells were seeded in 96-well plates (3x10⁴ cells per well hFSHR clone 16 cells (CHO cells stably transfected with the human FSH receptor in 150 µl of medium). The next day, test substance dilutions were made up. For this purpose, all the substances were diluted in ice-cold buffer 1 (with or without hFSH), and the substance dilutions were placed on ice until applied to the cells.

The cell supernatant was then aspirated off, and the cells were washed 2x with 200 µl of buffer 1. The cells were treated with 60 µl of the appropriate substance concentrations at 37°C for 2h. The cells were then lysed with 60 µl of buffer 2 (put onto the supernatant) (on a plate shaker at RT for 30 min).

The test conjugates (XL-665 and anti-cAMP cryptate) were diluted in buffer 3 in accordance with the manufacturers' information. The actual mixture for measurement was pipetted into a black 96-well plate (in each case 15 µl of the cell lysate diluted with 35 µl of buffer 1; firstly 25 µl of XL-665 conjugate were pipetted and, after 10 min, 25 µl of the anti-cAMP cryptate were added). This is followed by incubation at RT for 90 minutes. The measurement was carried out in a PheraStar (BMG).

### Tissue culture conditions

- 1) hFSHr clone 16: Ham's F12
PSG
10% FCS
700 µg/ml G 418 (Geneticin) from PAA.

Dose-effect curve (hFSH) for the human receptor: 1 e-8, 3e-9, 1 e-9, 3e-10, 1e-10, 3e-11, 1e-11, 3e-12 mol/l.

The test substances were employed in suitable dilutions in the absence (test for agonism) and in the presence of 1 e-9 mol/l hFSH.

### Evaluation

The values of the well ratio were averaged and then entered directly in SigmaPlot versus the concentrations. The maximum and minimum values were determined for each plate, and half the difference is to be regarded as IC₅₀.

The test results (Table 1) show that the compounds according to the invention have an FSH-antagonistic effect.

**Table 1. FSH-antagonistic effect of selected compounds in the HTRF assay**

| **Compound [Ex. #]** | **IC₅₀** |
|---|---|
| **2** | **130 nM** |
| **4** | **450 nM** |
| **8** | **14 nM** |
| **11** | **30 nM** |
| **12** | **350 nM** |
| **13** | **150 nM** |
| **14** | **450 nM** |
| **20** | **2 µM** |
| **23** | **450 nM** |

Being potent antagonists of the FSH receptor, compounds of the general formula I or pharmaceutically acceptable salts thereof can thus be used for the manufacture of medicaments to be used for the fertility control in male and/or in a female animals, in particular in men and/or women; as well as for the treatment and/or prevention of osteoporosis.

### Pharmaceutical compositions

The invention further relates to compounds of the general formula I or pharmaceutically acceptable salts thereof as therapeutic active ingredients, and to pharmaceutical compositions comprising at least one compound of the general formula I or pharmaceutically acceptable salts thereof, where appropriate together with pharmaceutically suitable excipients and/or carriers.

Pharmaceutically acceptable salts of the compounds of the general formula I can be prepared by methods known to the skilled person, depending on the nature of the compound of formula I, either by using as inorganic acids inter alia hydrochloric acid, hydrobromic acid, sulphuric acid and phosphoric acid, nitric acid, as carboxylic acids inter alia acetic acid, propionic acid, hexanoic acid, octanoic acid, decanoic acid, oleic acid, stearic acid, maleic acid, fumaric acid, succinic acid, benzoic acid, ascorbic acid, oxalic acid, salicylic acid, tartaric acid, citric acid, lactic acid, glycolic acid, malic acid, mandelic acid, cinnamic acid, glutamic acid, aspartic acid, and as sulphonic acids inter alia methanesulphonic acid, ethanesulphonic acid, toluenesulphonic acid, benzenesulphonic acid and naphthalenesulphonic acid;
or by using an appropriate base as inorganic base inter alia alkalimetal hydroxide, carbonate, or hydrogencarbonate, as organic base inter alia tertiary amines and N-heterocycles.

These pharmaceutical compositions and medicaments may be intended for oral, rectal, subcutaneous, transdermal, percutaneous, intravenous or intramuscular administration. They comprise besides conventional carriers and/or diluents at least one compound of the general formula I.

The medicaments of the invention are formulated using the customary solid or liquid carriers or diluents and the excipients customarily used in pharmaceutical technology, in accordance with the desired mode of administration with a suitable dosage in a known manner: i.e. by processing the active ingredient with the carrier substances, fillers, substances which influence disintegration, binders, humectants, lubricants, absorbents, diluents, test modifiers, colorants etc. which are used in pharmaceutical technology, and converting into the desired administration form. Reference should be made in this connection to Remington's Pharmaceutical Science, 15th ed. Mack Publishing Company, East Pennsylvania (1980) and to Gennaro, A. R. et al., Remington: The Science and Practice of Pharmacy (20th Edition., Lippincott Williams & Wilkins 2000, see especially Part 5: Pharmaceutical Manufactoring).

Pharmaceutical compositions according to the present invention are preferably administered orally. Suitable for oral administration are in particular tablets, (film)-coated tablets, sugar-coated tablets capsules, pills, powders, granules, pastilles, suspensions, emulsions, solutions or depot forms.

Appropriate tablets can be obtained for example by mixing the active ingredient with known excipients, for example inert diluents such as dextrose, sugar, sorbitol, mannitol, polyvinylpyrrolidone, disintegrants such as maize starch or alginic acid, binders such as starch or gelatine, lubricants such as magnesium stearate or talc and/ or agents to achieve a depot effect such as carboxylpolymethylene, carboxylmethylcellulose, cellulose acetate phthalate or polyvinyl acetate. The tablets may also consist of a plurality of layers.

Correspondingly, coated tablets can be produced by coating cores which have been produced in analogy to the tablets with agents normally used in tablet coatings, for example polyvinylpyrrolidone or shellac, gum Arabic, talc, titanium oxide or sugar. The tablet coating may also consist of a plurality of layers, it being possible to use the excipients mentioned above for tablets.

Solutions or suspensions with the compounds according to the invention of the general formula I may additionally comprise taste-improving agents such as saccharin, cyclamate or sugar and, for example, flavourings such as vanillin or orange extract. They may additionally comprise suspending aids such as sodium carboxymethylcellulose or preservatives such as *p*-hydroxybenzoates.

Capsules comprising the compounds of the general formula I can be produced for example by the compound(s) of the general formula I being mixed with an inert carrier such as lactose or sorbitol and encapsulated in gelatine capsules.

Parenteral preparations such as solutions for injection are also suitable. Preparations for injection and infusion are possible for parenteral administration. Appropriately prepared crystal suspensions can be used for intraarticular injection. Aqueous and oily solutions for injection or suspensions and corresponding depot preparations can be used for intramuscular injection. The novel compounds can be used for rectal administration in the form of suppositories, capsules, solutions (e.g. in the form of enemas) and ointments both for systemic and for local therapy.

Suitable suppositories can be produced for example by mixing with carriers intended for this purpose, such as neutral fats or polyethylene glycol or derivatives thereof.

Formulations suitable for topical application include gels, ointments, greasy ointments, creams, pastes, dusting powders, milk and tinctures. Topical use can also take place by means of a transdermal system, for example a patch. The concentration of the compounds of the general formula I in these preparations should typically be in the range of 0.01% - 20% in order to achieve an adequate pharmacological effect.

The invention further relates to pharmaceutical compositions in combination with packaging material suitable for said composition, wherein said packaging material including instructions for the use of the composition.

### Dose

Suitable doses for the compounds according to the present invention may vary from 0.005 mg to 50 mg per day per kg of body weight, depending on the age and constitution of the patient. It is possible to administer the necessary daily dose by single or multiple delivery. The preferred daily dose for larger mammals, for example humans, may vary in the range from 10 µg to 30 mg per kg of body weight.

The exact dose and regimen of administration of the drug substance (active ingredient, or a pharmaceutical composition thereof, may however vary with the particular compound, the route of administration, and the age, sex and condition of the individual to whom the medicament is administered. The dose, the dosage as well as the regimen of the administration may thus differ between a male and a female considerably.

The compounds according to the invention of the general formula I can be prepared as described below.

**Abbreviations used:**
- ACN: Acetonitrile
- DIBAC: Diisobutylaluminium hydride
- DMF: N,N-Dimethylformamide
- EDC: *N*-Ethyl-*N*'-(3-dimethylaminopropyl)carbodiimide
- EtOH: Ethanol
- HATU: O-(7-Azabenzotriazol-1-yl)-N,N,N',N'-tetramethyluronium hexafluorophosphate
- FMOC: (9H-Fluoren-9-ylmethoxy)carbonyl
- HOBt: 1-Hydroxy-1 H-benzotriazole
- MeCN: Acetonitrile
- MeOH: Methanol
- MTBE: Methyl tert-butyl ether
- NMM: 4-methylmorpholine
- NMP: N-Methylpyrrolidinone
- Rf: Reflux
- RT: Room temperature
- TBAF: Tetrabutylammonium fluoride
- TFA: Trifluoroacetic acid
- THF: Tetrahydrofuran

Compounds of the general formula I can in principle be prepared as shown in Scheme 7 by an amide forming reaction between a tryptophanol derivative IV and a carboxylic acid V. The reagents typically used for the coupling are EDC and HOBt.

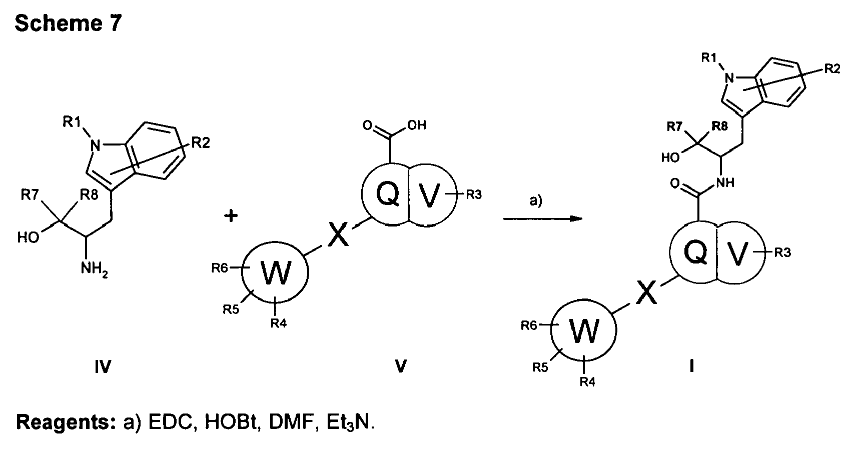

The tryptophanol derivatives of the formula IV with R7 = R8 = H can be prepared as shown in Scheme 8 from the corresponding amino acids which can be purchased or are known from the literature.

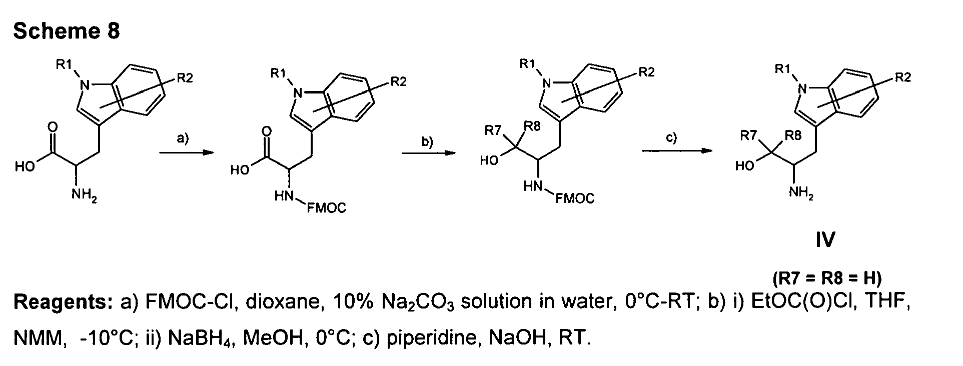

Compounds of the general formula I can in principle also be prepared as shown in Scheme 9 via an Grignard reaction from the corresponding esters XII.

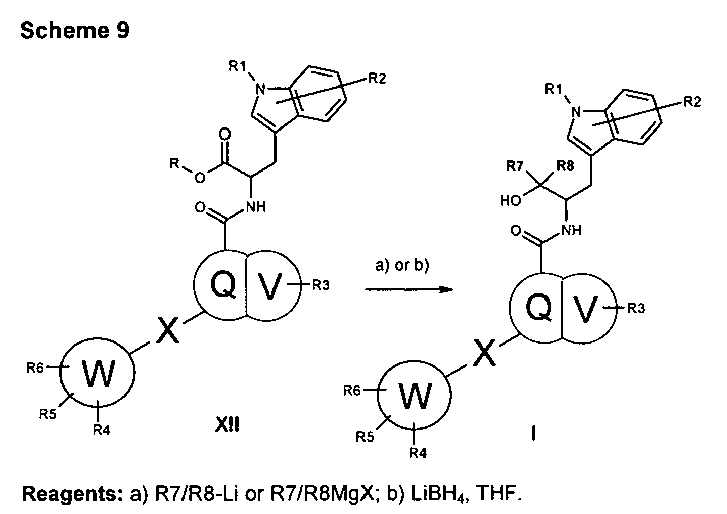

Compounds of the general formula XII can in principle be prepared as shown in Scheme 10 by an amide forming reaction between a tryptophan ester derivative XIII and a carboxylic acid V. The reagents typically used for the coupling are EDC and HOBt. The tryptophan ester derivatives XIII are commercially available or known from the literature.

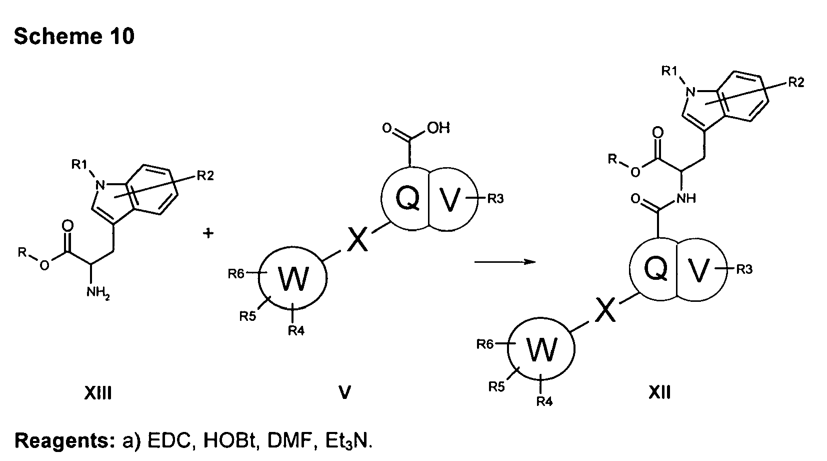

Carboxylic acids of the general formula XVII can in principle be prepared as shown in Scheme 11 via a Sonogashira type reaction between an acetylene derivative XV or XVIII and an aryl halide XIV or XIX, followed by ester hydrolysis.

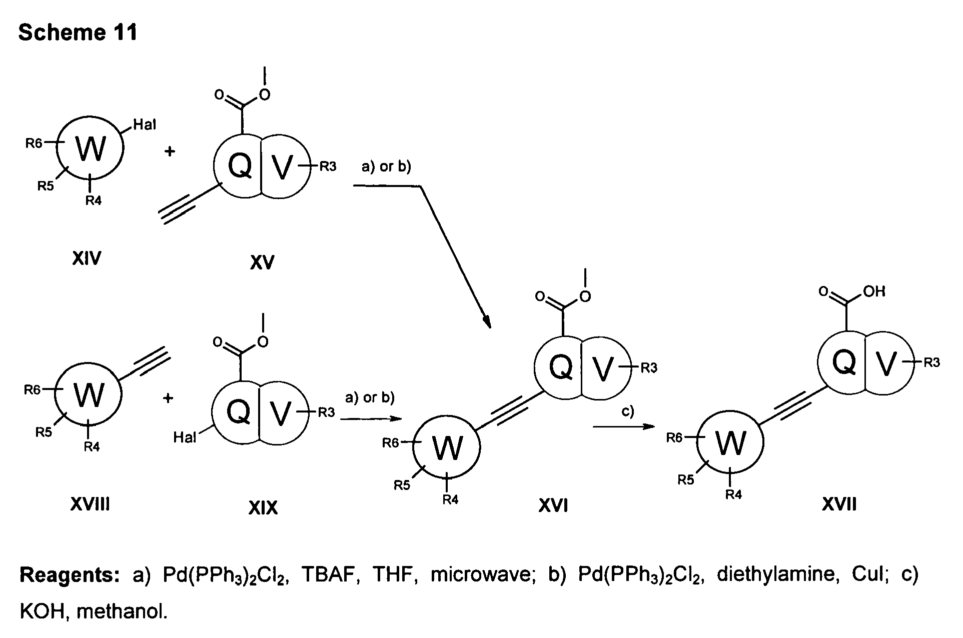

Esters of the general formula XV can in principle be prepared as shown in Scheme 12 via a Sonogashira type reaction between trimethylsilylacetylene and aryl halide XIX.

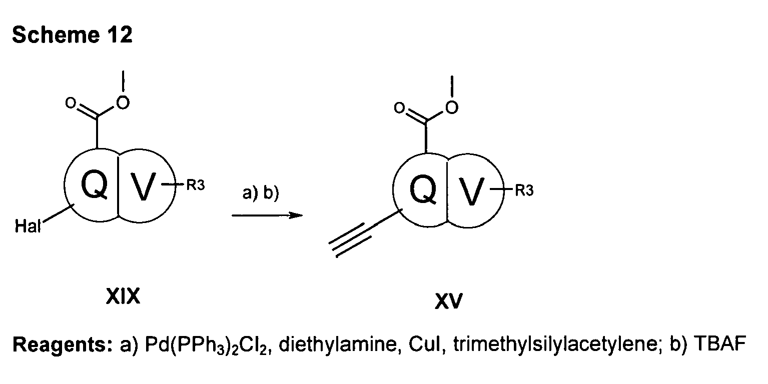

Carboxylic acids of the general formula V can in principle be prepared as shown in Scheme 13 via a Suzuki type reaction of an boronic acid derivative XX or XXI and an aryl halide XIV or XIX, followed by ester hydrolysis.

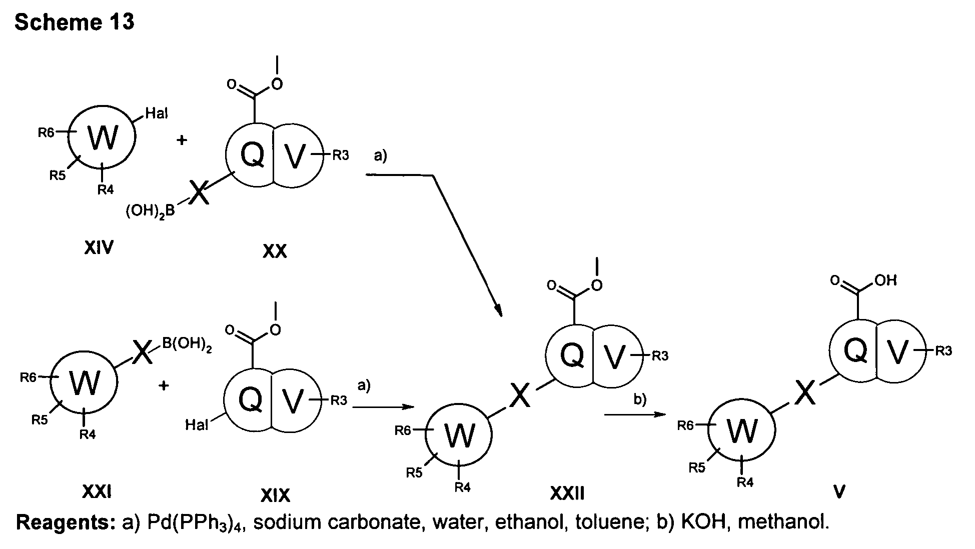

Compounds of the general formula I can in principle also be prepared as shown in Scheme 14 via Suzuki reaction of aryl halide XXIII and boronic acid XXI.

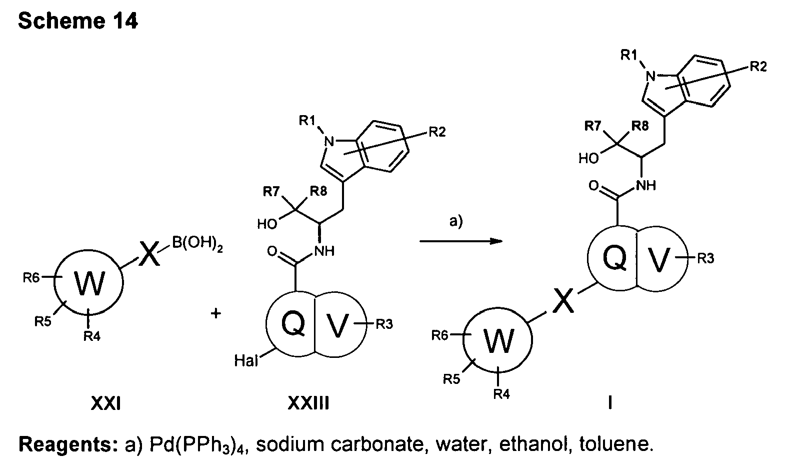

The acetylene derivatives of formula I can in principle also be prepared according to Scheme 15 via a Sonogashira type coupling of terminal acetylene XVIII or XXV with the corresponding aryl halides XIV or XXIII.

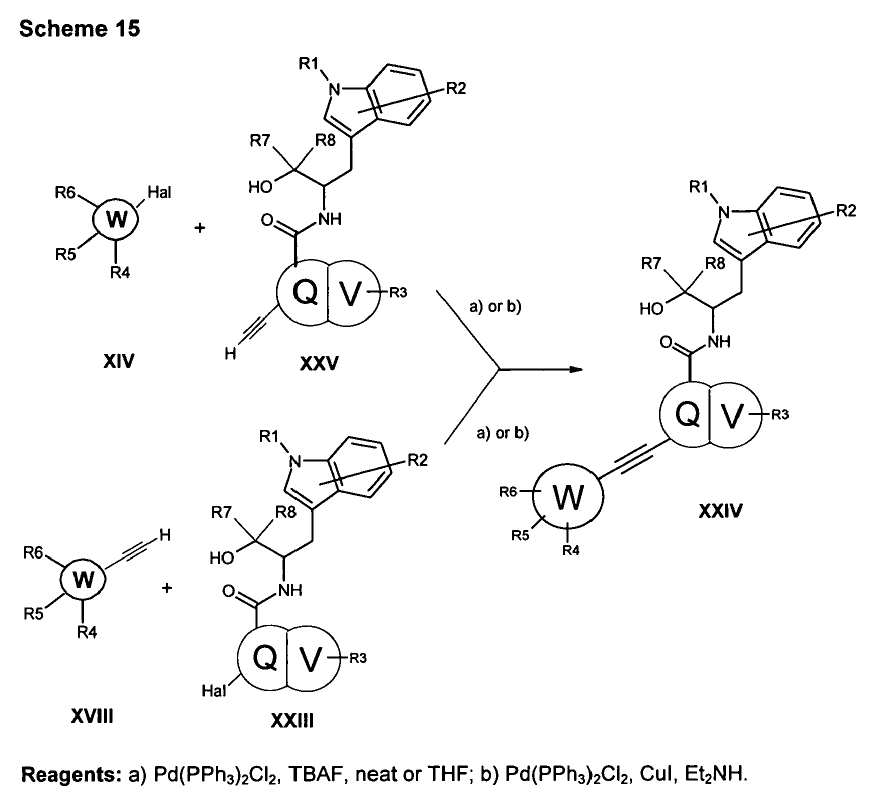

The acetylene derivatives of formula XXVI can in principle also be prepared according to Scheme 16 via a Sonogashira type coupling from terminal acetylene XXIII. The aryl halide XXIII itself can be prepared via an amide forming reaction from the tryptophanol derivative IV and carboxylic acid XXV.

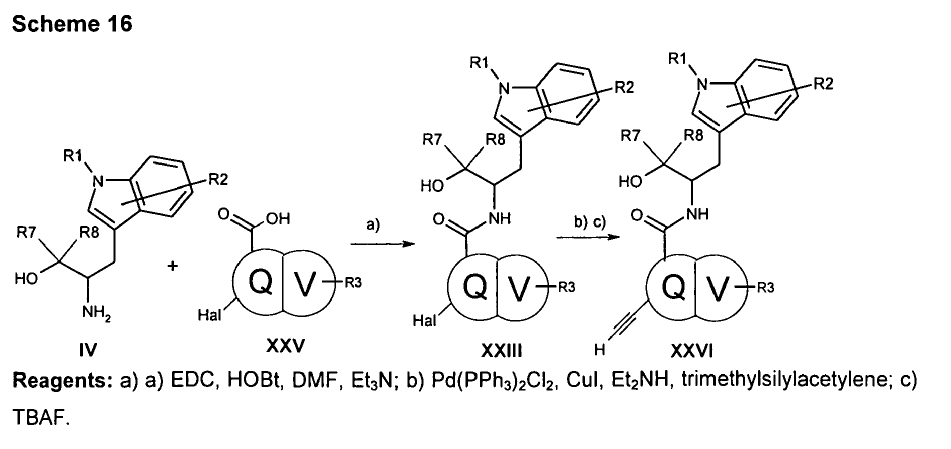

### Synthesis of the compounds according to the invention

### Example 1

**6-(3,4,5-Trimethoxy-phenyl)-2H-chromene-8-carboxylic acid [(R)-1-hydroxymethyl-2-(1 H-indol-3-yl)-ethyl]-amide** 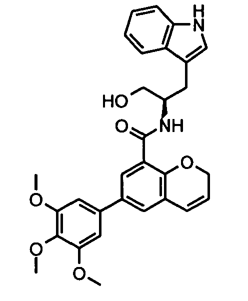
1a) 5-Iodo-2-prop-2-ynyloxy-benzoic acid methyl ester
   A suspension of 2-Hydroxy-5-iodo-benzoic acid methyl ester (20 g), potassium carbonate (29.8 g), propargylic bromide (27 mL) in acetone (200 mL) was stirred under reflux overnight. The reaction mixture was filtrated, the solvent evaporated and the crude product was recrystallized from ethyl acetate / petrol ether to yield the title compound (17.67 g, 78%). **¹H-NMR (DMSO-d₆):** 7.87 d (*J* = 2.3 Hz, 1H); 7.83 dd (*J* = 2.3 Hz / 8.8 Hz, 1H); 7.03 d (*J* = 8.8 Hz, 1H); 4.84 d (*J* = 2.3 Hz, 1H); 3.75 s (3H); 3.59 m (1H).
1b) 6-Iodo-2H-chromene-8-carboxylic acid methyl ester
   5-Iodo-2-prop-2-ynyloxy-benzoic acid methyl ester (10 g) in diethylaniline (50 mL) was stirred in a metal bath under reflux for 4 hours. The mixture was poured in a water / ice mixture and acidified by addition of concentrated HCl. The water phase was extracted with ethylacetate and dried over sodium sulphate. After evaporation of the solvent the title compound was obtained after flash chromatography in 19 % yield (1.9 g). **¹H-NMR (DMSO-d₆):** 7.73 d (*J* = 2.3 Hz, 1H); 7.59 (*J* = 2.3 Hz, 1H); 6.51 m (1H); 6.02 m (1H); 4.89 m (2H); 3.78 s (3H).
1c) 6-(3,4,5-Trimethoxy-phenyl)-2H-chromene-8-carboxylic acid methyl ester 6-Iodo-2H-chromene-8-carboxylic acid methyl ester (500 mg), 3,4,5-Trimethoxy phenyl boronic acid (371 mg), Pd(PPh₃)₄ (92 mg) in ethanol (5 mL), toluene (5 mL) and sodium carbonate solution in water (2 molar, 2 mL) were stirred at 100°C for 12 hours. The solvents were distilled off and the material was purified by flash chromatography to yield the title compound in 42% yield (240 mg). **¹H-NMR (DMSO-d₆):** 7.73 d *(J =* 2.5 Hz, 1H); 7.59 d (*J* = 2.5 Hz, 1H); 6.87 s (2H); 6.62 m (1H); 6.04 m (1H); 4.90 m (2H); 3.86 s (6H); 3.82 s (3H); 3.69 s (3H).
1d) 6-(3,4,5-Trimethoxy-phenyl)-2H-chromene-8-carboxylic acid
   A solution of 6-(3,4,5-Trimethoxy-phenyl)-2H-chromene-8-carboxylic acid methyl ester (240 mg) in methanolic KOH (10%, 10 mL) was stirred at 50°C for 2 hours. The solvent was distilled off under vacuum and the residue extracted with water / ether. The water phase was acidified by the addition of HCl (2N) and extracted with ether. The solvent was distilled off under vacuum and the title compound was isolated in 74% yield (170 mg). **¹H-NMR (DMSO-d₆):** 12.74 s (1H); 7.67 d (*J* = 2.5 Hz, 1H); 7.51 d (*J* = 2.5 Hz, 1H); 6.81 s (2H); 6.57 m (1H); 5.97 m (1H); 4.84 m (2H); 3.82 s (6H); 3.64 s (3H).
1e) 6-(3,4,5-Trimethoxy-phenyl)-2H-chromene-8-carboxylic acid [(R)-1-hydroxymethyl-2-(1 H-indol-3-yl)-ethyl]-amide
   A solution of 6-(3,4,5-Trimethoxy-phenyl)-2H-chromene-8-carboxylic acid (100 mg), HOBt hydrate (153 mg), EDC (192 mg) and (D) tryptophanol (190 mg) in DMF (5 ml) was stirred at ambient temperature overnight. The reaction mixture was poured in water, extracted with ethyl acetate and the combined organic layers dried over magnesium sulphate. The title compound was obtained in 75% yield (112 mg) after flash chromatography. **¹H-NMR (DMSO-d₆):** 10,81 s (1H); 8.06 d (*J* = 7.8 Hz, 1H); 7.84 d (*J* = 2.3 Hz, 1H); 7.65 d (*J* = 7.8 Hz, 1H); 7.48 d (*J* = 2.5 Hz, 1H); 7.29 d (*J* = 8.1 Hz, 1H); 7.16 d (*J* = 2.3 Hz, 1H); 7.03 m (1 H); 6.94 m (1H); 6.80 s (2H); 6.59 m (1H); 5.98 m (1H); 4.88 m (1H); 4.87 m (1H); 4.19 m (1H); 3.82 s (6H); 3.65 s (3H); 3.51 m (1H); 3.40 m (1H); 2.96 m (2H).

### Example 2

### 6-(3-Chloro-4-methylcarbamoyl-phenyl)-2H-chromene-8-carboxylic acid [(R)-2-hydroxy-1-(1H-indol-3-ylmethyl)-ethyl]-amide

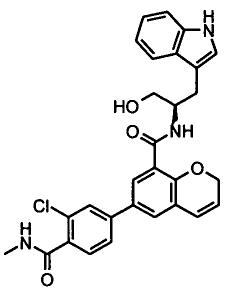

2a) 6-Iodo-2H-chromene-8-carboxylic acid
   A solution of 6-Iodo-2H-chromene-8-carboxylic acid methyl ester (440 mg) in methanolic KOH (10%, 2.1 mL) was stirred at ambient temperature overnight. The solvent was evaporated and the residue was extracted with water / ethyl acetate. The water phase was acidified by addition of HCl (1N) and extracted with ether. The organic phase was dried over sodium sulphate and the solvent was evaporated to yield 88% of the tilte compound (372 mg). **¹H-NMR (DMSO-d₆):** 12.84 s (1H); 7.67 d (*J* = 2.3 Hz, 1H); 7.51 d (*J* = 2.3 Hz, 1H); 6.48 m (1H); 6.44 m (1H); 5.96 m (1H); 5.92 m (1H); 4.83 m (2H).
2b) 6-Iodo-2H-chromene-8-carboxylic acid [(R)-2-hydroxy-1-(1H-indol-3-ylmethyl)-ethyl]-amide
   A soluion of 6-Iodo-2H-chromene-8-carboxylic acid (345 mg), (D)-tryptophanol (261 mg), EDC (263 mg), HOBt (185 mg) in DMF (5 mL) was stirred at ambient temperature overnight. The reaction mixture was poured into water and the precipitate was filtered off. The title compound was obtained after flash chromatography in 46% yield (250 mg). **¹H-NMR (DMSO-d₆):** 10.79 s (1H); 7.96 d (*J* = 7.9 Hz, 1H); 7.78 d (*J* = 2.3 Hz, 1H); 7.60 d (*J* = 7.7 Hz, 1H); 7.47 d (*J* = 2.3 Hz, 1H); 7.29 d (*J* = 7.9 Hz, 1H); 7.13 d (*J* = 2.1 Hz, 1H); 7.02 m (1H); 6.94 m (1H); 6.46 m (1H); 5.95 m (1H); 4.85 m (1H); 4.79 m (1H); 4.13 m (1H); 3.44 m (1H); 3.36 m (1H); 2.92 m (2H).
2c) 6-(3-Chloro-4-methylcarbamoyl-phenyl)-2H-chromene-8-carboxylic acid [(R)-2-hydroxy-1-(1 H-indol-3-ylmethyl)-ethyl]-amide
   A solution of 6-Iodo-2H-chromene-8-carboxylic acid [(R)-2-hydroxy-1-(1 H-indol-3-ylmethyl)-ethyl]-amide (200 mg), 3-Chloro-4-methylcarbamoyl-phenyl boronic acid (99 mg), Pd(PPh₃)₄ (24 mg) in ethanol (5 mL), toluene (5 mL) and an aqueous sodium carbonate solution (2M, 0.5 mL) was stirred at 100°C for 3 hours. The solvent was evaporated and the solid purified by flash chromatography to yield 46% of the title compound (100 mg). **¹H-NMR (DMSO-d₆):** 10,80 s (1H); 8.35 d (*J* = 4.6 Hz, 1H); 8.06 d (*J* = 7.8 Hz, 1H); 7.86 d (*J* = 2.3 Hz, 1H); 7.69 d (*J* = 1.8 Hz, 1H); 7.64 d (*J* = 7.8 Hz, 1H); 7.58 m (1H); 7.47 d (*J* = 7.8 Hz, 1H); 7.29 d (*J* = 8.1 Hz, 1H); 7.17 d (*J* = 2.3 Hz, 1H); 7.03 m (1H); 6.95 m (1H); 6.59 m (1H); 5.99 m (1H); 4.88 m (2H); 4.84 m (1H); 4.19 m (1H); 3.48 m (1H); 3.41 m (2H); 2.96 m (2H); 2.73 d (*J* = 4.6 Hz, 3H).

### Example 3

### 6-(3-Chloro-4-methylcarbamoyl-phenyl)-chroman-8-carboxylic acid [(R)-2-hydroxy-1-(1H-indol-3-ylmethyl)-ethyl]-amide

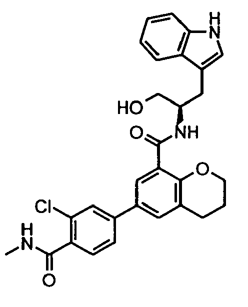

Hydrogenation of 6-(3-Chloro-4-methylcarbamoyl-phenyl)-2H-chromene-8-carboxylic acid [(R)-2-hydroxy-1-(1H-indol-3-ylmethyl)-ethyl]-amide (100 mg) in MeOH (3 mL) with Pd / C (10%) at ambient temperature yielded the title compound in 37% yield (37 mg) after filtration and evaporation of the solvent. **¹H-NMR (MeOD):** 8.41 d *(J =* 8.1 Hz, 1H); 7.94 d (*J* = 2.5 Hz, 1H); 7.64 d (*J* = 7.7 Hz, 1H); 7.57 d (*J* = 1.5 Hz, 1H); 7.41 m (3H); 7.13 s (1H); 7.06 m (1H); 6.96 m (1H); 4.43 m (1H); 4.03 m (1H); 3.90 m (1H); 3.64 m (2H); 3.09 m (2H); 2.89 s (3H); 2.76 m (2H); 1.87 m (2H).

### Example 4

### 6-o-Totylethynyl-2H-chromene-8-carboxylic acid [(R)-1-hydroxymethyl-2-(1H-indol-3-yl)-ethyl]-amide

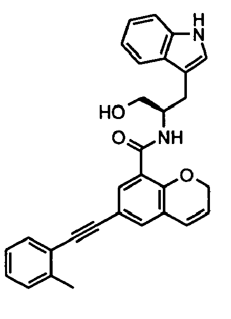

4a) 6-o-Tolylethynyl-2H-chromene-8-carboxylic acid methyl ester
   A mixture of 6-Iodo-2H-chromene-8-carboxylic acid methyl ester (500 mg), 1-Ethynyl-2-methyl-benzene (0.4 mL), Pd(PPh₃)₂Cl₂ (11 mg) and Cul (5 mg) in diethylamine (10 mL) was stirred at 60°C for 12 hours. The solvent was distilled off and the residue was extracted with water / ethyl acetate. The solvent of the organic phase was evaporated and the title compound was obtained after flash chromatography in 91% yield (440 mg). **¹H-NMR (DMSO-d₆):** 7.85 d (*J* = 2.1 Hz, 1H); 7.51 d (*J* = 7.4 Hz, 1H); 7.28 m (3H); 7.23 m (1H); 6.45 m (1H); 5.93 m (1H); 5.03 m (2H); 3.94 s (3H); 2.55 s (3H).
4b) 6-o-Tolylethynyl-2H-chromene-8-carboxylic acid
   A solution of 6-o-Tolylethynyl-2H-chromene-8-carboxylic acid methyl ester (180 mg) in methanolic KOH (10%, 10 mL) was refluxed for 10 minutes. The solvent was evaporated and the residue was extracted with water / ethyl acetate. The water phase was acidified by addition of HCl (2N) and extracted with ether. The organic phase was dried over sodium sulphate and the solvent was evaporated to yield 50 % of the tilte compound (86 mg). **¹H-NMR (DMSO-d₆):** 12.86 s (1H); 7.58 d (*J* = 2.3 Hz, 1H); 7.43 d *(J* = 7.1 Hz, 1H); 7.37 d (*J* = 2.3 Hz, 1H); 7.27 m (2H); 7.19 m (1H); 6.53 m (1H); 5.97 m (1H); 4.89 m (2H); 2.41 s (3H).
4c) 6-o-Tolylethynyl-2H-chromene-8-carboxylic acid [(R)-1-hydroxymethyl-2-(1H-indol-3-yl)-ethyl]-amideA soluion of 6-o-Tolylethynyl-2H-chromene-8-carboxylic acid (200 mg), (D)-tryptophanol (131 mg), EDC (132 mg), HOBt (106 mg) in DMF (10 mL) was stirred at ambient temperature overnight. The reaction mixture was poured into water and the precipitate was filtered off. The title compound was obtained after flash chromatography in 54 % yield (172 mg). **¹H-NMR (DMSO-d₆**): 10,84 s (1H); 8.05 d (*J* = 7.9 Hz, 1H); 7.73 d (*J* = 2.3 Hz, 1H); 7.68 d (*J* = 7.7 Hz, 1H); 7.50 d (*J* = 7.2 Hz, 1H); 7.38 d (*J* = 2.1 Hz, 1H); 7.36 m (3H); 7.26 m (1H); 7.20 d (*J* = 2.3 Hz, 1H); 7.07 m (1H); 6.99 m (1H); 6.58 m (1H); 6.00 m (1H); 4.90 m (3H); 4.21 m (1H); 3.53 m (1H); 3.43 m (1H); 2.99 m (2H); 2.47 s (3H).

The following compounds were obtained in analogy to the preparation methods described in detail:

| **Ex.** | **Product; *reagents*** | **Method analogous to** | **¹H-NMR (400 MHz) δ [ppm]** | **Structure** |
|---|---|---|---|---|
| **5** | 6-(2-Methoxy-phenylethynyl)-2H-chromene-8-carboxylic acid [(R)-1-hydroxymethyl-2-(1H-indol-3-yl)-ethyl]-amide; | **4** | **¹H-NMR (DMSO-d₆):** 10,80 s (1H); 8.05 d (*J* = 7.7 Hz, 1H); 7.83 d (*J* = 2.5 Hz, 1H); 7.64 d (*J* = 7.9 Hz, 1H); 7.48 d (*J* = 2.5 Hz, 1H); 7.30 d (*J* = 7.9 Hz, 1H); 7.16 d (*J* = 2.1 Hz, 1H); 7.03 m (1H); 6.94 m (1H); 6.80 s (2H); 6.59 m (1H); 5.99 m (1H); 4.87 m (1H); 4.85 m (2H); 4.18 m (1H); 3.65 s (3H); 3.47 m (1H); 3.40 m (1H); 2.96 m (2H). | 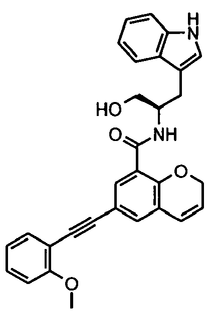 |
| | *6-(2-Methoxy-phenylethynyl)-2H-chromene-8-carboxylic acid* | | | |
| | *and* | | | |
| | *(D)-Tryptophanol* | | | |

### Example 6

### 2-Methyl-6-(3,4,5-trimethoxy-phenyl)-2H-chromene-8-carboxylic acid [(R)-1-hydroxymethyl-2-(1H-indol-3-yl)-ethyl]-amide

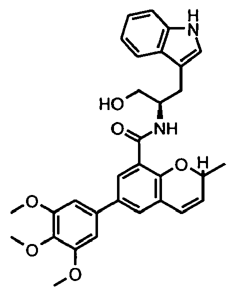

6a) 5-Iodo-2-(1-methyl-prop-2-ynyloxy)-benzoic acid methyl ester
   Ethynylcarbinol (10.0 g, 0.0358 mol), methyl-5-iodosalicylate ( 2.51 g, 0.0359 mol) and Ph₃P (9.43 g, 0.0360 mol) were dissolved in 15 ml of dry toluene under argon atmosphere with stirring. Diisopropyl azodicarboxylate (7.27 g, 0.036 mol) was added dropwise at 10 °C. The mixture was stirred overnight under argon atmosphere. The precipitate was filtered off and washed with 15 ml of dry, cooled toluene. The solvent was removed under reduced pressure. The residue was purified by column chromatography over silica gel (hexane/EtOAc 99:1). Yield of the title compound was 70 %. ESI-MS: 331 [M+1].
6b) 6-Iodo-2-methyl-2H-chromene-8-carboxylic acid methyl ester
   A solution of 5-Iodo-2-(1-methyl-prop-2-ynyloxy)-benzoic acid methyl ester (1 g) in diethylamino benzene (10 mL) was stirred in a preheated metal bath at 250°C for 10 minutes. The reaction mixture was cooled to ambient temperature and poured onto ice / concentrated HCl. The mixture was extracted with ethyl acetate and the solvent was removed under reduced pressure. The title compound was obtained in 31 % yield (310 mg) after flash chromatography. **¹H-NMR (CDCl₃):** 7.90 d (*J* = 2.3 Hz, 1H); 7.35 d (*J* = 2.3 Hz, 1H); 6.30 dd (*J* = 1.7 Hz /10.0 Hz, 1H); 5.77 dd (*J* = 3.4 Hz / 10.0 Hz, 1H); 5.11 m (1H); 3.87 s (3H); 1.46 d (*J* = 6.6 Hz, 3H).
6c) 6-Iodo-2-methyl-2H-chromene-8-carboxylic acid
   A solution of 6-Iodo-2-methyl-2H-chromene-8-carboxylic acid methyl ester (300 mg) in methanolic KOH (10%, 10 mL) was stirred at ambient temperature overnight. The solvent was distilled off under reduced pressure and the residue was extracted with water / ether. The water phase was acidified by addition of HCl (4N) and the precipitate filtered off. The title compound was obtained in 71 % yield (205 mg) after recrystallization from ethanol. **¹H-NMR (CDCl₃):** 8.25 d (*J* = 2.3 Hz, 1H); 7.48 d (*J* = 2.3 Hz, 1H); 6.39 dd (*J* = 1.7 Hz /10.1 Hz, 1H); 5.85 dd (*J* = 3.2 Hz / 10.1 Hz, 1H); 5.29 m (1H); 1.57 d (*J* = 6.8 Hz, 3H).
6d) 6-Iodo-2-methyl-2H-chromene-8-carboxylic acid ((R)-1-hydroxymethyl-2-( 1 H-indol-3-yl)-ethyl]-amide
   A solution of 6-Iodo-2-methyl-2H-chromene-8-carboxylic acid (180 mg), (D)-tryptophanol (130 mg), EDC (218 mg), HOBt (174 mg) in DMF (5 mL) was stirred at ambient temperature overnight. The reaction mixture was poured into water and the precipitate was filtered off. The title compound was obtained after flash chromatography in 68% yield (190 mg) as a 1:1 mixture of diastereomers. **ESI-MS: 489 [M+1]**.
6e) 2-Methyl-6-(3,4,5-trimethoxy-phenyl)-2H-chromene-8-carboxylic acid [(R)-1-hydroxymethyl-2-(1H-indol-3-yl)-ethyl]-amide
   A solution of 6-Iodo-2-methyl-2H-chromene-8-carboxylic acid [(R)-1-hydroxymethyl-2-(1H-indol-3-yl)-ethyl]-amide (160 mg), 3,4,5-Trimethoxy-phenyl boronic acid (78 mg), Pd(PPh₃)₄ (19 mg) in ethanol (5 mL), toluene (10 mL) and an aqueous sodium carbonate solution (2M, 2 mL) was stirred at 100°C for 3 hours. The solvent was evaporated and the solid purified by flash chromatography to yield 89% of the title compound (155 mg). The two diastereomers were separated via HPLC: **¹H-NMR (DMSO-d₆),** Diastereomer A: 10.79 s (1H); 8.27 d (*J* = 7.8 Hz, 1H); 7.92 d (*J* = 2.5 Hz, 1H); 7.67 d (*J* = 7.8 Hz, 1H); 7.52 d (*J* = 2.5 Hz, 1H); 7.30 d (*J* = 8.1 Hz, 1H); 7.14 d (*J* = 2.0 Hz, 1H); 7.03 m (1H); 6.94 m (1H); 6.81 s (2H); 6.58 dd (*J* = 9.9 Hz /1.8 Hz, 1H); 5.90 dd (*J* = 9.9 Hz / 3.0 Hz, 1H); 5.10 m (1H); 4.93 m (1H); 4.20 m (1H); 3.82 s (6H); 3.65 s (3H); 3.49 m (1H); 3.42 m (1H); 2.98 m (2H); 1.29 d (*J* = 6.6 Hz, 3H); **¹H-NMR (DMSO-dₛ), Diastereomer B:** 10,81 s (1H); 8.27 d (*J* = 8.1 Hz, 1H); 7.94 d (*J* = 2.3 Hz, 1H); 7.66 d (*J* = 7.8 Hz, 1H); 7.52 d (*J* = 2.5 Hz, 1H); 7.30 d (*J* = 8.1 Hz, 1H); 7.15 d (*J* = 2.0 Hz, 1H); 7.03 m (1H); 6.94 m (1H); 6.81 s (2H); 6.57 dd (*J* = 10.1 Hz / 1.8 Hz, 1H); 5.89 dd (*J* = 9.9 Hz / 3.0 Hz, 1H); 4.93 m (2H); 4.22 m (1H); 3.82 s (6H); 3.65 s (3H); 3.46 m (1H); 3.40 m (1H); 2.98 m (2H); 1.26 d (*J* = 6.8 Hz, 3H).

### Example 7

### 6-(3-Chloro-4-methylcarbamoyl-phenyl)-2,2-dimethyl-2H-chromene-8-carboxylic acid [(R)-2-hydroxy-1-(1H-indol-3-ylmethyl)-ethyl]-amide

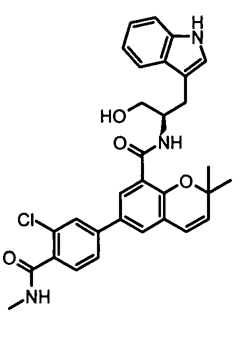

7a) 2-(1,1-Dimethyl-prop-2-ynyloxy)-5-iodo-benzoic acid methyl ester
   Ethynylcarbinol (10.0 g, 0.0358 mol), methyl-5-iodosalicylate (2.51 g, 0.0359 mol) and Ph₃P (9.43 g, 0.0360 mol) were dissolved in 15 ml of dry toluene under argon atmosphere with stirring. Diisopropyl azodicarboxylate 7.27 g (0.036 mol) was added dropwise at 10 °C. The mixture was stirred overnight under argon atmosphere. The precipitate was filtered off and washed with 15 ml of dry, cooled toluene. The solvent was removed under reduced pressure. The residue was purified by column chromatography over silica gel (hexane/EtOAc 99:1). Yield of the title compound was 46.8 %. **ESI-MS: 345 [M+1]**.
7b) 6-Iodo-2,2-dimethyl-2H-chromene-8-carboxylic acid methyl ester
   A solution of 2-(1,1-Dimethyl-prop-2-ynyloxy)-5-iodo-benzoic acid methyl ester (1.37 g) in diethylamino benzene (18 mL) was stirred in a preheated metal bath at 250°C for 90 minutes. The reaction mixture was cooled to ambient temperature and poured onto ice / concentrated HCl. The mixture was extracted with ethyl acetate and the solvent was removed under reduced pressure. The title compound was obtained in 86 % yield (1.19 g) after flash chromatography. **¹H-NMR (CDCl₃):** 7.91 d (*J* = 2.3 Hz, 1H); 7.37 d (*J* = 2.3 Hz, 1H); 6.24 d (*J* = 10.0 Hz, 1H); 5.71 d (*J* = 10.0 Hz, 1H); 3.87 s (3H); 1.46 s (6H).
7c) 6-Iodo-2,2-dimethyl-2H-chromene-8-carboxylic acid
   A solution of 6-Iodo-2,2-dimethyl-2H-chromene-8-carboxylic acid methyl ester (2.54 g), KOH (4 g) in methanol (30 mL) was stirred at ambient temperature overnight. The solvent was distilled off under reduced pressure and the residue was extracted with water / ether. The water phase was acidified by addition of HCl (4N) and the precipitate filtered off. The title compound was obtained in 69% yield (1.68 g). **¹H-NMR (CDCl₃):** 8.27 d (*J* = 2.3 Hz, 1H); 7.50 d (*J* = 2.3 Hz, 1H); 6.32 d (*J* = 10.0 Hz, 1H); 5.78 d (*J* = 10.0 Hz, 1H); 1.57 s (6H).
7d) 6-Iodo-2,2-dimethyl-2H-chromene-8-carboxylic acid [(R)-2-hydroxy-1 -(1H-indol-3-ylmethyl)-ethyl]-amide
   A solution of -Iodo-2,2-dimethyl-2H-chromene-8-carboxylic acid (1.68g), (D)-tryptophanol (1.15 g), EDC (1.17 g), HOBt (825 mg), triethylamine (1.41 mL) in DMF (40 mL) was stirred at ambient temperature overnight. The solvent was distilled off under reduced pressure. The title compound was obtained after flash chromatography in 60% yield (1.53 g). **¹H-NMR (DMSO-d₆):** 10.82 s (1H); 8.45 d (*J* = 7.9 Hz, 1H); 8.02 d (*J* = 2.3 Hz, 1H); 7.69 d (*J* = 7.7 Hz, 1H); 7.61 d (*J* = 2.3 Hz, 1H); 7.33 d (*J* = 7.9 Hz, 1H); 7.16 m (1H); 7.07 m (1H); 6.98 m (1H); 6.48 d (*J* = 10.0 Hz, 1H); 5.90 d (*J* = 10.0 Hz, 1H); 5.03 m (1H); 4.20 m (1H); 3.51 m (2H); 2.97 m (2H); 1.40 s (3H); 1.36 s (3H).
7e) 6-(3-Chloro-4-methylcarbamoyl-phenyl)-2,2-dimethyl-2H-chromene-8-carboxylic acid [(R)-2-hydroxy-1-(1H-indol-3-ylmethyl)-ethyl]-amide
   A solution of 6-Iodo-2,2-dimethyl-2H-chromene-8-carboxylic acid [(R)-2-hydroxy-1-(1H-indol-3-ylmethyl)-ethyl]-amide (200 mg), 3-Chloro-4-methylcarbamoyl-phenyl boronic acid (104 mg), Pd(PPh₃)₄ (12 mg) in ethanol (1.32 mL), toluene (2 mL) and an aqueous sodium carbonate solution (2M, 0.45 mL) was stirred in a sealed microwave reactor at 100°C for 30 minutes at 200W. The solvent was evaporated and the solid purified by flash chromatography to yield 49 % of the title compound (107 mg). **¹H-NMR (DMSO-d₆):** 10,78 s (1H); 8.51 d *(J* = 7.9 Hz, 1H); 8.36 d *(J* = 4.7 Hz, 1H); 8.05 d *(J* = 2.5 Hz, 1H); 7.72 m (2H); 7.66 m (2H); 7.47 d (*J* = 7.9 Hz, 1H); 7.30 d (*J* = 7.9 Hz, 1H); 7.12 d (*J* = 1.9 Hz, 1H); 7.03 m (1H); 6.95 m (1H); 6.54 d (*J* = 10.0 Hz, 1H); 5.89 d (*J* = 10.0 Hz, 1H); 5.72 s (2H); 5.00 m (1H); 4.20 m (1H); 3.49 m (1H); 3.44 m (1H); 2.95 m (2H); 2.73 d (*J* = 4.5 Hz, 3H); 1.40 s (3H); 1.36 s (3H).

The following compounds were obtained in analogy to the preparation methods described in detail:

| **Ex.** | **Product; *reagents*** | **Method analogous to** | **¹H-NMR (400 MHz) δ [ppm]** | **Structure** |
|---|---|---|---|---|
| **8** | 6-(3-Fluoro-5-methylcarbamoyl-phenyl)-2,2-dimethyl-2H-chromene-8-carboxylic acid [(R)-2-hydroxy-1-(1 H-indol-3-yl-methyl)-ethyl]-amide; | **7** | **¹H-NMR (DMSO-d₆):** 10,82 s (1H); 8.71 d (*J* = 4.5 Hz, 1H); 8.55 d (*J* = 7.7 Hz, 8.18 d (*J* = 2.5 Hz, 1 H); 7.99 s (1H); 7.75 d (*J* = 7.7 Hz, 1H); 7.18 d (*J* = 2.1 Hz, 1H); 7.08 m (1H); 7.02 m (1H); 6.59 d (*J* = 10.0 Hz, 1H); 5.95 d (*J* = 10.0 Hz, 1H); 5.77 s (2H); 5.04 m (1H); 4.26 m (1H); 4.09 m (1H); 3.55 m (1H); 3.49 m (1H); 3.02 d (*J* = 6.8 Hz, 2H); 2.83 d (*J* = 4.5 Hz, 3H); 1.46 s (3H); 1.42 s (3H). | 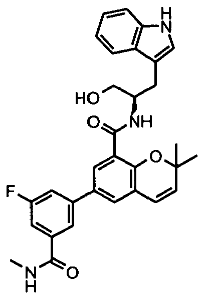 |
| | *6-Iodo-2,2-dimethyl-2H-chromene-8-carboxylic acid [(R)-1-hydroxymethyl-2-(1H-indol-3-yl)-ethyl]-amide* | | | |
| | *and* | | | |
| | *3-Fluoro-5-methylcarbamoyl-phenyl boronic acid* | | | |
| **9** | 2,2-Dimethyl-6-(3,4,5-trimethoxy-phenyl)-2H-chromene-8-carboxylic acid [(R)-2-hydroxy-1-(1H-indol-3-ylmethyl)-ethyl]-amide; | **7** | **¹H-NMR (DMSO-d₆):** 10,78 s (1H); 8.53 d (*J* = 8.1 Hz, 1H); 8.01 d (*J* = 2.5 Hz, 1H); 7.70 d (*J* = 7.5 Hz, 1H); 7.55 d (*J* = 7.30 Hz, 1H); 7.12 d (*J* = 2.1 Hz, 1H); 7.03 m (1H); 6.95 m (1H); 6.83 s (2H); 6.53 d (*J* = 10.0 Hz, 1H); 5.89 d (*J* = 10.0 Hz, 1H); 4.23 m (1H); 3.83 s (6H); 3.65 s (3H); 3.49 m (1H); 3.42 m (1H); 2.95 m (2H); 1.40 s (3H); 1.36 s (3H). | 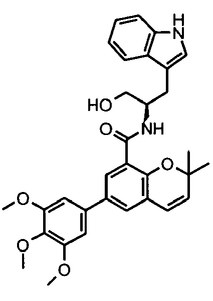 |
| | *6-Iodo-2,2-dimethyl-2H-chromene-8-carboxylic acid [(R)-1-hydroxymethyl-2-(1H-indol-3-yl)-ethyl]-amide* | | | |
| | *and* | | | |
| | *3,4,5-Trimethoxy-phenyl boronic acid* | | | |

### Example 10

### 2,2-Dimethyl-6-(4-methylcarbamoyl-phenylethynyl)-2H-chromene-8-carboxylic acid [(R)-1-hydroxymethyl-2-(1H-indol-3-yl)-ethyl]-amide

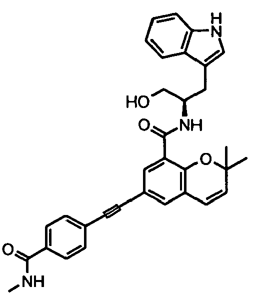

10a) 2,2-Dimethyl-6-(4-methylcarbamoyl-phenylethynyl)-2H-chromene-8-carboxylic acid [(R)-1-hydroxymethyl-2-(1H-indol-3-yl)-ethyl]-amide
   A solution of 6-Iodo-2,2-dimethyl-2H-chromene-8-carboxylic acid [(R)-2-hydroxy-1-(1H-indol-3-ylmethyl)-ethyl]-amide (120 mg), Pd(PPh₃)₂Cl₂ (5.5 mg), 4-Ethynyl-N-methylbenzamide (42 mg), TBAF x 3 water (190 mg) in THF (3 mL) and ethanol (0.3 mL) was stirred at 100°C in a sealed microwave reactor for 30 min (200W). The solvents were distilled off under reduced pressure, the crude mixture was taken up in dichlormethane and extracted with water. The solvent of the organic phase was removed and the title compound was obtained in 27% yield (35 mg) after flash chromatography. **¹H-NMR (DMSO-d₆):** 10,78 s (1H); 8.50 d (*J* = 4.7 Hz, 1H); 8.43 d (*J* = 8.1 Hz, 1H); 7.89 d (*J* = 2.3 Hz, 1H); 7.83 d (*J* = 8.5 Hz, 1H); 7.67 d (J = 7.5 Hz, 1H); 7.60 d (J = 8.5 Hz, 1H); 7.44 d (*J* = 2.3 Hz, 1H); 7.29 d (J = 7.9 Hz, 1H); 7.12 d (*J* = 2.3 Hz, 1H); 7.03 m (1H); 6.94 m (1H); 6.48 d (*J* = 10.0 Hz, 1H); 5.89 d (*J* = 10.0 Hz, 1H); 4.99 m (1H); 4.19 m (1H); 3.48 m (2H); 2.95 m (2H); 2.75 d (*J* = 4.5 Hz, 3H); 1.39 s (3H); 1.34 s (3H).

The following compounds were obtained in analogy to the preparation methods described in detail:

| **Ex.** | **Product; *reagents*** | **Method analogous to** | **¹H-NMR (400 MHz) δ [ppm]** | **Structure** |
|---|---|---|---|---|
| **11** | 2,2-Dimethyl-6-(3-methyl-carbamoyl-phenylethynyl)-2H-chromene-8-carboxylic acid [(R)-1-hydroxymethyl-2-(1H-indol-3-yl)-ethyl]-amide; | **10** | **¹H-NMR (DMSO-d₆):** 10,83 s (1H); 8.56 d (J = 3.0 Hz, 1H); 8.45 d (J = 7.5 Hz, 1H); 8.01 s (1H); 7.94 d (*J* = 2.3 Hz, 1H); 7.86 d (*J* = 7.7 Hz, 1H); 7.71 m (2H); 7.54 d (*J* = 7.7 Hz, 1H); 7.48 d (*J* = 2.1 Hz, 1H); 7.34 d (*J* = 7.7 Hz, 1H); 7.17 d (*J* = 2.1 Hz, 1H); 7.08 m (1H); 6.99 m (1H); 6.53 d (*J* = 10.0 Hz, 1H); 5.94 d (*J* = 10.0 Hz, 1H); 5.03 m (1 H); 4.26 m (1H); 3.49 m (2H); 2.99 m (2H); 2.80 d (*J* = 4.5 Hz, 3H); 1.44 s (3H); 1.39 s (3H). | 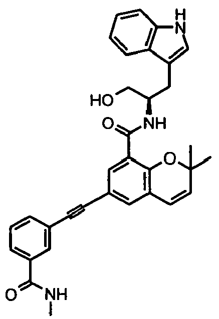 |
| | *6-Iodo-2,2-dimethyl-2H-chromene-8-carboxylic acid [(R)-1-hydroxymethyl-2-(1H-indol-3-yl)-ethyl]-amide* | | | |
| | *and* | | | |
| | *3-Ethynyl-N-methyl-benzamide* | | | |

### Example 12

### 5-(3,4-Dimethoxy-phenyl)-2-vinyl-2,3-dihydro-benzofuran-7-carboxylic acid [(R)-1-hydroxymethyl-2-(1H-indol-3-yl)-ethyl]-amide

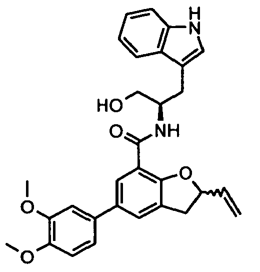

12a) (R)-2-{[5-(3,4-Dimethoxy-phenyl)-2-vinyl-2,3-dihydro-benzofuran-7-carbonyl]-amino}-3-(1 H-indol-3-yl)-propionic acid methyl ester
   A solution of 5-(3,4-Dimethoxy-phenyl)-2-vinyl-2,3-dihydro-benzofuran-7-carboxylic acid (300 mg), (D)-tryptophan methyl ester hydrochloride (280 mg), EDC (210 mg), triethylamine (0.78 mL) and HOBt (170 mg) in DMF (25 mL) was stirred at ambient temperature overnight. The solvent was distilled off under reduced pressure. The title compound was obtained after flash chromatography in 64% yield (308 mg) as a 1:1 mixture of diastereomers. **ESI-MS [M+1]**: 527.
12b) 5-(3,4-Dimethoxy-phenyl)-2-vinyl-2,3-dihydro-benzofuran-7-carboxylic acid [(R)-1-hydroxymethyl-2-(1 H-indol-3-yl)-ethyl]-amide
   To a solution of (R)-2-{[5-(3,4-Dimethoxy-phenyl)-2-vinyl-2,3-dihydro-benzofuran-7-carbonyl]-amino}-3-(1 H-indol-3-yl)-propionic acid methyl ester (135 mg) in THF (5 mL) was added slowly via a syringe a solution of lithiumborohydride in THF (2M, 0.26 mL). The reaction was allowed to stir at ambient temperature overnight and quenched by the addition of HCl (1N). The reaction mixture was extracted with ethyl acetate / water and the combined organic phases were dried over sodium sulphate. The title compound was obtained as a 1:1 mixture of diastereomers after flash chromatography in 80% yield (102 mg). **ESI-MS [M+1]:** 499.

### Example 13

### 5-(3,4-Dimethoxy-phenyl)-2-ethyl-2,3-dihydro-benzofuran-7-carboxylic acid [(R)-1-hydroxymethyl-2-(1H-indol-3-yl)-ethyl]-amide

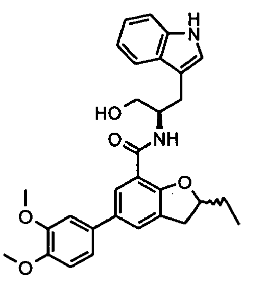

13a) 5-(3,4-Dimethoxy-phenyl)-2-ethyl-2,3-dihydro-benzofuran-7-carboxylic acid
   A suspension of 5-(3,4-Dimethoxy-phenyl)-2-vinyl-2,3-dihydro-benzofuran-7-carboxylic acid (100 mg) and palladium on charcoal (50 mg) in THF (6 mL) were hydrogenated at ambient temperature for 40 minutes. The catalyst was removed by filtration, the sovent was distilled off under reduced pressure. The title compound was obtained in 35 % yield (35 mg) after flash chromatography. **¹H-NMR (CDCl₃:** 8.02 m (1H); 7.58 m (1H); 7.09 m (1H); 7.05 m (1H); 6.93 d (*J* = 8.3 Hz, 1H); 5.07 m (1H); 3.95 s (3H); 3.92 s (3H); 3.42 m (1H); 3.05 m (1H); 1.97 m (1H); 1.85 m (1H); 1.09 m (3H).
13b) 5-(3,4-Dimethoxy-phenyl)-2-ethyl-2,3-dihydro-benzofuran-7-carboxylic acid [(R)-1-hydroxymethyl-2-(1H-indol-3-yl)-ethyl]-amide
   A solution of 5-(3,4-Dimethoxy-phenyl)-2-ethyl-2,3-dihydro-benzofuran-7-carboxylic a cid (35 mg), (D)-tryptophanol (21 mg), EDC (22 mg) and HOBt (17 mg) in DMF (2 mL) was stirred at ambient temperature overnight. The solvent was distilled off under reduced pressure. The title compound was obtained after flash chromatography in 84% yield (84 mg) as a 1:1 mixture of stereoisomers. The two diastereomers were separated via HPLC. **¹H-NMR (DMSO-d₆): Diastereomer A:** 10,78 s (1H); 7.93 d *(J* = 7.9 Hz, 1 H); 7.84 d (*J* = 2.1 Hz, 1H); 7.66 d (*J* = 7.9 Hz, 1H); 7.61 d (*J* = 1.7 Hz, 1H); 7.29 d (*J* = 7.9 Hz, 1H); 7.09 m (4H); 6.99 m (2H); 4.93 m (2H); 4.22 m (1H); 3.80 s (3H); 3.74 s (3H); 3.48 m (1H); 3.40 m (1H); 2.94 m (3H); 1.69 m (2H); 0.93 m (3H). **Diastereomer B:** 10,80 s (1H); 7.95 d (*J* = 8.1 Hz, 1H); 7.84 d (*J* = 2.1 Hz, 1H); 7.66 d (*J* = 7.9 Hz, 1H); 7.60 d (*J* = 1.9 Hz, 1H); 7.30 d (*J* = 8.1 Hz, 1H); 7.13 d (*J* = 2.1 Hz, 1H); 7.09 s (1H); 6.99 m (4H); 4.92 m (1H); 4.79 m (1H); 4.21 m (1H); 3.38 m (1H); 3.33 m (1H); 2.96 m (2H); 2.89 m (1H); 1.66 m (2H); 0.93 m (3H).

### Example 14

### 7-(3-Chloro-4-methylcarbamoyl-phenyl)-2,3-dihydro-benzo[1,4]dioxine-5-carboxylic acid [(R)-2-hydroxy-1-(1 H-indol-3-ylmethyl)-ethyl]-amide

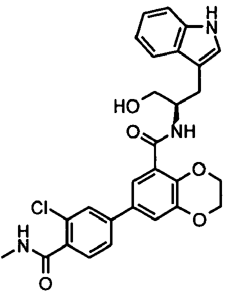

14a) 7-Bromo-2,3-dihydro-benzo[1,4]dioxine-5-carboxylic acid
   The title compound was prepared according to a literature procedure by V. K. Daukshas et. al. in Chem. Heterocyclic Compds. Engl Transl. 1978, 1188.
14b) 7-Bromo-2,3-dihydro-benzo[1,4]dioxine-5-carboxylic acid [(R)-2-hydroxy-1-(1H-indol-3-ylmethyl)-ethyl]-amide
   A solution of 7-Bromo-2,3-dihydro-benzo[1,4]dioxine-5-carboxylic acid (1 g), (D)-tryptophanol (808 mg), EDC (814 mg) and HOBt (650 mg) in DMF (15 mL) was stirred at ambient temperature overnight. The solvent was distilled off under reduced pressure. The title compound was obtained after flash chromatography in 76% yield (1.27 g). ¹H-NMR **(DMSO-d**₆): 10.84 s (1H); 7.99 m (1H); 7.66 d (*J* = 7.9 Hz, 1H); 7.37 m (2H); 7.20 d (*J* = 2.6 Hz, 1H); 7.19 d (*J* = 2.3 Hz, 1H); 7.07 m (1H); 6.99 m (1H); 4.89 m (1H); 4.28 m (4H); 4.23 m (1H); 3.53 m (1H); 3.47 m (1H); 2.99 m (2H).
14c) 7-(3-Chloro-4-methylcarbamoyl-phenyl)-2,3-dihydro-benzo[1,4]dioxine-5-carboxylic acid [(R)-2-hydroxy-1-(1H-indol-3-ylmethyl)-ethyl]-amide
   A solution of 7-Bromo-2,3-dihydro-benzo[1,4]dioxine-5-carboxylic acid [(R)-2-hydroxy-1-(1H-indol-3-ylmethyl)-ethyl]-amide (200 mg), 3-Chloro-4-methylcarbamoyl-phenyl boronic acid (109 mg), Pd(PPh₃)₄ (54 mg) in ethanol (6.2 mL), toluene (6.2 mL) and an aqueous sodium carbonate solution (1M, 1.16 mL) was stirred at 100°C overnight. The solvent was evaporated and the solid purified by flash chromatography to yield 43 % of the title compound (103 mg). **¹H-NMR (DMSO-d₆):** 10,80 s (1H); 8.33 d (*J* = 4.7 Hz, 3H); 7.99 d (*J* = 7.9 Hz, 3H); 7.67 m (2H); 7.55 m (3H); 7.43 d (*J* = 7.9 Hz, 3H); 7.33 d *(J* = 2.5 Hz, 3H); 7.31 d *(J* = 8.1 Hz, 3H); 7.16 d *(J* = 2.3 Hz, 3H); 7.03 m (1H); 6.95 m (1H); 4.86 m (1H); 4.28 m (4H); 4.23 m (1H); 3.47 m (1H); 3.41 m (1H); 2.96 m (2H); 2.73 d (*J* = 4.5 Hz, 3H).

The following compounds were obtained in analogy to the preparation methods described in detail:

| **Ex.** | **Product; *reagents*** | **Method analogous to** | **¹H-NMR (400 MHz) δ [ppm]** | **Structure** |
|---|---|---|---|---|
| **15** | 7-(3,4,5-Trimethoxy-phenyl)-2,3-dihydro-benzo[1,4]-dioxine-5-carboxylic acid [(R)-1-hydroxymethyl-2-(1H-indol-3-yl)-ethyl]-amide; *7-Bromo-2,3-dihydro-*benzo*[*1,*4]dioxine-5-carboxylic acid [(R)-1-hydroxy-methyl-2-(1H-indol-3-yl)-ethyl]-amide and 3,4,5-Trimethoxy-phenyl boronic acid* | **14** | **¹H-NMR (DMSO-d₆):** 10,80 s (1H); 7.99 d (*J* = 7.7 Hz, 3H); 7.64 d (*J* = 7.9 Hz, 3H); 7.53 d (*J* = 2.5 Hz, 3H); 7.28 m (2H); 7.16 d (*J* = 2.1 Hz, 3H); 7.03 m (1H); 6.94 m (1H); 6.78 s (2H); 4.87 m (1H); 4.27 m (4H); 4.22 m (1H); 3.81 s (6H); 3.64 s (3H); 3.47 m (1H); 3.40 m (1H); 2.95 m (2H). | 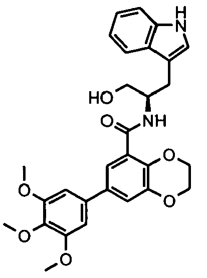 |
| **16** | 7-(3-Fluoro-5-methylcarbamoyl-phenyl)-2,3-dihydro-benzo[1,4]dioxine-5-carboxylic acid [(R)-2-hydroxy-1-(1H-indol-3-ylmethyl)-ethyl]-amide; *7-Bromo-2,3-dihydro-benzo[1, 4]dioxine-5-carboxylic acid [(R)-1-hydroxy-methyl-2-(1H-indol-3-yl)-ethyl]-amide and 3-Fluoro-5-methylcarbamoyl-phenyl boronic acid* | **14** | **¹H-NMR (DMSO-d₆):** 10,81 s (1H); 8.65 d (J = 2.6 Hz, 3H); 7.99 d (J = 7.9 Hz, 3H); 7.90 s (1H); 7.64 m (2H); 7.60 m (1H); 7.54 m (1H); 7.40 d (*J*= 2.5 Hz, 3H); 7.30 d (*J* = 8.1 Hz, 3H); 7.16 d (*J* = 2.1 Hz, 3H); 7.02 m (1H); 6.95 m (1H); 4.87 m (1H); 4.29 m (4H); 4.22 m (1H); 3.48 m (1H); 3.41 m (1H); 2.96 m (2H); 2.77 d (*J* = 4.5 Hz, 3H). | 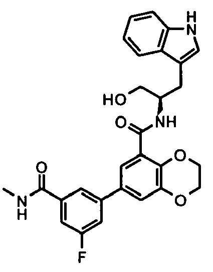 |
| **17** | 7-(3-Fluoro-5-methylcarbamoyl-phenyl)-2,3-dihydro-benzo[1,4]-dioxine-5-carboxylic acid [2-(6-fluoro-1H-indol-3-yl)-1-hydroxymethyl-ethyl]-amide; *7-Bromo-2,3-dihydro-benzo[1, 4]dioxine-5-carbo-xylic acid [2-(6-fluoro-1H-indol-3-yl)-1-hydroxymethyl-ethyl]-amide and 3-Fluoro-5-methylcarbamoyl-phenyl boronic acid* | **14** | **¹H-NMR (DMSO-d₆):** 10,88 s (1H); 8.66 d (*J* = 4.5 Hz, 1H); 8.02 d (*J* = 7.9 Hz, 1H); 7.91 s (1 H); 7.63 m (4H); 7.40 d (*J* = 2.5 Hz, 1H); 7.16 d (*J* = 2.1 Hz, 1H); 7.08 dd (*J* = 2.3 Hz / 10.2 Hz, 1H); 6.82 m (1H); 4.88 m (1H); 4.30 m (4H); 4.18 m (1H); 3.47 m (1H); 3.40 m (1H); 2.94 m (2H); 2.77 d (*J* = 4.5 Hz, 3H). | 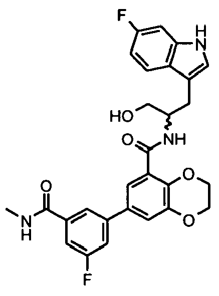 |
| **18** | 7-(3,4,5-Trimethoxy-phenyl)-2,3-dihydro-benzo-[1,4]-dioxine-5-carboxylic acid [2-(6-fluoro-1H-indol-3-yl)-1-hydroxymethyl-ethyl]-amide; *7-Bromo-2,3-dihydro-benzo-[1,4]dioxine-5-carboxylic acid [2-(6-fluoro-1H-indol-3-yl)-1-hydroxymethyl-ethyl]-amide and 3,4,5-Trimethoxy-phenyl boronic acid* | **14** | **¹H-NMR (DMSO-d₆):** 10,88 s (1H); 7.99 d (*J* = 7.9 Hz, 1H); 7.64 m (1H); 7.51 d (*J* = 2.3 Hz, 1H); 7.29 d (*J* = 2.5 Hz, 1H); 7.16d(J=1.8Hz, 1H); 7.06 dd (*J* = 2.1 Hz /10.0 Hz, 1H); 6.85 m (1H); 6.77 s (2H); 4.88 m (1H); 4.29 m (4H); 4.15 m (1H); 3.81 s (6H); 3.64 s (3H); 3.46 m (1H); 3.39 m (1H); 2.93 m (2H). | 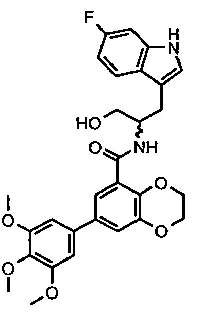 |
| **19** | 7-(3-Chloro-4-methylcarbamoyl-phenyl)-2,3-dihydro-benzo[1,4]-dioxine-5-carboxylic acid [2-(6-fluoro-1H-indol-3-yl)-1-hydroxymethyl-ethyl]-amide; *7-Bromo-2,3-dihydro-benzo[1,4]dioxine-5-carboxylic acid [2-(6-fluoro-1H-indol-3-yl)-1-hydroxymethyl-ethyl]-amide and 3-Chloro-4-methylcarbamoyl-phenyl boronic acid* | **14** | **¹H-NMR (DMSO-d₆):** 10,87 s (1H); 8.33 d (*J* = 4.7 Hz, 1H); 8.01 d (*J* = 7.9 Hz, 1H); 7.67 m (4H); 7.44 d (*J* = 8.1 Hz, 1H); 7.34 d (*J* = 2.5 Hz, 1H); 7.15 d (*J* = 2.1 Hz, 1H); 7.09 dd (*J* = 2.3 Hz/10.1 Hz, 1H); 6.82 m (1H); 4.88 m (1H); 4.30 m (4H); 4.08 m (1H); 3.48 m (1H); 3.38 m (1H); 2.93 m (2H); 2.72 d (J = 4.5 Hz, 3H). | 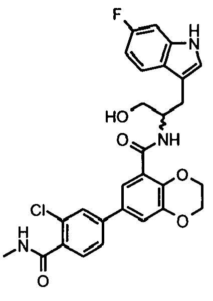 |

### Example 20

### 5-(3,4-Difluoro-5-methoxy-phenyl)-2,3-dihydro-benzofuran-7-carboxylic acid [(R)-2-hydroxy-1-(1H-indol-3-ylmethyl)-ethyl]-amide

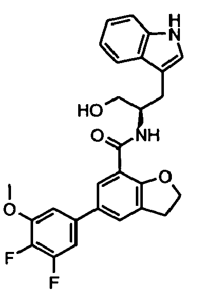

20a) 5-(3,4-Difluoro-5-methoxy-phenyl)-2,3-dihydro-benzofuran-7-carboxylic acid methyl ester
   A solution of 5-Bromo-2,3-dihydro-benzofuran-7-carboxylic acid methyl ester (150 mg), 3,4-Difluoro-5-methoxy-phenyl boronic acid (132 mg), Pd(PPh₃)₄ (67 mg) in ethanol (6.3 mL), toluene (6.3 mL) and an TBAF solution in THF (1M, 1.17 mL) was stirred at reflux overnight. The solvents were evaporated and the solid purified by flash chromatography to yield 42 % of the title compound (79 mg). **¹H-NMR (DMSO-d₆**): 7.82 s (2H); 7.20 m (2H); 4.69 m (2H); 3.96 s (3H); 3.82 s (3H); 3.27 m (2H).
20b) 5-(3,4-Difluoro-5-methoxy-phenyl)-2,3-dihydro-benzofuran-7-carboxylic acid
   A solution of 5-(3,4-Difluoro-5-methoxy-phenyl)-2,3-dihydro-benzofuran-7-carboxylic acid methyl ester (67 mg) and KOH (175 mg) in methanol (2.3 mL) was stirred at ambient temperature for four days. The solvent was distilled off under reduced pressure and the residue was extracted with water / ether. The water phase was acidified by addition of HCl (4N) and the precipitate filtered off. The title compound was obtained in 91 % yield (58 mg). **¹H-NMR (DMSO-d₆):** 12.80 s (1H); 7.79 m (2H); 7.17 m (2H); 4.67 m (2H); 3.96 s (3H); 3.26 m (2H).
20c) 5-(3,4-Difluoro-5-methoxy-phenyl)-2,3-dihydro-benzofuran-7-carboxylic acid [(R)-2-hydroxy-1-(1H-indol-3-ylmethyl)-ethyl]-amide
   A solution of 5-(3,4-Difluoro-5-methoxy-phenyl)-2,3-dihydro-benzofuran-7-carboxylic acid (58 mg), (D)-tryptophanol (33 mg), EDC (40 mg) and HOBt (28 mg) in DMF (1.6 mL) was stirred at ambient temperature overnight. The solvent was distilled off under reduced pressure. The title compound was obtained after flash chromatography in 50% yield (41 mg). **¹H-NMR (DMSO-d₆):** 10,83 s (1H); 7.89 m (2H); 7.74 s (1H); 7.70 d *(J* = 7.8 Hz, 1H); 7.33 d *(J =* 8.2 Hz, 1H); 7.19 m (3H); 7.06 m (1H); 6.98 m (1H); 4.98 m (1H); 4.72 m (2H); 4.25 m (1H); 3.96 s (3H); 3.51 m (1H); 3.41 m (1H); 3.26 m (2H); 2.98 m (2H).

The following compounds were obtained in analogy to the preparation methods described in detail:

| **Ex.** | **Product; *reagents*** | **Method analogous to** | **¹H-NMR (400 MHz) δ [ppm]** | **Structure** |
|---|---|---|---|---|
| **21** | 5-(3,4-Dimethoxy-phenyl)-2,3-dihydro-benzofuran-7-carboxylic acid [(R)-1-hydroxymethyl-2-(1 H-indol-3-yl)-ethyl]-amide; *5-(3, 4-Dimethoxy-phenyl)-2, 3-dihydro-benzofuran-7-carboxylic acid and (D)-Tryptophanol* | **20** | **¹H-NMR (DMSO-d₆):** 10,84 s (1H); 7.87 m (2H); 7.69 m (2H); 7.33 d (*J* = 7.8 Hz, 1H); 7.17-6.96 m (6H); 4.98 m (1H); 4.70 m (2H); 4.25 m (1H); 3.83 s (3H); 3.78 s (3H); 3.52 m (1H); 3.41 m (1H); 3.28 m (2H); 2.98 m (2H). | 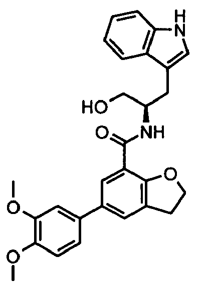 |
| **22** | 5-(3-Methoxy-phenyl)-2,3-dihydro-benzofuran-7-carbo-xylic acid [(R)-1-hydroxy-methyl-2-(1H-indol-3-yl)-ethyl]-amide; *5-(3-Methoxy-phen yl)-2, 3-dihydro-benzofuran-7-carboxylic acid and (D)-Tryptophanol* | **20** | **¹H-NMR (DMSO-d₆):** 10,84 s (1H); 7.89 m (2H); 7.71 m (2H); 7.35 m (2H); 7.17 m (2H); 7.11 s (1H); 7.06 m (1H); 6.98 m (1H); 6.90 dd (*J* = 1.9 Hz / 8.2 Hz, 1H); 4.98 m (1H); 4.71 m (2H); 4.25 m (1H); 3.81 s (3H); 3.52 m (1H); 3.41 m (1H); 3.28 m (2H); 2.99 m (2H). | 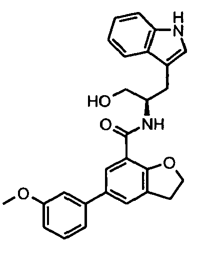 |
| **23** | 5-Benzo[1,3]dioxol-5-yl-2,3-dihydro-benzofuran-7-carboxylic acid [(R)-1-hydroxy-methyl-2-(1H-indol-3-yl)-ethyl]-amide; *5-Benzo[1, 3]dioxol-5-yl-2,3-dihydro-benzofuran-7-carboxylic acid and (D)-Tryptophanol* | **20** | **¹H-NMR (DMSO-d₆):** 10,83 s (1H); 7.87 d (*J* = 7.8 Hz, 1H); 7.82 s (1H); 7.70 d (*J*=7.8 Hz, 1H); 7.62 s (1H); 7.33 d (*J* = 8.2 Hz, 1H); 7.16 m (2H); 7.06 m (2H); 6.97 m (2H); 6.04 s (2H); 4.97 m (1H); 4.68 m (2H); 4.24 m (1H); 3.50 m (1H); 3.40 m (1H); 3.26 m (2H); 2.99 m (2H). | 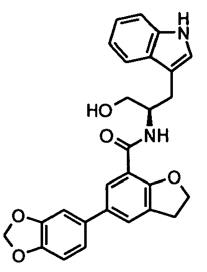 |
| **24** | 5-(3-Fluoro-4-methoxy-phenyl)-2,3-dihydro-benzo-furan-7-carboxylic acid [(R)-2-hydroxy-1-(1H-indol-3-ylmethyl)-ethyl]-amide; *5-(3-Fluoro-4-methoxy-phenyl)-2,3-dihydro-benzo-furan-7-carboxylic acid and (D)-Tryptophanol* | **20** | **¹H-NMR (DMSO-d₆):** 10,84 s (1H); 7.86 m (2H); 7.70 d (*J* = 7.8 Hz, 1H); 7.68 s (1H); 7.45 m (1H); 7.39 d (*J* = 8.6 Hz, 1H); 7.33 d (*J* = 7.8 Hz, 1H); 7.21 m (1H); 7.17 m (1H); 7.06 m (1H); 6.98 m (1H); 4.98 m (1H); 4.70 m (2H); 4.25 m (1H); 3.86 s (3H); 3.51 m (1H); 3.44 m (1H); 3.27 m (2H); 2.99 m (2H). | 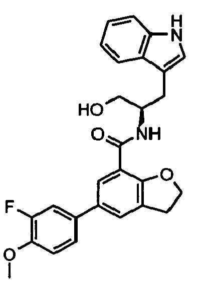 |
| **25** | 5-(3,4,5-Trimethoxy-phenyl)-2,3-dihydro-benzofuran-7-carboxylic acid [(R)-1-hydroxymethyl-2-(1H-indol-3-yl)-ethyl]-amide; *5-(3,4,5-Trimethoxy-phenyl)-2,3-dihydro-benzofuran-7-carboxylic acid and (D)-Tryptophanol* | **20** | **¹H-NMR (DMSO-d₆):** 10,84 s (1H); 7.89 m (2H); 7.33 d (*J* = 7.8 Hz, 1H); 7.17 m (1H); 7.06 m (1H); 6.98 m (1H); 6.83 s (2H); 4.98 m (1H); 4.71 m (2H); 4.25 m (1H); 3.85 s (6H); 3.68 s (3H); 3.51 m (1H); 3.41 m (1H); 3.29 m (2H); 2.98 m (2H). | 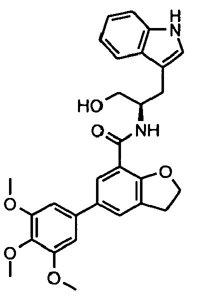 |

### Example 26

### 7-(3-Chloro-4-methylcarbamoyl-phenyl)-2,3,4,5-tetrahydro-benzo[b]oxepine-9-carboxylic acid [(R)-2-hydroxy-1-(1 H-indol-3-ylmethyl)-ethyl]-amide

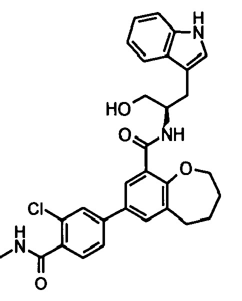

26a) 7-Bromo-2,3,4,5-tetrahydro-benzo[b]oxepine-9-carboxylic acid
   A solution of 7-Bromo-2,3,4,5-tetrahydro-benzo[b]oxepine-9-carboxylic acid methyl ester (10 g, preparation described in example 32) and KOH (10 g) in methanol (100 mL) was stirred at ambient temperature overnight. The solvent was distilled off under reduced pressure and the residue was extracted with water / ether. The water phase was acidified by addition of HCl (4N) and the precipitate filtered off. The title compound was obtained in 95% yield (9 g). **¹H-NMR (DMSO-d₆):** 12.96 s (1H); 7.53 d (*J* = 2.8 Hz, 1H); 7.48 d (*J* = 2.8 Hz, 1H); 3.90 m (2H); 2.72 m (2H); 1.85 m (2H); 1.60 m (2H).
26b) 7-Bromo-2,3,4,5-tetrahydro-benzo[b]oxepine-9-carboxylic acid [(R)-2-hydroxy-1-(1 H-indol-3-ylmethyl)-ethyl]-amide
   A solution of 7-Bromo-2,3,4,5-tetrahydro-benzo[b]oxepine-9-carboxylic acid (2.2 g), (D)-tryptophanol (1.54 g), EDC (1.87 g), HOBt (1.49 g) and triethylamine (2.25 mL) in DMF (8 mL) was stirred at ambient temperature overnight. The solvent was distilled off under reduced pressure. The title compound was obtained after flash chromatography in 69 % yield (3.6 g). **¹H-NMR (DMSO-d₆):** 10.78 s (1H); 8.33 d *(J =* 8.1 Hz, 1H); 7.63 m (2H); 7.51 d (*J* = 2.5 Hz, 1H); 7.29 d (*J* = 8.1 Hz, 1H); 7.12 d (*J* = 2.3 Hz, 1H); 7.02 m (1H); 6.94 m (1H); 4.87 m (1H); 4.15 m (1H); 3.81 m (2H); 3.49 m (1H); 3.41 m (1H); 2.96 m (1H); 2.89 m (1H); 2.71 m (2H); 1.83 m (2H); 1.58 m (2H).
26c) 7-(3-Chloro-4-methylcarbamoyl-phenyl)-2,3,4,5-tetrahydro-benzo[b]oxepine-9-carboxylic acid [(R)-2-hydroxy-1-(1H-indol-3-ylmethyl)-ethyl]-amide
   A solution of 7-Bromo-2,3,4,5-tetrahydro-benzo[b]oxepine-9-carboxylic acid [(R)-2-hydroxy-1-(1H-indol-3-ylmethyl)-ethyl]-amide (100 mg), 3-Chloro-4-methylcarbamoylphenyl boronic acid (49 mg), Pd(PPh₃)₄ (8 mg) in ethanol (1 mL), toluene (1 mL) and an aqueous sodium carbonate solution (2M, 0.23 mL) was stirred in a sealed microwave reactor at 100°C for 30 minutes at 200W. The solvent was evaporated and the solid purified by flash chromatography to yield 58 % of the title compound (69 mg). **¹H-NMR (DMSO-d₆):** 10,79 s (1H); 8.38 m (1H); 7.84 d (*J* = 2.3 Hz, 1H); 7.72 d (*J* = 1.5 Hz, 1H); 7.70 d (*J* = 2.3 Hz, 1H); 7.66 d (*J* = 7.8 Hz, 1H); 7.62 m (1H); 7.46 d (*J* = 8.1 Hz, 1H); 7.30d(*J*=7.8Hz, 1H);7.14m(1H); 7.03 m (1H); 6.95 m (1H); 4.89 m (1H); 4.21 m (1H); 3.87 m (2H); 3.49 m (1H); 3.43 m (1H); 2.98 m (2H); 2.82 m (2H); 2.73 d (J = 4.0 Hz, 3H); 1.87 m (2H); 1.65 m (2H).

The following compounds were obtained in analogy to the preparation methods described in detail:

| **Ex.** | **Product; *reagents*** | **Method analogous to** | **¹H-NMR (400 MHz) δ [ppm]** | **Structure** |
|---|---|---|---|---|
| **27** | 7-(4-Carbamoyl-3-chloro-phenyl)-2,3,4,5-tetrahydro-benzo[b]oxepine-9-carboxylic acid [(R)-2-hydroxy-1-(1H-indol-3-ylmethyl)-ethyl]-amide; *7-Bromo-2, 3, 4, 5-tetrahydro-benzo[b]oxepine-9-carboxylic acid [(R)-2-hydroxy-1-(1H-indol-3-ylmethyl)-ethyl]-amide and* 3-Chloro-4-carbamoyl-phenyl boronic acid | **26** | **¹H-NMR (DMSO-d₆):** 10.79 s (1H); 8.39 d (*J* = 8.1 Hz, 1H); 7.89 s (1H); 7.84 d (*J* = 2.5 Hz, 1H); 7.71 d (*J* = 1.8 Hz, 1H); 7.69 d (*J* = 2.5 Hz, 1H);7.66 d *(J* = 7.6 Hz, 1 H); 7.60 m (2H); 7.49 d (J = 8.1 Hz, 1H); 7.29 d (*J* = 8.1 Hz, 1H); 7.14 d (*J* = 2.3 Hz, 1H); 7.03 m (1H); 6.95 m (1H); 4.89 m (1H); 4.20 m (1H); 3.87 m (2H); 3.49 m (1H); 3.43 m (1H); 2.95 m (2H); 2.82 m (2H); 1.87 m (2H); 1.65 m (2H). | 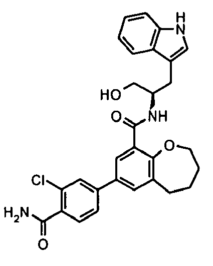 |
| **28** | 7-[3-Chloro-4-(morpholine-4-carbonyl)-phenyl]-2,3,4,5-tetrahydro-benzo[b]oxepine-9-carboxylic acid [(R)-2-hydroxy-1-(1 H-indol-3-yl-methyl)-ethyl]-amide; *7-Bromo-2, 3, 4, 5-tetrahydro-benzo[b]oxepine-9-carboxylic acid [(R)-2-hydroxy-1-(1H-indol-3-ylmethyl)-ethyl]-amide and 3-Chloro-4-(morpholine-4-carbonyl)-phenyl boronic acid* | **26** | **¹H-NMR (DMSO-d₆):** 10.78 s (1H); 8.37 d (*J* = 7.9 Hz, 1H); 7.83 d (*J* = 2.5 Hz, 1H); 7.75 d (*J* = 1.5 Hz, 1H); 7.67 m (3H); 7.43 d (*J* = 7.9 Hz, 1H); 7.29 d (*J* = 7.9 Hz, 1H); 7.13 d (*J* = 2.1 Hz, 1H); 7.02 m (1H); 6.94 m (1H); 4.88 m (1H); 4.19 m (1H); 3.87 m (2H); 3.64 m (4H); 3.52 m (4H); 3.49 m (1H); 3.12 m (2H); 2.96 m (2H); 2.82 m (2H); 1.87 m (2H); 1.65 m (2H). | 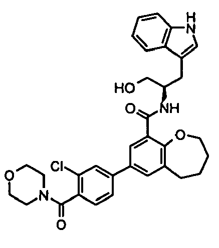 |
| **29** | 7-(3-Chloro-4-methylcarbamoyl-phenyl)-2,3,4,5-tetrahydro-benzo[b]oxepine-9-carboxylic acid [1-(6-fluoro-1H-indol-3-ylmethyl)-2-hydroxy-ethyl]-amide; *7-Bromo-2,3,4,5-tetrahydrobenzo[b]oxepine-9-carboxylic acid [1-(6-fluoro-1H-indol-3-ylmethyl)-2-hydroxy-ethyl]-amide and 3-Chloro-4-methylcarbamoyl-phenyl boronic acid* | **26** | **¹H-NMR (DMSO-d₆):** 10.86 s (1H); 8.38 m (2H); 7.84 d (*J* = 2.6 Hz, 1H); 7.72 d (*J* = 1.7 Hz, 1H); 7.69 d (*J* = 2.5 Hz, 1H); 7.63 m (2H); 7.46 d (*J* = 7.9 Hz, 1H); 7.14 d (*J* = 1.9 Hz, 1H); 7.06 dd (*J* = 2.5 Hz / 10.4 Hz, 1H); 6.82 m (1H); 4.89 m (1H); 4.17 m (1H); 3.89 m (2H); 3.48 m (1H); 3.43 m (1H); 2.91 m (2H); 2.85 m (2H); 2.73 d (J = 4.5 Hz, 3H); 1.88 m (2H); 1.65 m (2H). | 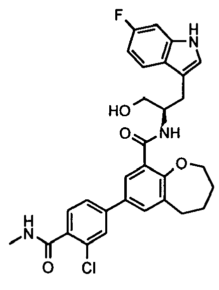 |
| **30** | 7-(4-Carbamoyl-3-chloro-phenyl)-2,3,4,5-tetrahydro-benzo[b]oxepine-9-carboxylic acid [1-(6-fluoro-1H-indol-3-ylmethyl)-2-hydroxy-ethyl]-amide; *7-Bromo-2,3,4,5-tetrahydro-benzo[b]oxepine-9-carboxylic acid [1-(6-fluoro-1H-indol-3-ylmethyl)-2-hydroxy-ethyl]-amide and 3-Chloro-4-carbamoyl-phenyl boronic acid* | **26** | **¹H-NMR (DMSO-d₆):** 10.85 s (1H); 8.39 d (*J* = 7.9 Hz, 1H); 7.88 s (1H); 7.83 d (*J* = 2.5 Hz, 1H); 7.71 d (*J* = 1.7 Hz, 1H); 7.69 d (*J* = 2.5 Hz, 1H); 7.63 m (3H); 7.49 d (*J* = 7.9 Hz, 1H); 7.14 d (*J* = 2.1 Hz, 1H); 7.06 dd (*J* = 2.3 Hz /10.2 Hz, 1H); 6.82 m (1H); 4.89 m (1H); 4.17 m (1H); 3.89 m (2H); 3.48 m (1H); 3.43 m (1H); 2.93 m (2H); 2.84 m (2H); 1.88 m (2H); 1.65 m (2H). | 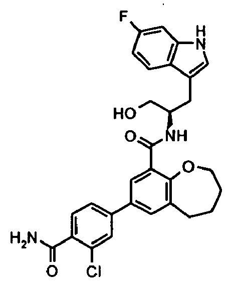 |
| **31** | 7-[3-Chloro-4-(morpholine-4-carbonyl)-phenyl]-2,3,4,5-tetrahydro-benzo[b]oxepine-9-carboxylic acid [(R)-1-(6-fluoro-1H-indol-3-ylmethyl)-2-hydroxy-ethyl]-amide; *7-Bromo-2,3,4,5-tetrahydro-benzo[b]oxepine-9-carboxylic acid [1-(6-fluoro-1H-indol-3-ylmethyl)-2-hydroxy-ethyl]-amide and 3-Chloro-4-(morpholine-4-carbonyl) phenyl boronic acid* | **26** | **¹H-NMR (DMSO-d₆):** 10.85 s (1H); 8.37 d (*J* = 8.1 Hz, 1H); 7.82 d (*J* = 2.3 Hz, 1H); 7.75 d (*J* = 1.5 Hz, 1H); 7.68 d (*J* = 2.5 Hz, 1H); 7.65 m (2H); 7.42 d (*J* = 7.9 Hz, 1H); 7.14 d (*J* = 1.9 Hz, 1H); 7.05 dd (*J* = 2.3 Hz /10.2 Hz, 1H); 6.81 m (1H); 4.88 m (1H); 4.17 m (1H); 3.88 m (2H); 3.63 m (4H); 3.51 m (4H); 3.48 m (2H); 2.93 m (2H); 2.84 m (2H); 1.88 m (2H); 1.66 m (2H). | 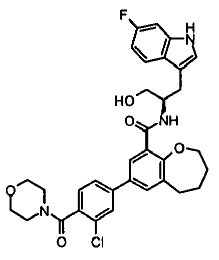 |

### Example 32

### 7-(3-Fluoro-5-methylcarbamoyl-phenyl)-2,3,4,5-tetrahydro-benzo[b]oxepine-9-carboxylic acid [2-(4-fluoro-1H-indol-3-yl)-1-hydroxymethyl-ethyl] - amide

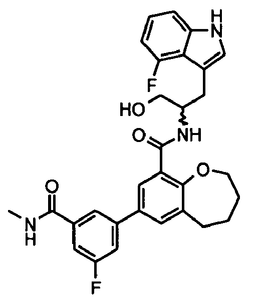

32a) 2-Allyloxy-5-bromo-benzoic acid methyl ester
   The title compound was prepared from 2-Hydroxy-5-bromo-benzoic acid methyl ester in analogy to the described literature procedure via an O-alkylation. See Eur. J. Med. Chem. 1997, 32, page 385.
32b) 3-Allyl-5-bromo-2-hydroxy-benzoic acid methyl ester
   The title compound was prepared via a Claisen rearrangement from 2-Allyloxy-5-bromobenzoic acid methyl ester in analogy to the described literature procedure. See J. Med. Chem. 1992, 35, page 310.
32c) 3-Allyl-2-allyloxy-5-bromo-benzoic acid methyl ester
   The title compound was prepared from -Allyl-5-bromo-2-hydroxy-benzoic acid methyl ester in analogy to the described literature procedure via an O-alkylation. See Eur. J. Med. Chem. 1997, 32, page 385.
32d) 7-Bromo-2,5-dihydro-benzo[b]oxepine-9-carboxylic acid methyl ester
   The title compound was prepared from 3-Allyl-2-allyloxy-5-bromo-benzoic acid methyl ester in analogy to the described literature procedure via an olefin metathesis reaction. See Heterocycles 2002, 57, page 1997.
32e) 7-Bromo-2,3,4,5-tetrahydro-benzo[b]oxepine-9-carboxylic acid methyl ester
   The title compound was prepared from 7-Bromo-2,5-dihydro-benzo[b]oxepine-9-carboxylic acid methyl ester in analogy to the described literature procedure via a hydrogenation reaction. See Org. Lett. 2006, 15, page 3279.
32f) 7-(3-Fluoro-5-methylcarbamoyl-phenyl)-2,3,4,5-tetrahydro-benzo[b]oxepine-9-carboxylic acid methyl ester
   A solution of 7-Bromo-2,3,4,5-tetrahydro-benzo[b]oxepine-9-carboxylic acid methyl ester (2.52 g), 3-Fluoro-5-methylcarbamoyl-phenyl boronic acid (1.82 g), Pd(PPh₃)₄ (306 mg) in ethanol (35 mL), toluene (35 mL) and an aqueous sodium carbonate solution (2M, 8.83 mL) was stirred under reflux for 2 hours. The solvent was evaporated and the solid purified by flash chromatography to yield 97 % of the title compound (3.06 g). ¹H-**NMR (DMSO-d₆):** 8.63 d (J = 4.6 Hz, 1H); 7.93 m (1H); 7.78 d (*J* = 2.5 Hz, 1H); 7.75 d (*J* = 2.5 Hz, 1H); 7.66 m (1H); 7.55 m (1H); 3.97 m (2H); 3.80 s (3H); 2.86 m (2H); 2.78 d (*J* = 4.6 Hz, 3H); 1.89 m (2H); 1.67 m (2H).
32g) 7-(3-Fluoro-5-methylcarbamoyl-phenyl)-2,3,4,5-tetrahydro-benzo[b]oxepine-9-carboxylic acid
   A solution of 7-(3-Fluoro-5-methylcarbamoyl-phenyl)-2,3,4,5-tetrahydro-benzo[b]oxepine-9-carboxylic acid methyl ester (3.05 g) and KOH (2.39 g) in methanol (25 mL) was stirred at ambient temperature overnight. The solvent was distilled off under reduced pressure and the residue was extracted with water / ether. The water phase was acidified by addition of HCl (4N) and the precipitate filtered off. The title compound was obtained in 90% yield (2.62 g). **¹H-NMR (DMSO-d₆):** 12.83 s (1H); 8.63 d (*J* = 4.5 Hz, 1H); 7.93 m (1H); 7.74 m (2H); 7.68 m (1H); 7.55 m (1H); 3.98 m (2H); 2.82 m (2H); 2.78 d (*J* = 4.5 Hz, 3H); 1.87 m (2H); 1.66 m (2H).
32h) 7-(3-Fluoro-5-methylcarbamoyl-phenyl)-2,3,4,5-tetrahydro-benzo[b]oxepine-9-carboxylic acid [2-(4-fluoro-1 H-indol-3-yl)-1-hydroxymethyl-ethyl]-amide
   A solution of 7-(3-Fluoro-5-methylcarbamoyl-phenyl)-2,3,4,5-tetrahydro-benzo[b]oxepine-9-carboxylic acid (100 mg), 2-Amino-3-(4-fluoro-1 H-indol-3-yl)-propan-1-ol (61 mg), EDC (61 mg), HOBt (45 mg) and triethylamine (81 µL) in DMF (4 mL) was stirred at ambient temperature overnight. The solvent was distilled off under reduced pressure. The title compound was obtained after flash chromatography in 68 % yield (106 mg). **¹H-NMR (DMSO-d₆):** 11.10 s (1H); 8.68 d (*J* = 4.5 Hz, 1H); 8.29 d (*J* = 8.5 Hz, 1H); 7.96 s (1H); 7.87 d (*J* = 2.5 Hz, 1H); 7.73 d (*J* = 2.5 Hz, 1H); 7.67 d (*J* = 10.0 Hz, 1H); 7.59 d (*J* = 9.6 Hz, 1H); 7.20 d (*J* = 1.9 Hz, 1H); 7.17 d (*J* = 8.1 Hz, 1H); 7.00 m (2H); 6.72 d (*J* = 7.7 Hz, 1 H); 6.68 d (*J* = 7.7 Hz, 1H); 4.88 m (1H); 4.36 m (1H); 3.90 m (2H); 3.57 m (2H); 3.16 m (1H); 3.04 m (1H); 2.87 m (2H); 2.83 d (*J* = 4.5 Hz, 3H); 1.90 m (2H); 1.70 m (2H).

The following compounds were obtained in analogy to the preparation methods described in detail:

| **Ex.** | **Product; *reagents*** | **Method analogous to** | **¹H-NMR (400 MHz) δ [ppm]** | **Structure** |
|---|---|---|---|---|
| **33** | 7-(3-Fluoro-5-methylcarbamoyl-phenyl)-2,3,4,5-tetra-hydro-benzo[b]oxepine-9-carboxylic acid [2-(5-fluoro-1H-indol-3-yl)-1-hydroxy-methyl-ethyl]-amide; *7-(3-Fluoro-5-methylcarbamoyl-phenyl)-2, 3, 4, 5-tetra-hydro-benzo[b]oxepine-9-carboxylic acid and 2-Amino-3-(5-fluoro-1 H-indol-3-yl)-propan-1-ol* | **32** | **¹H-NMR (DMSO-d₆):** 10.94 s(1H); 8.70 d (*J* = 4.5 Hz, 1H); 8.44 d (*J* = 8.1 Hz, 1H); 7.98 m (2H); 7.77 d (*J* = 2.5 Hz, 1H); 7.67 d (*J* = 7.9 Hz, 1H); 7.59 d (*J* = 9.4 Hz, 1H); 7.47 dd *(J* = 2.5 Hz / 10.2 Hz, 1H); 7.34 m (2H); 7.27 d (*J* = 2.1 Hz, 1H); 6.91 m (1H); 4.95 m (1H); 4.21 m (1H); 3.94 m (2H); 3.53 m (1H); 3.48 m (1H); 2.97 m (2H); 2.84 m (5H); 1.94 m (2H); 1.71 m (2H). | 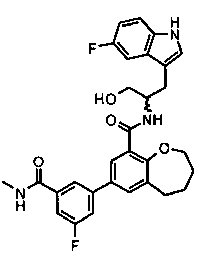 |
| **34** | 7-(3-Fluoro-5-methylcarbamoyl-phenyl)-2,3,4,5-tetrahydro-benzo[b]oxepine-9-carboxylic acid [2-(6-fluoro-1H-indol-3-yl)-1-hydroxy-methyl-ethyl]-amide; *7-(3-Fluoro-5-methylcarbamoyl-phenyl)-2,3,4,5-tetra-hydro-benzo[b]oxepine-9-carboxylic acid and 2-Amino-3-[5-((E)-2-fluoro-propenyl)-4-vinyl-1H-pyrrol-3-yl]-propan-1-ol* | **32** | ¹H-NMR (DMSO-d₆): 10.90 s (1H); 8.70 d (*J* = 4.5 Hz, 1H); 8.44 d (*J* = 8.1 Hz, 1H); 7.98 m (2H); 7.77 d (*J* = 2.5 Hz, 1H); 7.66 m (3H); 7.19 d (*J* = 1.9 Hz, 1H); 7.13 dd (*J* = 2.3 Hz / 10.2 Hz, 1H); 6.86 m (1H); 4.94 m (1H); 4.23 m (1H); 3.93 m (2H); 3.54 m (1H); 3.48 m (1H); 2.99 m (2H); 2.84 m (5H); 1.94 m (2H); 1.71 m (2H). | 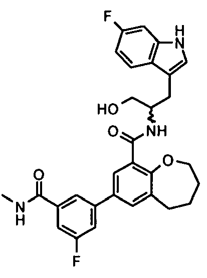 |
| **35** | 7-(3-Fluoro-5-methylcarba-moyl-phenyl)-2,3,4,5-tetra-hydro-benzo[b]oxepine-9-carboxylic acid [(R)-2-hydroxy-1-(1 H-indol-3-yl-methyl)-ethyl]-amide; *7-(3-Fluoro-5-methylcarba-moyl-phenyl)-2,3,4,5-tetra-hydro-benzo[b]oxepine-9-carboxylic acid and* (D)-Tryptophanol | **32** | **¹H-NMR (DMSO-d₆):** 10.82 s (1H); 8.70 d (*J* = 4.5 Hz, 1H); 8.43 d (*J* = 7.9 Hz, 1H); 7.99 d (*J* = 2.1 Hz, 1H); 7.76 d (*J* = 2.5 Hz, 1H); 7.66 m (3H); 7.34 d (*J* = 7.9 Hz, 1H); 7.19 d (*J* = 2.1 Hz, 1H); 7.07 m (1H); 6.99 m (1H); 4.92 m (1H); 4.25 m (1H); 3.92 m (2H); 3.54 m (1H); 3.49 m (1H); 3.01 m (2H); 2.84 m (5H); 1.93 m (2H); 1.70 m (2H). | 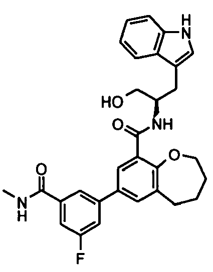 |

### Example 36

### 7-(4-Methylcarbamoyl-phenylethynyl)-2,3,4,5-tetrahydro-benzo[b]oxepine-9-carboxylic acid [(R)-1-hydroxymethyl-2-(1H-indol-3-yl)-ethyl]-amide

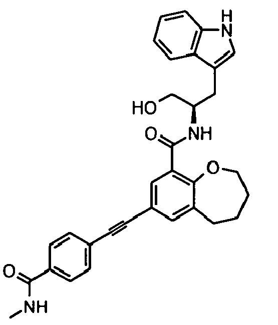

A solution of 7-Bromo-2,3,4,5-tetrahydro-benzo[b]oxepine-9-carboxylic acid [(R)-2-hydroxy-1-(1H-indol-3-ylmethyl)-ethyl]-amide (100 mg), Pd(PPh₃)₂Cl₂ (7 mg), 4-Ethynyl-N-methyl-benzamide (37 mg), TBAF x 3 water (290 mg) in THF (2 mL) and ethanol (0.2 mL) was stirred at 110°C in a sealed microwave reactor for 30 min (200W). The solvents were distilled off under reduced pressure, the crude mixture was taken up in dichlormethane and extracted with water. The solvent of the organic phase was removed and the title compound was obtained in 43% yield (51 mg) after flash chromatography.

**¹H-NMR (DMSO-d₆)**: 10.78 s (1H); 8.49 d (*J* = 4.6 Hz, 1H); 8.31 d (*J* = 8.1 Hz, 1H); 7.84 d (J = 8.3 Hz, 2H); 7.69 d (*J* = 2.3 Hz, 1H); 7.65 d (*J* = 7.8 Hz, 1H); 7.59 d (*J* = 8.3 Hz, 2H); 7.50 d (*J* = 2.0 Hz, 1H); 7.29 d (*J* = 8.0 Hz, 1H); 7.14 d (*J* = 2.3 Hz, 1H); 7.03 m (1H); 6.95 m (1H); 4.87 m (1H); 4.20 m (1H); 3.85 m (2H); 3.48 m (1H); 3.40 m (1H); 2.98 m (1H); 2.90 m (1H); 2.75 d (*J* = 4.6 Hz, 3H); 1.85 m (2H); 1.61 m (1H).

The following compounds were obtained in analogy to the preparation methods described in detail:

| **Ex.** | **Product; *reagents*** | **Method analogous to** | **¹H-NMR (400 MHz) δ [ppm]** | **Structure** |
|---|---|---|---|---|
| **37** | 7-(3-Methylcarbamoyl-phenylethynyl)-2,3,4,5-tetrahydro-benzo[b]oxepine-9-carboxylic acid [(R)-1-hydroxymethyl-2-(1H-indol-3-yl)-ethyl]-amide; *7-Bromo-2,3,4,5-tetrahydro-benzo[b]oxepine-9-carboxylic acid [(R)-2-hydroxy-1-(1H-indol-3-ylmethyl)-ethyl]-amide and 3-Ethynyl-N-methyl-benzamide* | **36** | **¹H-NMR (DMSO-d₆):** 10.79 s (1H); 8.53 d (*J* = 4.5 Hz, 1H); 8.32 d (*J* = 8.1 Hz, 1H); 7.96 s (1H); 7.82 d (*J* = 7.9 Hz, 1H); 7.69 d (*J* = 2.3 Hz, 1H); 7.63 m (2H); 7.50 m (2H); 7.30 d (*J* = 7.9 Hz, 1H); 7.13d (*J*= 1.9 Hz, 1H); 7.03 m (1H); 6.94 m (1H); 4.88 m (1H); 4.20 m (1H); 3.85 m (2H); 3.47 m (2H); 2.95 m (2H); 2.76 m (5H); 1.85 m (2H); 1.62 m (2H). | 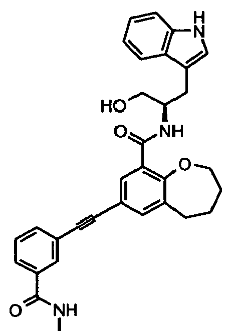 |

### Example 38

### 7-(3-Fluoro-5-methylcarbamoyl-phenyl)-2,3,4,5-tetrahydro-benzo[b]oxepine-9-carboxylic acid [2-(5,6-difluoro-1H-indol-3-yl)-1-hydroxymethyl-ethyl]-amide

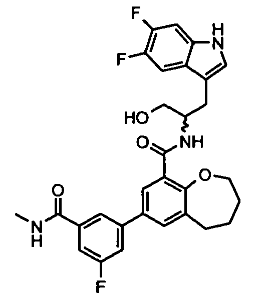

38a) (5,6-Difluoro-1 H-indol-3-yl)-acetaldehyde
   At 0°C, phosphoryl chloride (22.03 g) was slowly added dropwise to DMF (19.1 g), and the mixture was stirred at 0-5°C for half an hour and then at room temperature for one hour. The mixture was cooled again to 0°C, and a solution of 5,6-difluoro-1 H-indole (20 g) in DMF (20 g) was slowly added dropwise. The mixture was stirred at 0°C for 30 minutes and then at room temperature for a further 15 hours. The reaction mixture was poured onto ice (200 g) and basified to pH 10 with NaOH. The crystalline title compound was filtered off, washed with water and dried in vacuo (yield 22.7 g, 96%). **MS (ESI,+)**: 196 (M+1).
38b) [2-(5,6-Difluoro-1H-indol-3-yl)ethyl]diethylamine
   Sodium triacetoxyborohydride (26.3 g) was added in portions to a solution of (5,6-difluoro-1 H-indol-3-yl)acetaldehyde (15 g) and diethylamine (6.66 g) in absolute dichloromethane (300 ml) with 2 drops of trifluoroacetic acid, and the mixture was stirred at room temperature for 24 hours. The solvent was distilled off in a rotary evaporator, and the residue was mixed with 10% strength aqueous sodium bicarbonate solution and extracted with ethyl acetate. The combined organic phases were dried over sodium sulphate and concentrated in a rotary evaporator. The crude product was purified by flash chromatography, and the title compound was obtained in 68% yield (13.5 g). **MS (ESI,+)**: 253 (M+1).
38c) Ethyl 3-(5,6-difluoro-1 H-indol-3-yl)-2-nitropropionate
   A mixture of gramine (8 g) and ethyl 2-nitroacetate (8.9 g) was stirred in absolute toluene at 90-100°C for 4 hours. The reaction mixture was concentrated in a rotary evaporator, and the crude product was purified by flash chromatography (chloroform : methanol 19 :1), after which the title compound was obtained in a yield of 11.7 g as a 1:2 mixture with ethyl 2-nitroacetate. **MS (ESI,+):** 299 (M+1).
38d) Ethyl 2-amino-3-(5,6-difluoro-1H-indol-3-yl)propionate
   The mixture from the above stage was stirred with ammonium formate (9.9 g) and Pd (4.1 g, 10% on activated carbon) in 300 ml of ethanol under reflux for 15 hours. The reaction mixture was concentrated in a rotary evaporator, diluted with water (100 ml) and extracted with ethyl acetate. The combined organic phases were dried over sodium sulphate and concentrated in a rotary evaporator. The residue was purified by flash chromatography (silica, chloroform : methanol 19 :1) and recrystallized as HCl salt from ethanol. The title compound was obtained in a yield of 2.7 g. **MS (ESI,+)**: 269 (M+1).
38e) 3-(5,6-Difluoro-1 H-indol-3-yl)-2-{[7-(3-fluoro-5-methylcarbamoyl-phenyl)-2,3,4,5-tetrahydro-benzo[b]oxepine-9-carbonyl]-amino]-propionic acid ethyl ester
   A solution of 7-(3-Fluoro-5-methylcarbamoyl-phenyl)-2,3,4,5-tetrahydro-benzo[b]oxepine-9-carboxylic acid (100 mg), Ethyl 2-amino-3-(5,6-difluoro-1 H-indol-3-yl)propionate (86 mg), EDC (61 mg), HOBt (45 mg) and triethylamine (81 µL) in DMF (4 mL) was stirred at ambient temperature overnight. The solvent was distilled off under reduced pressure. The title compound was obtained after flash chromatography in 78 % yield (134 mg). **¹H-NMR (DMSO-d₆):** 11.55 s (1H); 8.72 d *(J* = 7.3 Hz, 1H); 8.65 d *(J* = 4.6 Hz, 1H); 7.92 d (*J* = 2.3 Hz, 2H); 7.74 s (1H); 7.60 m (1 H); 7.75 m (1H); 7.30 d (J = 2.0 Hz, 1H); 7.12 dd (*J* = 2.3 Hz / 9.6 Hz, 1H); 6.90 m(1H); 4.78 m(1H); 4.06 m (2H); 3.70 m (2H); 3.26 m (2H); 2.85 m (2H); 2.78 d (*J* = 4.6 Hz, 3H); 1.83 m (2H); 1.70 m (2H); 1.12 m (3H).
38f) 7-(3-Fluoro-5-methylcarbamoyl-phenyl)-2,3,4,5-tetrahydro-benzo[b]oxepine-9-carboxylic acid [2-(5,6-difluoro-1H-indol-3-yl)-1-hydroxymethyl-ethyl]-amide
   A solution of 3-(5,6-Difluoro-1H-indol-3-yl)-2-{[7-(3-fluoro-5-methylcarbamoyl-phenyl)-2,3,4,5-tetrahydro-benzo[b]oxepine-9-carbonyl]-amino}-propionic acid ethyl ester (129 mg) was dissolved in THF (10 mL) and a solution of lithium borohydride (2M in THF, 326 µL) and methanol (48 µL) was added at ambient temperature.

The reaction was stirred overnight, then quenched with methanol and water. The solvent was distilled off under reduced pressure and the title compound was obtained after flash chromatography in 66 % yield (79 mg). ¹H-NMR (DMSO-d₆): 11.00 s (1H); 8.71 d (*J* = 4.3 Hz, 1H); 8.45 d (*J* = 8.1 Hz, 1H); 7.98 m (1H); 7.96 d (*J* = 2.5 Hz, 1H); 7.76 d (*J* = 2.5 Hz, 1H); 7.68 m (2H); 7.59 m (1H); 7.34 m (1H); 7.26 m (1H); 4.96 m (1H); 4.19 m (1H); 3.95 m (2H); 3.53 m (1H); 3.48 m (1H); 2.97 m (2H); 2.89 m (2H); 2.83 d (*J* = 4.3 Hz, 3H); 1.94 m (2H); 1.72 m (2H).

### Example 39

### 7-(3-Fluoro-5-methylcarbamoyl-phenyl)-2,3,4,5-tetrahydro-benzo[b]oxepine-9-carboxylic acid [2-(5,7-diftuoro-1H-indol-3-yl)-1-hydroxymethyl-ethyl]-amide

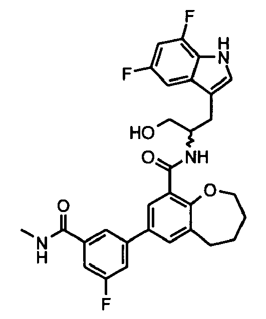

39a) 2-Acetylamino-2-{3-[(2,4-difluoro-phenyl)-hydrazono]-propyl}-malonic acid diethyl ester
   The solution of diethyl (acetylamino)malonate (20 mmol) in toluene (30 ml) stirred at 0°C was treated under Ar with NaOMe (0.057 g, 1.1 mmol) in MeOH and freshly distilled acrylaldehyde (1.5 ml) in toluene was added dropwise in 30 minutes. The solution was stirred at 0°C. After 1 hr acetic acid (0.2 ml) and (2,4-Difluoro-phenyl)-hydrazine (20 mmol) were added. The reaction mixture was refluxed for 1.5 hr, cooled and left in the freezer (0°C) overnight. Toluene was evaporated under reduced pressure and hexane was added to give a solid residue. This was filtered off and washed with hexane to give the title compound (75-80%). This compound was immediately used in the next step without further purification.
39b) 2-Acetylamino-2-(5,7-difluoro-1H-indol-3-ylmethyl)-malonic acid diethyl ester
   The solution of 2-Acetylamino-2-{3-[(2,4-difluoro-phenyl)-hydrazono]-propyl}-malonic acid diethyl ester (0.42 mol) in 5% H₂SO₄ (750 ml) was refluxed for 6 hr under Ar.
   After cooling at rt the oil formed was washed with water at 60°C and residue was dissolved in CH₂Cl₂, washed with water and brine, the organic phase was dried (Na₂SO₄) and concentrated to give a gummy residue. This was chromatographed on silica gel (toluene) to give the title compound (65%) as brown solid.
39c) 2-Acetylamino-2-(5,7-difluoro-1 H-indol-3-ylmethyl)-malonic acid
   The solution of 2-Acetylamino-2-(5,7-difluoro-1 H-indol-3-ylmethyl)-malonic acid diethyl ester (0.1 mol) in water (190 ml) and NaOH (20 g) was heated at 90°C for 4 hr under Ar. After cooling to 0°C HCl (50 ml) was added portionwise at stirring. The reaction mixture was left in the freezer (0°C) overnight. The obtained solid was filtered and oven dried at 45°C at reduced pressure yielding the title compound (~50%).
39d) 2-Acetylamino-3-(5,7-difluoro-1 H-indol-3-yl)-propionic acid
   The solution of 2-Acetylamino-2-(5,7-difluoro-1 H-indol-3-ylmethyl)-malonic acid (3.2 g) in water (15 ml) was refluxed for 3 hr under Ar. The black oil formed was extracted with hot water (3x50 ml). The water extracts were combined and treated with activated charcoal. After cooling at rt the reaction mixture was left in the freezer (0°C) overnight. The solid formed was filtered off, washed with water and dried yielding 1.1 g of the title compound. MS (ESI,+): 283 (M+1).
39e) 2-Amino-3-(5,7-difluoro-1 H-indol-3-yl)-propionic acid
   The solution of 2-Acetylamino-3-(5,7-difluoro-1 H-indol-3-yl)-propionic acid (2.8 g) in 2N HCl (25 ml) was refluxed for 4 hr under Ar. After cooling at rt the solvent was removed under reduced pressure at 50°C. The residue (-2.7 g) was dissolved in water (6 ml) and sodium acetate (0.85 g) was added at 50°C. After cooling at rt the reaction mixture was left in the freezer (0°C) overnight. The solid formed was filtered off, washed with cold water (2 ml) and dried yielding after recrystallization from abs. EtOH 2.0 g of the title compound. MS (ESI,+): 241 (M+1).
39f) 2-Amino-3-(5,7-difluoro-1H-indol-3-yl)-propionic acid ethyl ester 2-Amino-3-(5,7-difluoro-1H-indol-3-yl)-propionic acid (3.0 g) was added portionwise at - 10°C to the stirred solution of SOCl₂ (2.2 ml) in abs. EtOH (30 ml) uder Ar. The reaction mixture was stirred for 2 hr at -10°C and 48 hr at rt. The solid formed was filtered off, washed with ethyl ether and dried yielding 2.5 g of the title compound as hydrochloride. MS (ESI,+): 269 (M+1).
39g) 3-(5,7-Difluoro-1H-indol-3-yl)-2-{[7-(3-fluoro-5-methylcarbamoyl-phenyl)-2,3,4,5-tetrahydro-benzo[b]oxepine-9-carbonyl]-amino}-propionic acid ethyl ester
   A solution of 7-(3-Fluoro-5-methy[carbamoyl-phenyl)-2,3,4,5-tetrahydro-benzo[b]oxepine-9-carboxylic acid (100 mg), Ethyl 2-Amino-3-(5,7-difluoro-1 H-indol-3-yl)-propionic acid ethyl ester (86 mg), EDC (61 mg), HOBt (45 mg) and triethylamine (81 µL) in DMF (4 mL) was stirred at ambient temperature overnight. The solvent was distilled off under reduced pressure. The title compound was obtained after flash chromatography in 78 % yield (134 mg). **¹H-NMR (DMSO-d₆):** 11.55 s (1H); 8.73 d (*J* = 7.3 Hz, 1H); 8.65 d (*J* = 4.6 Hz, 1H); 7.92 s (1H); 7.89 d (*J* = 2.5 Hz, 1H); 7.74 d (*J* = 2.3 Hz, 1H); 7.60 m (1H); 7.57 m (1H); 7.30 d (*J* = 2.0 Hz, 1H); 7.12 dd (*J* = 9.6 Hz / 2.0 Hz, 1H); 6.90 m (1H); 4.78 m (1H); 4.06 m (2H); 3.70 m (2H); 3.26 m (2H); 2.85 m (2H); 2.79 d (J = 4.6 Hz, 3H); 1.83 m (2H); 1.69 m (2H); 1.12 m (3H).
39h) 7-(3-Fluoro-5-methylcarbamoyl-phenyl)-2,3,4,5-tetrahydro-benzo[b]oxepine-9-carboxylic acid [2-(5,7-difluoro-1H-indol-3-yl)-1-hydroxymethyl-ethyl]-amide
   A solution of 3-(5,7-Difluoro-1H-indol-3-yl)-2-{[7-(3-fluoro-5-methylcarbamoyl-phenyl)-2,3,4,5-tetrahydro-benzo[b]oxepine-9-carbonyl]-amino}-propionic acid ethyl ester (124 mg) was dissolved in THF (10 mL) and a solution of lithium borohydride (2M in THF, 313 µL) and methanol (47 µL) was added at ambient temperature. The reaction was stirred overnight, then quenched with methanol and water. The solvent was distilled off under reduced pressure and the title compound was obtained after flash chromatography in 72 % yield (83 mg). ¹H-NMR (DMSO-d₆): 11.46 s (1H); 8.69 d (*J* = 4.5 Hz, 1H); 8.44 d (J = 8.1 Hz, 1H); 7.98 s (1H); 7.94 d (*J* = 2.5 Hz, 1H); 7.76 d (*J* = 2.5 Hz, 1H); 7.68 m (1H); 7.61 m (1H); 7.35 dd (*J* = 9.8 Hz / 2.1 Hz, 1H); 7.34 d (*J* = 2.1 Hz, 1H); 6.93 m (1H); 4.96 m (1H); 4.20 m (1H); 3.95 m (2H); 3.53 m (2H); 2.94 m (2H); 2.84 d (*J* = 4.5 Hz, 3H); 1.94 m (2H); 1.72 m (2H).

### Example 40

### 7-((E)-Styryl)-2,3,4,5-tetrahydro-benzo[b]oxepine-9-carboxylic acid [(R)-1-hydroxymethyl-2-(1H-indol-3-yl)-ethyl]-amide

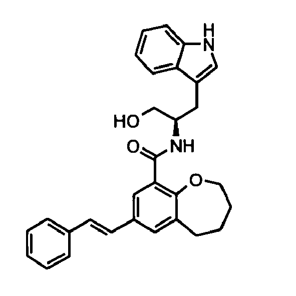

A solution of 7-Bromo-2,3,4,5-tetrahydro-benzo[b]oxepine-9-carboxylic acid [(R)-2-hydroxy-1-(1 H-indol-3-ylmethyl)-ethyl]-amide (100 mg), (E)-2-Phenyl-ethene boronic acid (35 mg), Pd(PPh₃)₄ (8.3 mg) in ethanol (1 mL), toluene (1 mL) and an aqueous sodium carbonate solution (2M, 0.23 mL) was stirred in a sealed microwave reactor at 100°C for 30 minutes. The solvent was evaporated and the solid purified by flash chromatography to yield 69 % of the title compound (73 mg). **¹H-NMR (DMSO-d₆):** 10.79 s (1H); 8.36 d (*J* = 7.8 Hz, 1H); 7.78 d (*J* = 2.5 Hz, 1H); 7.67 d (*J* = 7.8 Hz, 1H); 7.56 m (3H); 7.34 m (3H); 7.23 m (1H); 7.18 m (3H); 7.03 m (1H); 6.95 m (1H); 4.88 m (1H); 4.21 m (1H); 3.83 m (2H); 3.49 m (1H); 3.43 m (1H); 2.98 m (2H); 2.77 m (2H); 1.85 m (2H); 1.63 m (2H).

## Claims

1. Compounds of the formula I 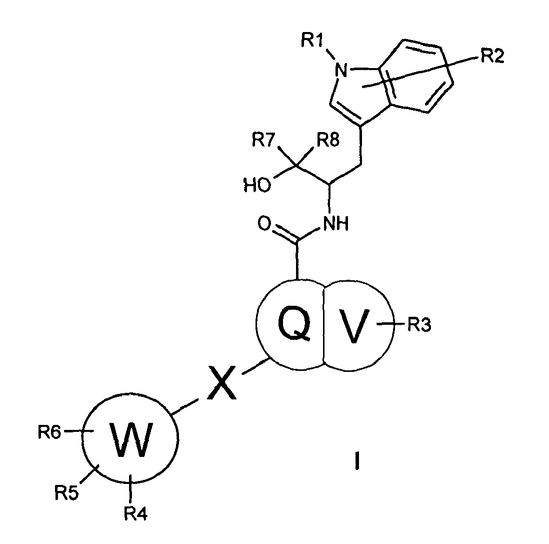 in which
R1 is hydrogen, C₁-C₆-alkyl, C₃-C₆-alkenyl, C₃-C₆-alkynyl, C₃-C₇-cycloalkyl, C₁-C₆-alkyloxy-C₁-C₆-alkylene, C₃-C₇-cycloalkyloxy-C₁-C₆-alkylene, C₁-C₆-alkylamino-C₁-C₆-alkylene, di(C₁-C₆-alkyl)amino-C₁-C₆-alkylene, phenyloxy-C₁-C₆-alkylene;
wherein the hydrocarbon chains may optionally be substituted once or more times by fluorine, cyano, hydroxy, amino or the groups: 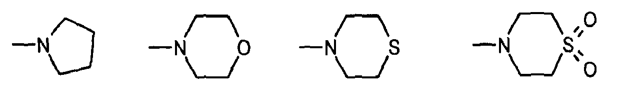 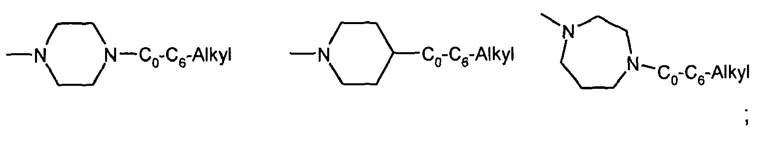
R2 is hydrogen, halogen, cyano, -SO₂Me, C₁-C₆-alkyl, C₂-C₆-alkenyl, C₂-C₆-alkynyl, C₁-C₆-alkyloxy or benzyloxy,
wherein the hydrocarbon chains may optionally be fluorinated once or more times;
R3 is hydrogen, hydroxy, halogen, nitro, amino, cyano, C₁-C₆-alkyl, C₂-C₆-alkenyl or C₂-C₆-alkynyl, C₃-C₇-cycloalkyl, hydroxy-C₁-C₆-alkylene, hydroxy-C₃-C₆-alkenylene, hydroxy-C₃-C₆-alkynylene, C₁-C₆-alkyloxy, C₁-C₆-alkyloxy-C₁-C₆-alkylene, C₃-C₇-cycloalkyloxy, C₃-C₇-cycloalkyl-C₁-C₆-alkylenoxy, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, C₁-C₆-alkylamino-C₁-C₆-alkylene, di(C₁-C₆-alkyl)amino-C₁-C₆-alkylene, phenyloxy-C₁-C₆-alkylene;
where the hydrocarbon chains therein may optionally be substituted one or more times by fluorine, cyano, hydroxy, amino or the groups 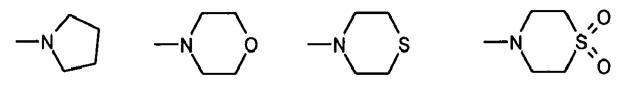 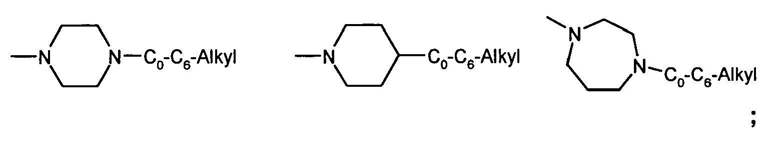
R4, R5, R6 are independently of one another hydrogen, hydroxy, halogen, nitro, amino, cyano, phenyl, C₁-C₆-alkyl, C₂-C₆-alkenyl or C₂-C₆-alkynyl, C₃-C₇-cycloalkyl, C₃-C₇-cycloalkyl-C₁-C₆-alkylene, C₃-C₇-heterocycloalkyl, where the hydrocarbon chains therein may optionally be substituted one or more times by fluorine, cyano or the groups: 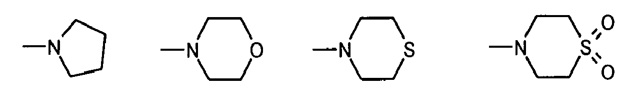 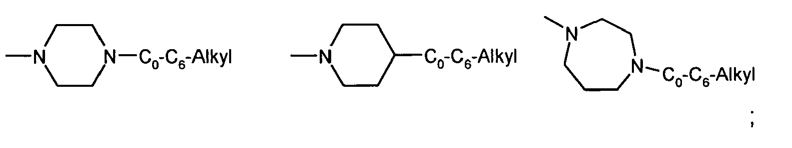 or
independently of one another hydroxy-C₁-C₆-alkylene, hydroxy-C₃-C₆-alkenylene, hydroxy-C₃-C₆-alkynylene, C₁-C₆-alkyloxy, C₃-C₇-cycloalkyl-oxy, C₃-C₇-cycloalkyl-C₁-C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, phenyloxy-C₁-C₆-alkylene,
C₁-C₆-alkylamino, di(C₁-C₆-alkyl)amino, C₁-C₆-alkylamino-C₁-C₆-alkylene, di(C₁-C₆)-alkylamino-C₁-C₆-alkylene, C₃-C₇-cycloalkyl-(C₀-C₆-alkyl)amino,
C₁-C₆-acyl-(C₀-C₆-alkyl)amido, C₁-C₆-alkylaminocarbonyl, di(C₁-C₆-alkyl)aminocarbonyl, (C₃-C₇-cycloalkyl)aminocarbonyl, di(C₃-C₇-cyclo-alkyl)aminocarbonyl, C₃-C₇-cycloalkyl-C₁-C₆-alkyleneaminocarbonyl, C₁-C₆-alkylcarbonyl, C₃-C₇-cycloalkylcarbonyl,
carboxy, carboxamido [-C(O)NH₂], C₁-C₆-alkyloxycarbonyl, C₁-C₃-alkylsulphanyl, C₁-C₆-alkysulphonyl, C₃-C₇-cycloalkylsulphonyl, C₃-C₇-cycloalkyl-C₁-C₆-alkylenesulphonyl,
C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido, -N(C₀-C₆-alkyl)-C(O)-C₁-C₆-alkyl, -N(C₀-C₆-alkyl)-C(O)-C₃-C₇-cycloalkyl, -N(C₀-C₆-alkyl)-C(O)-N-di(C₀-C₆-alkyl), -N(C₀-C₆-alkyl)-C(O)-O-(C₀-C₆)alkyl, -N(C₀-C₆-alkyl)-C(O)-NH-C₃-C₇-cycloalkyl,
-N(C₀-C₆-alkyl)-SO₂-C₁-C₆-alkyl, -N(C₀-C₆-alkyl)-SO₂C₃-C₇-cycloalkyl, -N(C₀-C₆-alkyl)-SO₂-N-di(C₀-C₆-alkyl), -N(C₀-C₆-alkyl)-SO₂-NH-(C₃-C₇)-cycloalkyl,
-C(O)-N(H)-C₂-C₆-alkylene-(C₁-C₆-alkyl)amine, -C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycloalkyl-C₁-C₆-alkyl)amine,
-S(O₂)-N(H)-C₂-C₆-alkylene-(C₁-C₆-alkyl)amine, -S(O₂)-N(H)-C₂-C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine,
-O-C₂-C₆-alkylene-(C₁-C₆-alkyl)amine, -O-C₂-C₆-alkylene-[di(C₁-C₆-alkylene)]amine,
or the groups: 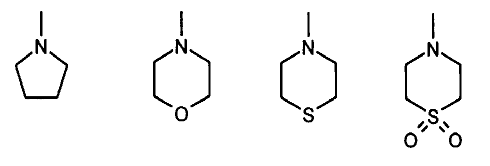 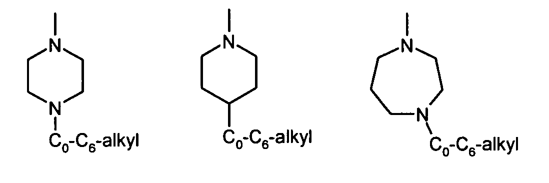 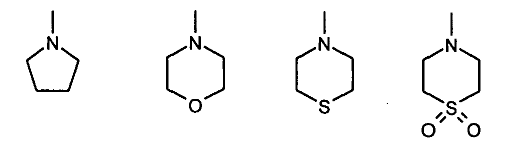 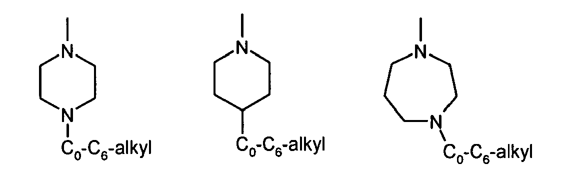 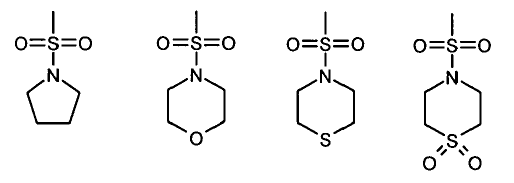 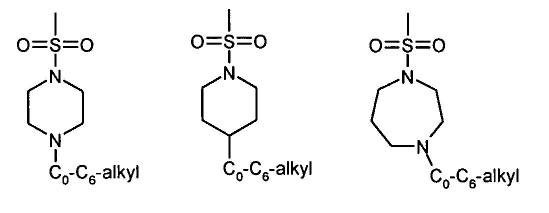 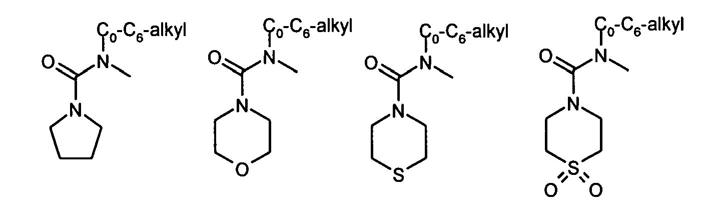 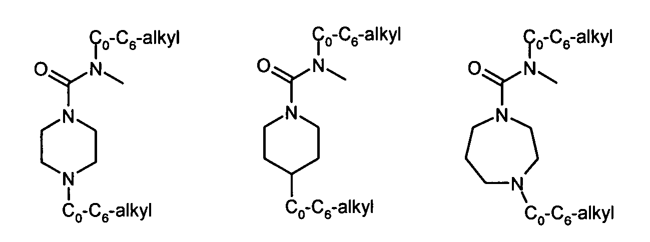 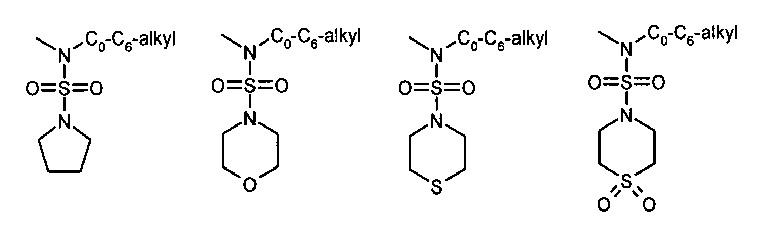 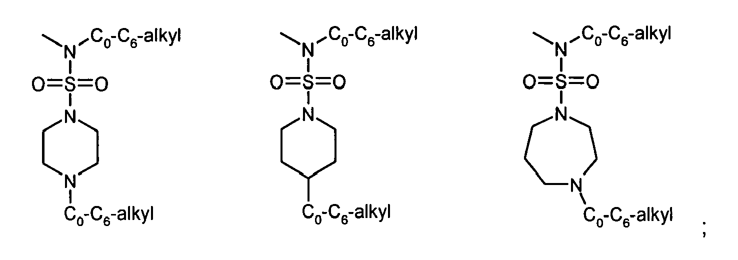
R7, R8 may be independently of one another hydrogen, methyl, ethyl, where the methyl and ethyl radicals may be fluorinated one or more times;
wherein
R2 may substitute one or more positions of the aryl or heteroaryl ring in the indole residue;
R3 may substitute one or more positions of the monocyclic aryl or mono-cyclic heteroaryl ring of Q and/or V;
R5 and R6 may together form a heterocycloalkyl or cycloalkyl group;
Q is a monocyclic aryl or a monocyclic heteroaryl group;
V is a cycloalkylen, cycloalkenylen, heterocycloalkylen or heterocycloalkenylen group;
W is an aryl or heteroaryl group;
X is a bond, C₁-C₄-alkylene, C₂-C₄-alkenylene or C₂-C₄-alkynylene group.

2. Compounds according to claim 1, namely acyltryptophanols of the formula II 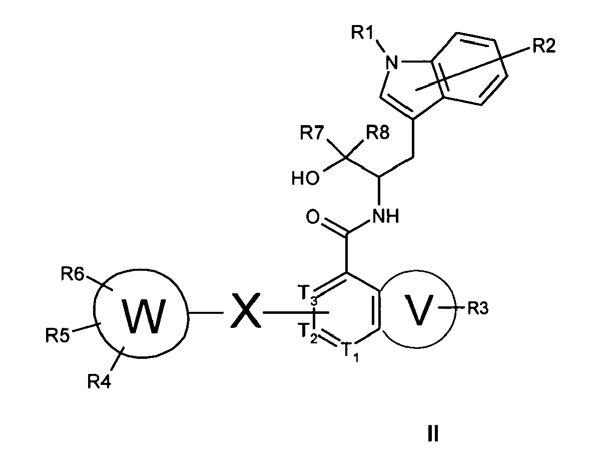 in which the radicals R1 to R8, X, V and W have the same meaning as defined in claim 1
and
T1, T2, T3 are each independently of one another a nitrogen atom or an R3-C group.

3. Compounds according to claim 1, namely acyltryptophanols of the formula III 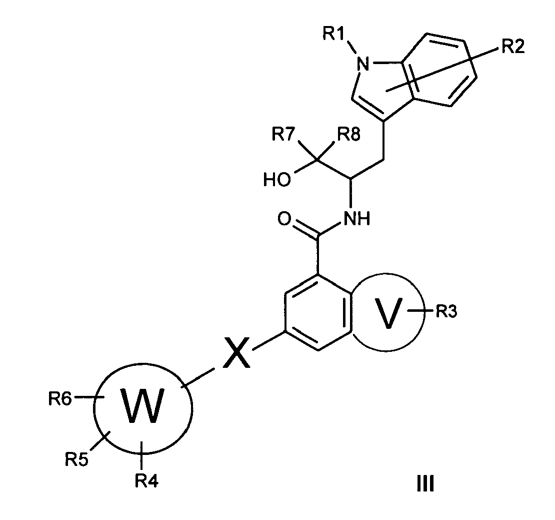 in which R1 to R8, X, V and W have the same meaning as defined in claim 1.

4. Compounds according to any of the preceding claims, namely
**1** 6-(3,4,5-Trimethoxy-phenyl)-2H-chromene-8-carboxylic acid [(R)-1-hydroxy-methyl-2-(1 H-indol-3-yl)-ethyl]-amide;
**2** 6-(3-Chloro-4-methylcarbamoyl-phenyl)-2H-chromene-8-carboxylic acid [(R)-2-hydroxy-1-(1 H-indol-3-ylmethyl)-ethyl]-amide;
**3** 6-(3-Chloro-4-methylcarbamoyl-phenyl)-chroman-8-carboxylic acid [(R)-2-hydroxy-1-(1 H-indol-3-ylmethyl)-ethyl]-amide;
**4** 6-o-Tolylethynyl-2H-chromene-8-carboxylic acid [(R)-1-hydroxymethyl-2-(1H-indol-3-yl)-ethyl]-amide;
**5** 6-(2-Methoxy-phenylethynyl)-2H-chromene-8-carboxylic acid [(R)-1-hydroxy-methyl-2-(1 H-indol-3-yl)-ethyl]-amide;
**6** 2-Methyl-6-(3,4,5-trimethoxy-phenyl)-2H-chromene-8-carboxylic acid [(R)-1-hydroxymethyl-2-(1H-indol-3-yl)-ethyl]-amide;
**7** 6-(3-Chloro-4-methylcarbamoyl-phenyl)-2,2-dimethyl-2H-chromene-8-carboxylic acid [(R)-2-hydroxy-1 -(1 H-indol-3-ylmethyl)-ethyl]-amide;
**8** 6-(3-Fluoro-5-methylcarbamoyl-phenyl)-2,2-dimethyl-2H-chromene-8-carboxylic acid [(R)-2-hydroxy-1-(1H-indol-3-ylmethyl)-ethyl]-amide;
**9** 2,2-Dimethyl-6-(3,4,5-trimethoxy-phenyl)-2H-chromene-8-carboxylic acid [(R)-2-hydroxy-1-(1 H-indol-3-ylmethyl)-ethyl]-amide;
**10** 2,2-Dimethyl-6-(4-methylcarbamoyl-phenylethynyl)-2H-chromene-8-carboxylic acid [(R)-1-hydroxymethyl-2-(1H-indol-3-yl)-ethyl]-amide;
**11** 2,2-Dimethyl-6-(3-methylcarbamoyl-phenylethynyl)-2H-chromene-8-carboxylic acid [(R)-1-hydroxymethyl-2-(1 H-indol-3-yl)-ethyl]-amide;
**12** 5-(3,4-Dimethoxy-phenyl)-2-vinyl-2,3-dihydro-benzofuran-7-carboxylic acid [(R)-1-hydroxymethyl-2-(1H-indol-3-yl)-ethyl]-amide;
**13** 5-(3,4-Dimethoxy-phenyl)-2-ethyl-2,3-dihydro-benzofuran-7-carboxylic acid [(R)-1-hydroxymethyl-2-(1H-indol-3-yl)-ethyl]-amide;
**14** 7-(3-Chloro-4-methylcarbamoyl-phenyl)-2,3-dihydro-benzo[1,4]dioxine-5-carboxylic acid [(R)-2-hydroxy-1-(1H-indol-3-ylmethyl)-ethyl]-amide;
**15** 7-(3,4,5-Trimethoxy-phenyl)-2,3-dihydro-benzo[1,4]dioxine-5-carboxylic acid [(R)-1-hydroxymethyl-2-(1H-indol-3-yl)-ethyl]-amide;
**16** 7-(3-Fluoro-5-methylcarbamoyl-phenyl)-2,3-dihydro-benzo[1,4]dioxine-5-carboxylic acid [(R)-2-hydroxy-1-(1H-indol-3-ylmethyl)-ethyl]-amide;
**17** 7-(3-Fluoro-5-methylcarbamoyl-phenyl)-2,3-dihydro-benzo[1,4]dioxine-5-carboxylic acid [2-(6-fluoro-1 H-indol-3-yl)-1-hydroxymethyl-ethyl]-amide;
**18** 7-(3,4,5-Trimethoxy-phenyl)-2,3-dihydro-benzo-[1,4]dioxine-5-carboxylic acid [2-(6-fluoro-1 H-indol-3-yl)-1-hydroxymethyl-ethyl]-amide;
**19** 7-(3-Chloro-4-methylcarbamoyl-phenyl)-2,3-dihydro-benzo[1,4]dioxine-5-carboxylic acid [2-(6-fluoro-1 H-indol-3-yl)-1-hydroxymethyl-ethyl]-amide;
**20** 5-(3,4-Difluoro-5-methoxy-phenyl)-2,3-dihydro-benzofuran-7-carboxylic acid [(R)-2-hydroxy-1-(1 H-indol-3-ylmethyl)-ethyl]-amide;
**21** 5-(3,4-Dimethoxy-phenyl)-2,3-dihydro-benzofuran-7-carboxylic acid [(R)-1-hydroxymethyl-2-(1H-indol-3-yl)-ethyl]-amide;
**22** 5-(3-Methoxy-phenyl)-2,3-dihydro-benzofuran-7-carboxylic acid [(R)-1-hydroxy-methyl-2-(1 H-indol-3-yl)-ethyl]-amide;
**23** 5-Benzo[1,3]dioxol-5-yl-2,3-dihydro-benzofuran-7-carboxylic acid [(R)-1-hydroxymethyl-2-(1 H-indol-3-yl)-ethyl]-amide;
**24** 5-(3-Fluoro-4-methoxy-phenyl)-2,3-dihydro-benzofuran-7-carboxylic acid [(R)-2-hydroxy-1-(1 H-indol-3-ylmethyl)-ethyl]-amide;
**25** 5-(3,4,5-Trimethoxy-phenyl)-2,3-dihydro-benzofuran-7-carboxylic acid [(R)-1-hydroxymethyl-2-(1H-indol-3-yl)-ethyl]-amide;
**26** 7-(3-Chloro-4-methylcarbamoyl-phenyl)-2,3,4,5-tetrahydro-benzo[b]oxepine-9-carboxylic acid [(R)-2-hydroxy-1-(1 H-indol-3-ylmethyl)- ethyl]-amide;
**27** 7-(4-Carbamoyl-3-chloro-phenyl)-2,3,4,5-tetrahydro-benzo[b]oxepine-9-carboxylic acid [(R)-2-hydroxy-1-(1H-indol-3-ylmethyl)-ethyl]-amide;
**28** 7-[3-Chloro-4-(morpholine-4-carbonyl)-phenyl]-2,3,4,5-tetrahydro-benzo[b]-oxepine-9-carboxylic acid [(R)-2-hydroxy-1-(1H-indol-3-ylmethyl)-ethyl]-amide;
**29** 7-(3-Chloro-4-methylcarbamoyl-phenyl)-2,3,4,5-tetrahydro-benzo[b]oxepine-9-carboxylic acid [1-(6-fluoro-1 H-indol-3-ylmethyl)-2-hydroxy-ethyl]-amide;
**30** 7-(4-Carbamoyl-3-chloro-phenyl)-2,3,4,5-tetrahydro-benzo[b]oxepine-9-carboxylic acid [1-(6-fluoro-1H-indol-3-ylmethyl)-2-hydroxy-ethyl]-amide;
**31** 7-[3-Chloro-4-(morpholine-4-carbonyl)-phenyl]-2,3,4,5-tetrahydro-benzo[b]-oxepine-9-carboxylic acid [(R)-1-(6-fluoro-1H-indol-3-ylmethyl)-2-hydroxy-ethyl]-amide;
**32** 7-(3-Fluoro-5-methylcarbamoyl-phenyl)-2,3,4,5-tetrahydro-benzo[b]oxepine-9-carboxylic acid [2-(4-fluoro-1H-indol-3-yl)-1-hydroxymethyl-ethyl]-amide;
**33** 7-(3-Fluoro-5-methylcarbamoyl-phenyl)-2,3,4,5-tetrahydro-benzo[b]oxepine-9-carboxylic acid [2-(5-fluoro-1 H-indol-3-yl)-1-hydroxymethyl-ethyl]-amide;
**34** 7-(3-Fluoro-5-methylcarbamoyl-phenyl)-2,3,4,5-tetrahydro-benzo[b]oxepine-9-carboxylic acid [2-(6-fluoro-1 H-indol-3-yl)-1-hydroxymethyl-ethyl]-amide;
**35** 7-(3-Fluoro-5-methylcarbamoyl-phenyl)-2,3,4,5-tetrahydro-benzo[b]oxepine-9-carboxylic acid [(R)-2-hydroxy-1-(1 H-indol-3-ylmethyl)-ethyl]-amide;
**36** 7-(4-Methylcarbamoyl-phenylethynyl)-2,3,4,5-tetrahydro-benzo[b]oxepine-9-carboxylic acid [(R)-1-hydroxymethyl-2-(1 H-indol-3-yl)-ethyl]-amide;
**37** 7-(3-Methylcarbamoyl-phenylethynyl)-2,3,4,5-tetrahydro-benzo[b]oxepine-9-carboxylic acid [(R)-1-hydroxymethyl-2-(1H-indol-3-yl)-ethyl]-amide;
**38** 7-(3-Fluoro-5-methylcarbamoyl-phenyl)-2,3,4,5-tetrahydro-benzo[b]oxepine-9-carboxylic acid [2-(5,6-difluoro-1 H-indol-3-yl)-1-hydroxymethyl-ethyl]-amide;
**39** 7-(3-Fluoro-5-methylcarbamoyl-phenyl)-2,3,4,5-tetrahydro-benzo[b]oxepine-9-carboxylic acid [2-(5,7-difluoro-1 H-indol-3-yl)-1-hydroxymethyl-ethyl]-amide;
**40** 7-((E)-Styryl)-2,3,4,5-tetrahydro-benzo[b]oxepine-9-carboxylic acid [(R)-1-hydroxymethyl-2-(1 H-indol-3-yl)-ethyl]-amide;

5. Process for preparing compounds of the formula I of claim 1, wherein a tryptophanol derivative of the formula IV 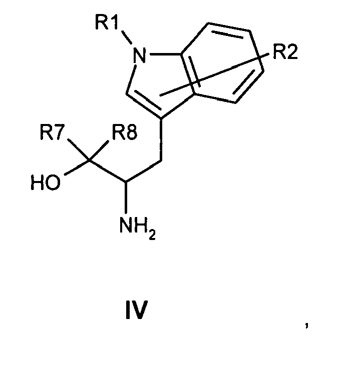 in which the radicals R1, R2, R7 and R8 have the same meaning as defined in claim 1,
is coupled with a carboxylic acid of the formula V 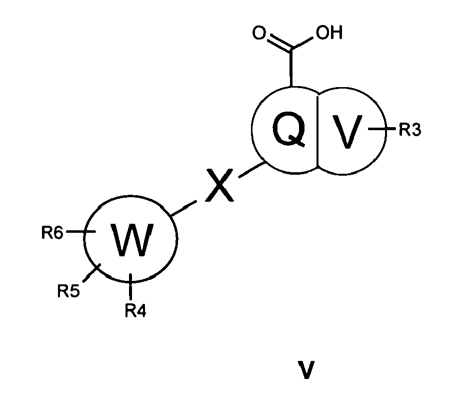 in which R3, R4, R5, R6, Q, X, V and W have the same meaning as defined in claim 1,
in an amide forming reaction comprising
a) conversion of said carboxylic acids into an intermediate active ester or carbonyl chloride with a suitable peptide-coupling reagent, or with thionyl chloride, oxalyl chloride, phosgene or derivatives thereof, where appropriate in the presence of a base,
b) reacting the active intermediate resulting from step a) with said tryptophanol.

6. Process according to claim 5 for preparing compounds of the formula II of claim 2, wherein a tryptophanol derivative of the formula IV is coupled with a carboxylic acid of the formula VI 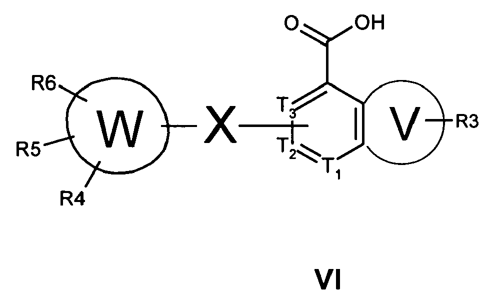 in which R3, R4, R5, R6, T₁, T₂, T₃, X, V and W have the same meaning as defined in claim 2.

7. Process according to claim 5 for preparing compounds of the formulae III of claim 3, wherein a tryptophanol derivative of the formula IV is coupled with a carboxylic acid of the formula VII 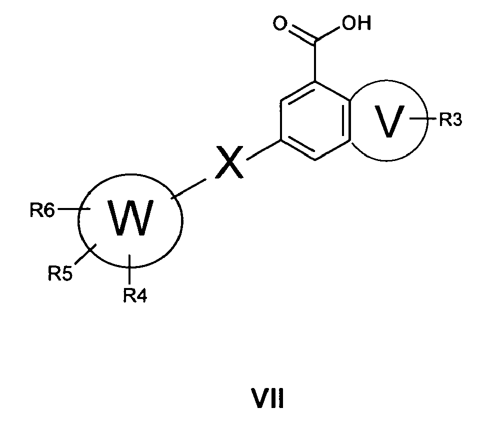 In which R3, R4, R5, R6, V, W and X have the same meaning as defined in claim 1.

8. Process for preparing compounds of the formula I of claim 1, wherein the building block of the formula XI 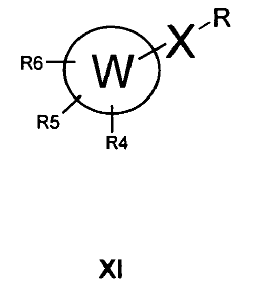 in which R4, R5, R6, W and X have the same meaning as defined in claim 1
and
R is a -B(OH)₂, -C≡C-H, -Zn-Hal or -Sn(alkyl)₃) group,
is coupled in a metal catalyzed cross-coupling reaction
with an aryl halide of the formula VIII 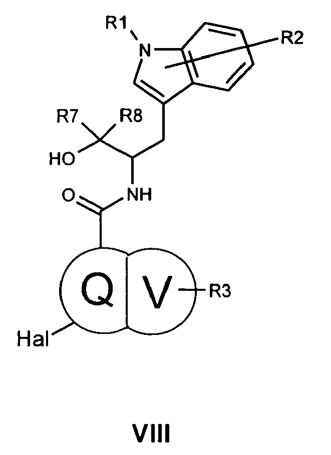 in which R1, R2, R3, R7, R8, Q, V have the same meaning as defined in claim 1, and
Hal stands for a chlorine, bromine or iodine.

9. Process according to claim 8 for preparing compounds of the formula II of claim 2, wherein the building block of the formula XI is coupled with an aryl halide of the formula IX 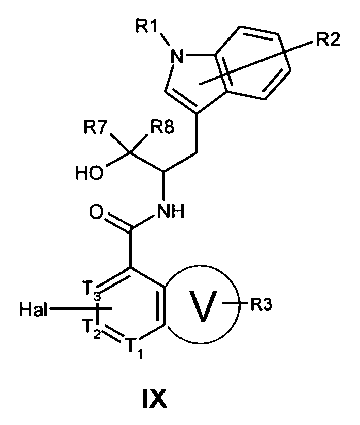 in which R1, R2, R3, R7, R8, V have the same meaning as defined in claim 1, and
Hal stands for a chlorine, bromine or iodine.

10. Process according to claim 8 for preparing compounds of the formula III of claim 3, wherein the building block of the formula XI is coupled with an aryl halide of the formula X 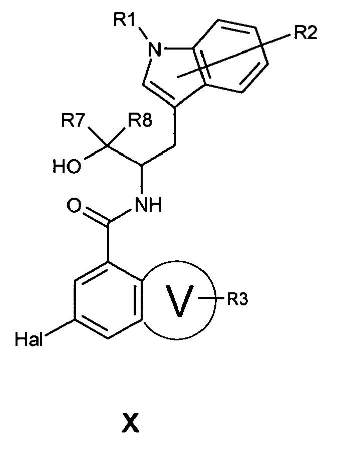 in which R1, R2, R3, R7, R8 and V have the same meaning as defined in claim 1,
and
Hal stands for a chlorine, bromine or iodine.

11. Carboxylic acids as intermediates in a process according to any of claims 5 to 7, namely
6-(3,4,5-Trimethoxy-phenyl)-2H-chromene-8-carboxylic acid;
6-o-Tolylethynyl-2H-chromene-8-carboxylic acid;
6-(2-Methoxy-phenylethynyl)-2H-chromene-8-carboxylic acid;
5-(3,4-Dimethoxy-phenyl)-2-vinyl-2,3-dihydro-benzofuran-7-carboxylic acid;
5-(3,4-Dimethoxy-phenyl)-2-ethyl-2,3-dihydro-benzofuran-7-carboxylic acid;
5-(3,4-Difluoro-5-methoxy-phenyl)-2,3-dihydro-benzofuran-7-carboxylic acid;
5-(3,4-Dimethoxy-phenyl)-2,3-dihydro-benzofuran-7-carboxylic acid;
5-(3-Methoxy-phenyl)-2,3-dihydro-benzofuran-7-carboxylic acid;
5-Benzo[1,3]dioxol-5-yl-2,3-dihydro-benzofuran-7-carboxylic acid;
5-(3-Fluoro-4-methoxy-phenyl)-2,3-dihydro-benzofuran-7-carboxylic acid;
5-(3,4,5-Trimethoxy-phenyl)-2,3-dihydro-benzofuran-7-carboxylic acid;
7-(3-Fluoro-5-methylcarbamoyl-phenyl)-2,3,4,5-tetrahydro-benzo[b]oxepine-9-carboxylic acid
and the methyl, ethyl, propyl and butyl esters thereof.

12. Aryl halides as intermediates in a process according to any of claims 8 to 10, namely
6-Iodo-2H-chromene-8-carboxylic acid [(R)-2-hydroxy-1-(1 H-indol-3-ylmethyl)-ethyl]-amide;
6-Iodo-2-methyl-2H-chromene-8-carboxylic acid [(R)-1-hydroxymethyl-2-(1 H-indol-3-yl)-ethyl]-amide;
6-Iodo-2,2-dimethyl-2H-chromene-8-carboxylic acid [(R)-2-hydroxy-1-(1 H-indol-3-ylmethyl)-ethyl]-amide;
7-Bromo-2,3-dihydro-benzo[1,4]dioxine-5-carboxylic acid [(R)-2-hydroxy-1-(1 H-indol-3-ylmethyl)-ethyl]-amide;
7-Bromo-2,3-dihydro-benzo[1,4]dioxine-5-carboxylic acid [2-(6-fluoro-1H-indol-3-yl)-1-hydroxymethyl-ethyl]-amide;
7-Bromo-2,3,4,5-tetrahydro-benzo[b]oxepine-9-carboxylic acid [(R)-2-hydroxy-1-(1H-indol-3-ylmethyl)-ethyl]-amide;
7-Bromo-2,3,4,5-tetrahydro-benzo[b]oxepine-9-carboxylic acid [1-(6-fluoro-1H-indol-3-ylmethyl)-2-hydroxy-ethyl]-amide.

13. Pharmaceutical compositions comprising at least one of the compounds according to any of claims 1 to 4 with pharmaceutically suitable excipients and/or carriers.

14. Use of the compounds of the general formula I according to any of claims 1 to 4 for the fertility control in men or in women.

15. Process for producing medicaments comprising at least one of the compounds of the general formula I according to any of claims 1 to 4 for the prevention and/or treatment of osteoporosis.

## Amended claims

### Amended claims in accordance with Rule 137(2) EPC.

**1.** Compounds of the formula I 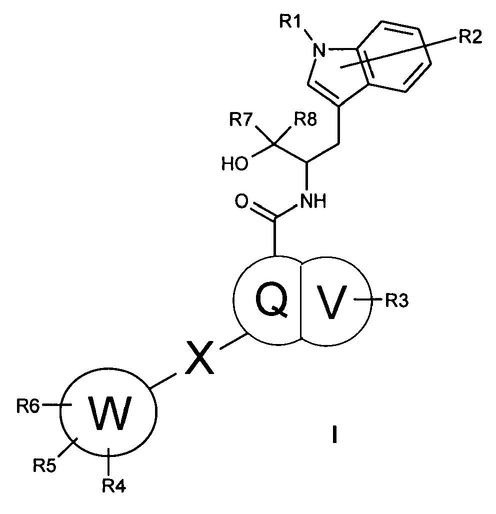 in which
R1 is hydrogen, C₁-C₆-alkyl, C₃-C₆-alkenyl, C₃-C₆-alkynyl, C₃-C₇-cycloalkyl, C₁-C₆-alkyloxy-C₁-C₆-alkylene, C₃-C₇-cycloalkyloxy-C₁-C₆-alkylene, C₁-C₆-alkylamino-C₁-C₆-alkylene, di(C₁-C₆-alkyl)amino-C₁-C₆-alkylene, phenyloxy-C₁-C₆-alkylene;
wherein the hydrocarbon chains may optionally be substituted once or more times by fluorine, cyano, hydroxy, amino or the groups: 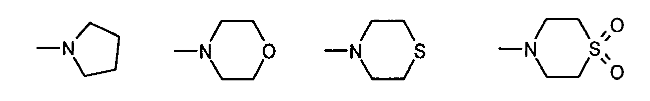 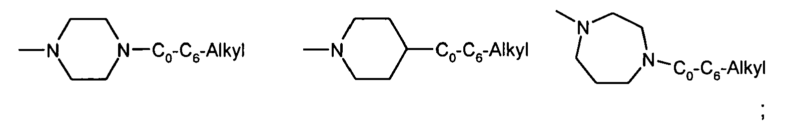
R2 is hydrogen, halogen, cyano, -SO₂Me, C₁-C₆-alkyl, C₂-C₆-alkenyl, C₂-C₆-alkynyl, C₁-C₆-alkyloxy or benzyloxy,
wherein the hydrocarbon chains may optionally be fluorinated once or more times;
R3 is hydrogen, hydroxy, halogen, nitro, amino, cyano, C₁-C₆-alkyl, C₂-C₆-alkenyl or C₂-C₆-alkynyl, C₃-C₇-cycloalkyl, hydroxy-C₁-C₆-alkylene, hydroxy-C₃-C₆-alkenylene, hydroxy-C₃-C₆-alkynylene, C₁-C₆-alkyloxy, C₁-C₆-alkyloxy-C₁-C₆-alkylene, C₃-C₇-cycloalkyloxy, C₃-C₇-cycloalkyl-C₁-C₆-alkylenoxy, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, C₁-C₆-al kylamino-C₁-C₆-alkylene, di(C₁-C₆-alkyl)amino-C₁-C₆-alkylene, phenyloxy-C₁-C₆-alkylene;
where the hydrocarbon chains therein may optionally be substituted one or more times by fluorine, cyano, hydroxy, amino or the groups 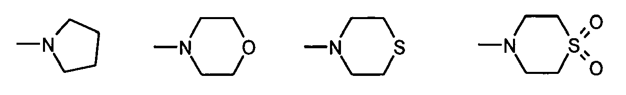 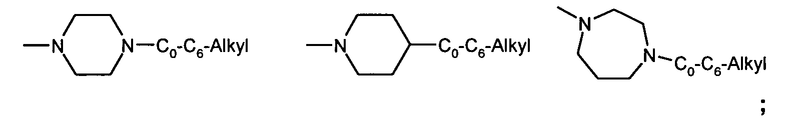
R4, R5, R6 are independently of one another hydrogen, hydroxy, halogen, nitro, amino, cyano, phenyl, C₁-C₆-alkyl, C₂-C₆-alkenyl or C₂-C₆-alkynyl, C₃-C₇-cycloalkyl, C₃-C₇-cycloalkyl-C₁-C₆-alkylene, C₃-C₇-heterocycloalkyl, where the hydrocarbon chains therein may optionally be substituted one or more times by fluorine, cyano or the groups: 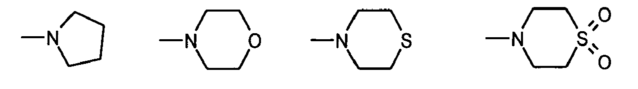 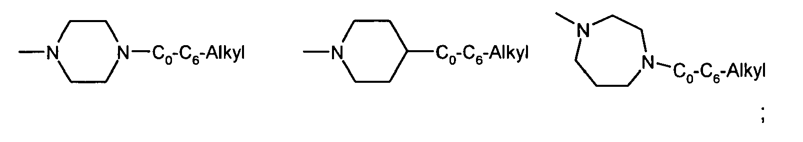 or
independently of one another hydroxy-C₁-C₆-alkylene, hydroxy-C₃-C₆-alkenylene, hydroxy-C₃-C₆-alkynylene, C₁-C₆-alkyloxy, C₃-C₇-cycloalkyloxy, C₃-C₇-cycloalkyl-C₁-C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, phenyloxy-C₁-C₆-alkylene, C₁-C₆-alkylamino, di(C₁-C₆-alkyl)amino, C₁-C₆-alkylamino-C₁-C₆-alkylene, di(C₁-C₆)-alkylamino-C₁-C₆-alkylene, C₃-C₇-cycloalkyl-(C₀-C₆-alkyl)amino, C₁-C₆-acyl-(C₀-C₆-alkyl)amido, C₁-C₆-alkylaminocarbonyl, di(C₁-C₆-alkyl)aminocarbonyl, (C₃-C₇-cycloalkyl)aminocarbonyl, di(C₃-C₇-cycloalkyl)aminocarbonyl, C₃-C₇-cycloalkyl-C₁-C₆-alkyleneaminocarbonyl, C₁-C₆-alkylcarbonyl, C₃-C₇-cycloalkylcarbonyl, carboxy, carboxamido [-C(O)NH₂], C₁-C₆-alkyloxycarbonyl, C₁-C₃-alkylsulphanyl, C₁-C₆-alkysulphonyl, C₃-C₇-cycloalkylsulphonyl, C₃-C₇-cycloalkyl-C₁-C₆-alkylenesulphonyl, C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido, -N(C₀-C₆-alkyl)-C(O)-C₁-C₆-alkyl, -N(C₀-C₆-alkyl)-C(O)-C₃-C₇-cycloalkyl, -N(C₀-C₆-alkyl)-C(O)-N-di(C₀-C₆-alkyl), -N(C₀-C₆-alkyl)-C(O)-O-(C₀-C₆)alkyl, -N(C₀-C₆-alkyl)-C(O)-NH-C₃-C₇-cycloalkyl, -N(C₀-C₆-alkyl)-SO₂-C₁-C₆-alkyl, -N(C₀-C₆-alkyl)-SO₂-C₃-C₇-cycloalkyl, -N(C₀-C₆-alkyl)-SO₂-N-di(C₀-C₆-alkyl), -N(C₀-C₆-alkyl)-SO₂-NH-(C₃-C₇)-cycloalkyl, -C(O)-N(H)-C₂-C₆-alkylene-(C₁-C₆-alkyl)amine, -C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycloalkyl-C₁-C₆-alkyl)amine, -S(O₂)-N(H)-C₂-C₆-alkylene-(C₁-C₆-alkyl)amine, -S(O₂)-N(H)-C₂-C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine, -O-C₂-C₆-alkylene-(C₁-C₆-alkyl)amine, -O-C₂-C₆-alkylene-[di(C₁-C₆-alkylene)]amine,
or the groups: 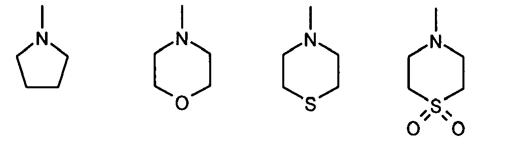 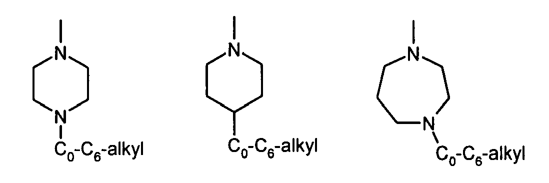 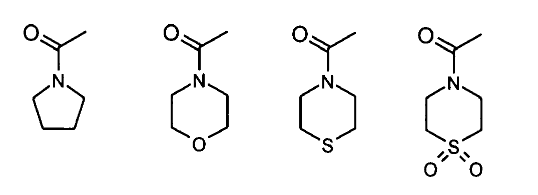 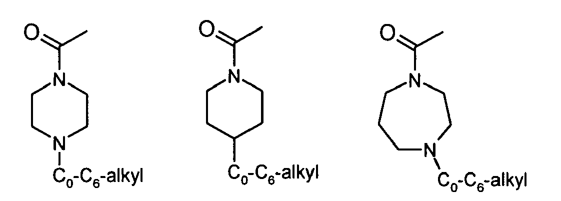 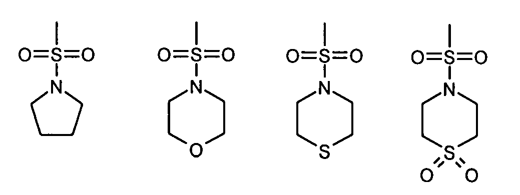 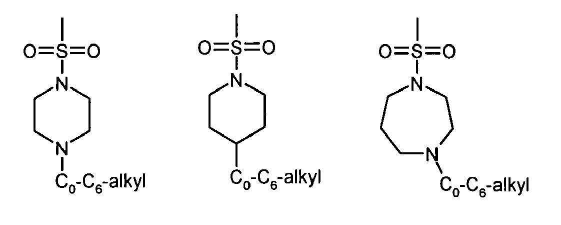 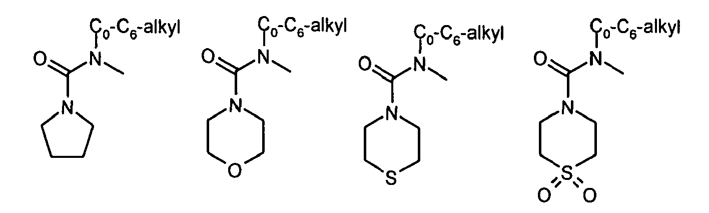 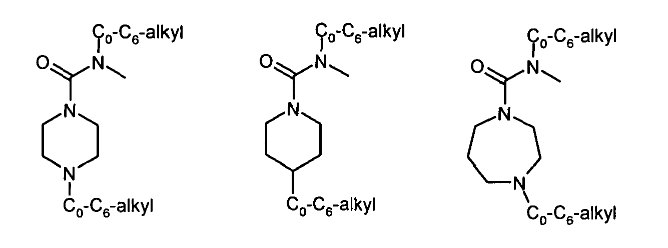 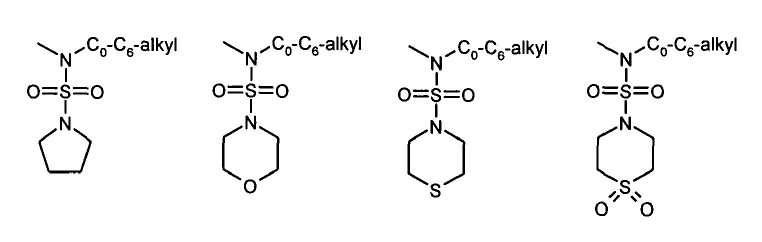 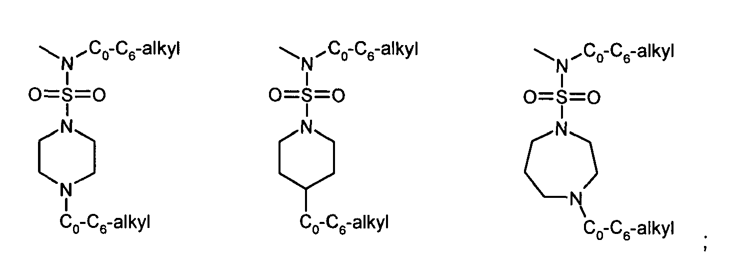
R7, R8 may be independently of one another hydrogen, methyl, ethyl, where the methyl and ethyl radicals may be fluorinated one or more times;
wherein
R2 may substitute one or more positions of the aryl or heteroaryl ring in the indole residue;
R3 may substitute one or more positions of the monocyclic aryl or monocyclic heteroaryl ring of Q and/or V;
R5 and R6 may together form a heterocycloalkyl or cycloalkyl group;
Q is a monocyclic aryl or a monocyclic heteroaryl group;
V is a cycloalkylen, cycloalkenylen, heterocycloalkylen or heterocycloalkenylen group;
W is an aryl or heteroaryl group;
X is a bond, C₁-C₄-alkylene, C₂-C₄-alkenylene or C₂-C₄-alkynylene group.

**2.** Compounds according to claim 1, namely acyltryptophanols of the formula II 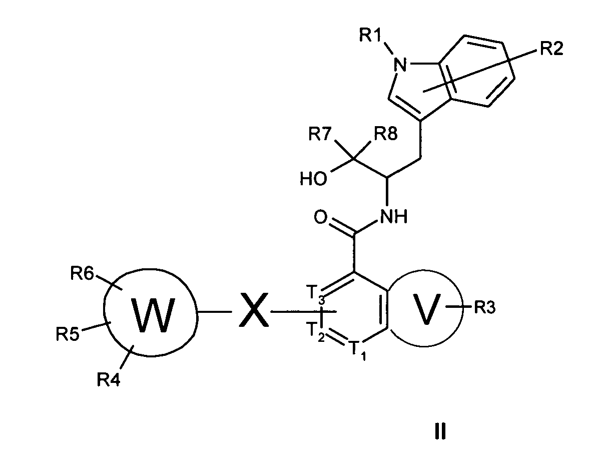 in which the radicals R1 to R8, X, V and W have the same meaning as defined in claim 1
and
T1, T2, T3 are each independently of one another a nitrogen atom or an R3-C group.

**3.** Compounds according to claim 1, namely acyltryptophanols of the formula III 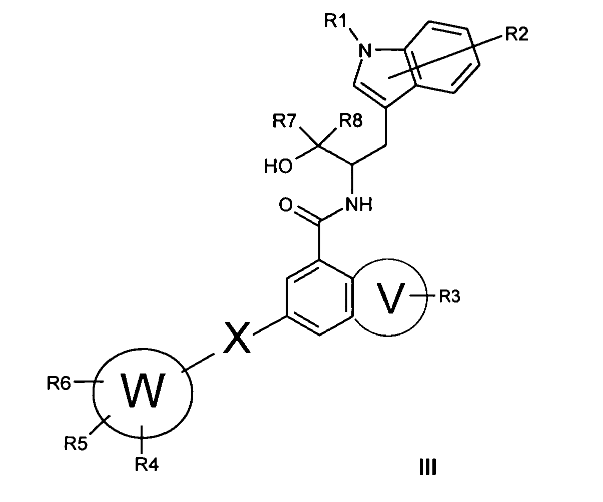 in which R1 to R8, X, V and W have the same meaning as defined in claim 1.

**4.** Compounds according to any of the preceding claims, namely
**1** 6-(3,4,5-Trimethoxy-phenyl)-2H-chromene-8-carboxylic acid [(R)-1-hydroxymethyl-2-(1H-indol-3-yl)-ethyl]-amide;
**2** 6-(3-Chloro-4-methylcarbamoyl-phenyl)-2H-chromene-8-carboxylic acid [(R)-2-hydroxy-1-(1H-indol-3-ylmethyl)-ethyl]-amide;
**3** 6-(3-Chloro-4-methylcarbamoyl-phenyl)-chroman-8-carboxylic acid [(R)-2-hydroxy-1-(1H-indol-3-ylmethyl)-ethyl]-amide;
**4** 6-o-Tolylethynyl-2H-chromene-8-carboxylic acid [(R)-1-hydroxymethyl-2-(1H-indol-3-yl)-ethyl]-amide;
**5** 6-(2-Methoxy-phenylethynyl)-2H-chromene-8-carboxylic acid [(R)-1-hydroxymethyl-2-(1H-indol-3-yl)-ethyl]-amide;
**6** 2-Methyl-6-(3,4,5-trimethoxy-phenyl)-2H-chromene-8-carboxylic acid [(R)-1-hydroxymethyl-2-(1H-indol-3-yl)-ethyl]-amide;
**7** 6-(3-Chloro-4-methylcarbamoyl-phenyl)-2,2-dimethyl-2H-chromene-8-carboxylic acid [(R)-2-hydroxy-1-(1H-indol-3-ylmethyl)-ethyl]-amide;
**8** 6-(3-Fluoro-5-methylcarbamoyl-phenyl)-2,2-dimethyl-2H-chromene-8-carboxylic acid [(R)-2-hydroxy-1-(1H-indol-3-ylmethyl)-ethyl]-amide;
**9** 2,2-Dimethyl-6-(3,4,5-trimethoxy-phenyl)-2H-chromene-8-carboxylic acid [(R)-2-hydroxy-1-(1H-indol-3-ylmethyl)-ethyl]-amide;
**10** 2,2-Dimethyl-6-(4-methylcarbamoyl-phenylethynyl)-2H-chromene-8-carboxylic acid [(R)-1-hydroxymethyl-2-(1H-indol-3-yl)-ethyl]-amide;
**11** 2,2-Dimethyl-6-(3-methylcarbamoyl-phenylethynyl)-2H-chromene-8-carboxylic acid [(R)-1-hydroxymethyl-2-(1H-indol-3-yl)-ethyl]-amide;
**12** 5-(3,4-Dimethoxy-phenyl)-2-vinyl-2,3-dihydro-benzofuran-7-carboxylic acid [(R)-1-hydroxymethyl-2-(1H-indol-3-yl)-ethyl]-amide;
**13** 5-(3,4-Dimethoxy-phenyl)-2-ethyl-2,3-dihydro-benzofuran-7-carboxylic acid [(R)-1-hydroxymethyl-2-(1H-indol-3-yl)-ethyl]-amide;
**14** 7-(3-Chloro-4-methylcarbamoyl-phenyl)-2,3-dihydro-benzo[1,4]dioxine-5-carboxylic acid [(R)-2-hydroxy-1-(1H-indol-3-ylmethyl)-ethyl]-amide;
**15** 7-(3,4,5-Trimethoxy-phenyl)-2,3-dihydro-benzo[1,4]dioxine-5-carboxylic acid [(R)-1-hydroxymethyl-2-(1H-indol-3-yl)-ethyl]-amide;
**16** 7-(3-Fluoro-5-methylcarbamoyl-phenyl)-2,3-dihydro-benzo[1,4]dioxine-5-carboxylic acid [(R)-2-hydroxy-1-(1H-indol-3-ylmethyl)-ethyl]-amide;
**17** 7-(3-Fluoro-5-methylcarbamoyl-phenyl)-2,3-dihydro-benzo[1,4]dioxine-5-carboxylic acid [2-(6-fluoro-1H-indol-3-yl)-1-hydroxymethyl-ethyl]-amide;
**18** 7-(3,4,5-Trimethoxy-phenyl)-2,3-dihydro-benzo-[1,4]dioxine-5-carboxylic acid [2-(6-fluoro-1H-indol-3-yl)-1-hydroxymethyl-ethyl]-amide;
**19** 7-(3-Chloro-4-methylcarbamoyl-phenyl)-2,3-dihydro-benzo[1,4]dioxine-5-carboxylic acid [2-(6-fluoro-1H-indol-3-yl)-1-hydroxymethyl-ethyl]-amide;
**20** 5-(3,4-Difluoro-5-methoxy-phenyl)-2,3-dihydro-benzofuran-7-carboxylic acid [(R)-2-hydroxy-1-(1H-indol-3-ylmethyl)-ethyl]-amide;
**21** 5-(3,4-Dimethoxy-phenyl)-2,3-dihydro-benzofuran-7-carboxylic acid [(R)-1-hydroxymethyl-2-(1H-indol-3-yl)-ethyl]-amide;
**22** 5-(3-Methoxy-phenyl)-2,3-dihydro-benzofuran-7-carboxylic acid [(R)-1-hydroxymethyl-2-(1H-indol-3-yl)-ethyl]-amide;
**23** 5-Benzo[1,3]dioxol-5-yl-2,3-dihydro-benzofuran-7-carboxylic acid [(R)-1-hydroxymethyl-2-(1H-indol-3-yl)-ethyl]-amide;
**24** 5-(3-Fluoro-4-methoxy-phenyl)-2,3-dihydro-benzofuran-7-carboxylic acid [(R)-2-hydroxy-1-(1H-indol-3-ylmethyl)-ethyl]-amide;
**25** 5-(3,4,5-Trimethoxy-phenyl)-2,3-dihydro-benzofuran-7-carboxylic acid [(R)-1-hydroxymethyl-2-(1H-indol-3-yl)-ethyl]-amide;
**26** 7-(3-Chloro-4-methylcarbamoyl-phenyl)-2,3,4,5-tetrahydro-benzo[b]oxepine-9-carboxylic acid [(R)-2-hydroxy-1-(1H-indol-3-ylmethyl)- ethyl]-amide;
**27** 7-(4-Carbamoyl-3-chloro-phenyl)-2,3,4,5-tetrahydro-benzo[b]oxepine-9-carboxylic acid [(R)-2-hydroxy-1-(1H-indol-3-ylmethyl)-ethyl]-amide;
**28** 7-[3-Chloro-4-(morpholine-4-carbonyl)-phenyl]-2,3,4,5-tetrahydro-benzo[b]-oxepine-9-carboxylic acid [(R)-2-hydroxy-1-(1H-indol-3-ylmethyl)-ethyl]-amide;
**29** 7-(3-Chloro-4-methylcarbamoyl-phenyl)-2,3,4,5-tetrahydro-benzo[b]oxepine-9-carboxylic acid [1-(6-fluoro-1H-indol-3-ylmethyl)-2-hydroxy-ethyl]-amide;
**30** 7-(4-Carbamoyl-3-chloro-phenyl)-2,3,4,5-tetrahydro-benzo[b]oxepine-9-carboxylic acid [1-(6-fluoro-1H-indol-3-ylmethyl)-2-hydroxy-ethyl]-amide;
**31** 7-[3-Chloro-4-(morpholine-4-carbonyl)-phenyl]-2,3,4,5-tetrahydro-benzo[b]-oxepine-9-carboxylic acid [(R)-1-(6-fluoro-1H-indol-3-ylmethyl)-2-hydroxyethyl]-amide;
**32** 7-(3-Fluoro-5-methylcarbamoyl-phenyl)-2,3,4,5-tetrahydro-benzo[b]oxepine-9-carboxylic acid [2-(4-fluoro-1H-indol-3-yl)-1-hydroxymethyl-ethyl]-amide;
**33** 7-(3-Fluoro-5-methylcarbamoyl-phenyl)-2,3,4,5-tetrahydro-benzo[b]oxepine-9-carboxylic acid [2-(5-fluoro-1H-indol-3-yl)-1-hydroxymethyl-ethyl]-amide;
**34** 7-(3-Fluoro-5-methylcarbamoyl-phenyl)-2,3,4,5-tetrahydro-benzo[b]oxepine-9-carboxylic acid [2-(6-fluoro-1H-indol-3-yl)-1-hydroxymethyl-ethyl]-amide;
**35** 7-(3-Fluoro-5-methylcarbamoyl-phenyl)-2,3,4,5-tetrahydro-benzo[b]oxepine-9-carboxylic acid [(R)-2-hydroxy-1-(1H-indol-3-ylmethyl)-ethyl]-amide;
**36** 7-(4-Methylcarbamoyl-phenylethynyl)-2,3,4,5-tetrahydro-benzo[b]oxepine-9-carboxylic acid [(R)-1-hydroxymethyl-2-(1H-indol-3-yl)-ethyl]-amide;
**37** 7-(3-Methylcarbamoyl-phenylethynyl)-2,3,4,5-tetrahydro-benzo[b]oxepine-9-carboxylic acid [(R)-1-hydroxymethyl-2-(1H-indol-3-yl)-ethyl]-amide;
**38** 7-(3-Fluoro-5-methylcarbamoyl-phenyl)-2,3,4,5-tetrahydro-benzo[b]oxepine-9-carboxylic acid [2-(5,6-difluoro-1H-indol-3-yl)-1-hydroxymethyl-ethyl]-amide;
**39** 7-(3-Fluoro-5-methylcarbamoyl-phenyl)-2,3,4,5-tetrahydro-benzo[b]oxepine-9-carboxylic acid [2-(5,7-difluoro-1H-indol-3-yl)-1-hydroxymethyl-ethyl]-amide;
**40** 7-((E)-Styryl)-2,3,4,5-tetrahydro-benzo[b]oxepine-9-carboxylic acid [(R)-1-hydroxymethyl-2-(1H-indol-3-yl)-ethyl]-amide;

**5.** Process for preparing compounds of the formula I of claim 1, wherein a tryptophanol derivative of the formula IV 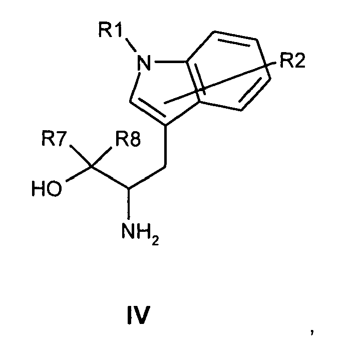 in which the radicals R1, R2, R7 and R8 have the same meaning as defined in claim 1,
is coupled with a carboxylic acid of the formula V 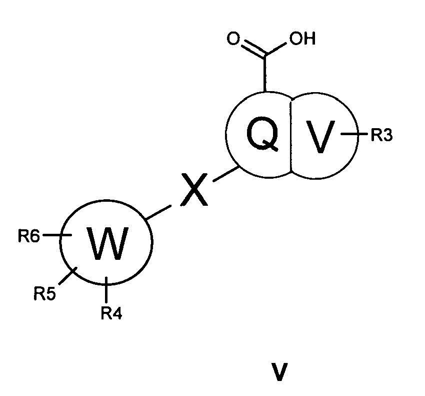 in which R3, R4, R5, R6, Q, X, V and W have the same meaning as defined in claim 1,
in an amide forming reaction comprising
a) conversion of said carboxylic acids into an intermediate active ester or carbonyl chloride with a suitable peptide-coupling reagent, or with thionyl chloride, oxalyl chloride, phosgene or derivatives thereof, where appropriate in the presence of a base,
b) reacting the active intermediate resulting from step a) with said tryptophanol.

**6.** Process according to claim 5 for preparing compounds of the formula II of claim 2, wherein a tryptophanol derivative of the formula IV is coupled with a carboxylic acid of the formula VI 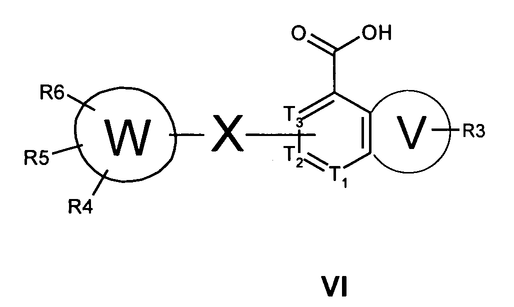 in which R3, R4, R5, R6, T₁, T₂, T₃, X, V and W have the same meaning as defined in claim 2.

**7.** Process according to claim 5 for preparing compounds of the formulae III of claim 3, wherein a tryptophanol derivative of the formula IV is coupled with a carboxylic acid of the formula VII 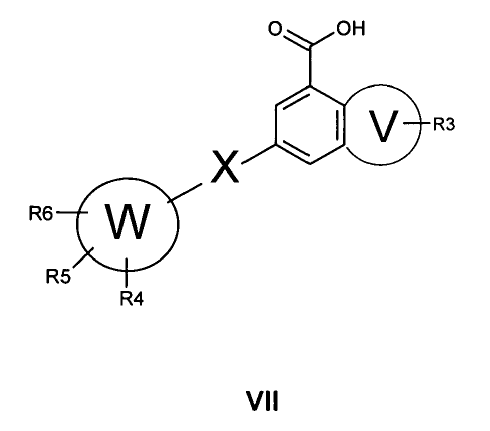 In which R3, R4, R5, R6, V, W and X have the same meaning as defined in claim 1.

**8.** Process for preparing compounds of the formula I of claim 1, wherein the building block of the formula XI 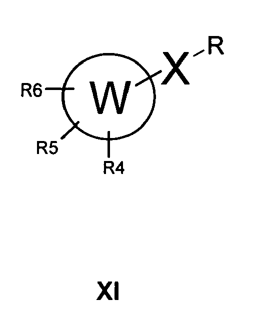 in which R4, R5, R6, W and X have the same meaning as defined in claim 1
and
R is a -B(OH)₂, -C≡C-H, -Zn-Hal or -Sn(alkyl)₃) group,
is coupled in a metal catalyzed cross-coupling reaction
with an aryl halide of the formula VIII 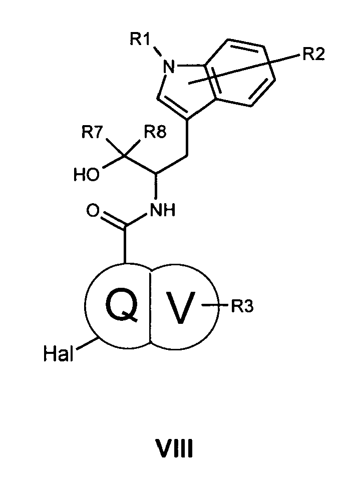 in which R1, R2, R3, R7, R8, Q, V have the same meaning as defined in claim 1, and
Hal stands for a chlorine, bromine or iodine.

**9.** Process according to claim 8 for preparing compounds of the formula II of claim 2, wherein the building block of the formula XI is coupled with an aryl halide of the formula IX 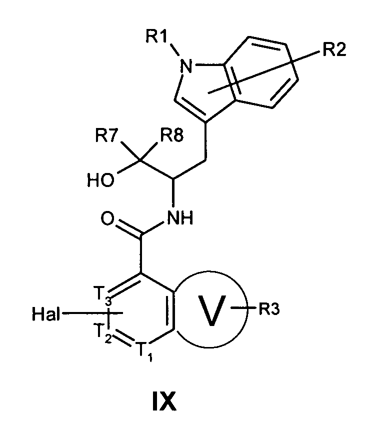 in which R1, R2, R3, R7, R8, V have the same meaning as defined in claim 1, and
Hal stands for a chlorine, bromine or iodine.

**10.** Process according to claim 8 for preparing compounds of the formula III of claim 3, wherein the building block of the formula XI is coupled with an aryl halide of the formula X 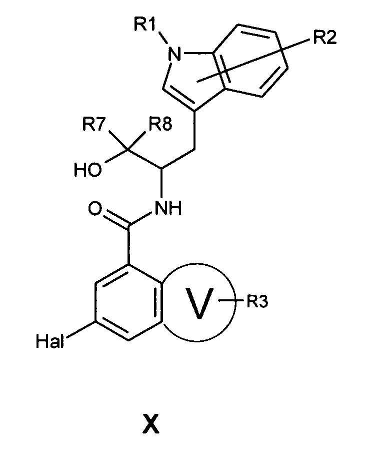 in which R1, R2, R3, R7, R8 and V have the same meaning as defined in claim 1,
and
Hal stands for a chlorine, bromine or iodine.

**11.** Carboxylic acids as intermediates in a process according to any of claims 5 to 7, namely
6-(3,4,5-Trimethoxy-phenyl)-2H-chromene-8-carboxylic acid;
6-o-Tolylethynyl-2H-chromene-8-carboxylic acid;
6-(2-Methoxy-phenylethynyl)-2H-chromene-8-carboxylic acid;
5-(3,4-Dimethoxy-phenyl)-2-vinyl-2,3-dihydro-benzofuran-7-carboxylic acid;
5-(3,4-Dimethoxy-phenyl)-2-ethyl-2,3-dihydro-benzofuran-7-carboxylic acid;
5-(3,4-Difluoro-5-methoxy-phenyl)-2,3-dihydro-benzofuran-7-carboxylic acid;
5-(3,4-Dimethoxy-phenyl)-2,3-dihydro-benzofuran-7-carboxylic acid;
5-(3-Methoxy-phenyl)-2,3-dihydro-benzofuran-7-carboxylic acid;
5-Benzo[1,3]dioxol-5-yl-2,3-dihydro-benzofuran-7-carboxylic acid;
5-(3-Fluoro-4-methoxy-phenyl)-2,3-dihydro-benzofuran-7-carboxylic acid;
5-(3,4,5-Trimethoxy-phenyl)-2,3-dihydro-benzofuran-7-carboxylic acid;
7-(3-Fluoro-5-methylcarbamoyl-phenyl)-2,3,4,5-tetrahydro-benzo[b]oxepine-9-carboxylic acid
and the methyl, ethyl, propyl and butyl esters thereof.

**12.** Aryl halides as intermediates in a process according to any of claims 8 to 10, namely
6-Iodo-2H-chromene-8-carboxylic acid [(R)-2-hydroxy-1-(1H-indol-3-ylmethyl)-ethyl]-amide;
6-Iodo-2-methyl-2H-chromene-8-carboxylic acid [(R)-1-hydroxymethyl-2-(1H-indol-3-yl)-ethyl]-amide;
6-Iodo-2,2-dimethyl-2H-chromene-8-carboxylic acid [(R)-2-hydroxy-1-(1H-indol-3-ylmethyl)-ethyl]-amide;
7-Bromo-2,3-dihydro-benzo[1,4]dioxine-5-carboxylic acid [(R)-2-hydroxy-1-(1H-indol-3-ylmethyl)-ethyl]-amide;
7-Bromo-2,3-dihydro-benzo[1,4]dioxine-5-carboxylic acid [2-(6-fluoro-1H-indol-3-yl)-1-hydroxymethyl-ethyl]-amide;
7-Bromo-2,3,4,5-tetrahydro-benzo[b]oxepine-9-carboxylic acid [(R)-2-hydroxy-1-(1H-indol-3-ylmethyl)-ethyl]-amide;
7-Bromo-2,3,4,5-tetrahydro-benzo[b]oxepine-9-carboxylic acid [1-(6-fluoro-1H-indol-3-ylmethyl)-2-hydroxy-ethyl]-amide.

**13.** Pharmaceutical compositions comprising at least one of the compounds according to any of claims 1 to 4 with pharmaceutically suitable excipients and/or carriers.

**14.** Use of the compounds of the general formula I according to any of claims 1 to 4 for the fertility control in men or in women.

**15.** Use of at least one of the compounds of the general formula I according to any of claims 1 to 4 for the manufacture of a medicament for the prevention and/or treatment of osteoporosis.
